# EUROPEAN PATENT SPECIFICATION

(11) **EP 4 253 378 B1**
(45) Date of publication and mention of the grant of the patent: **10.09.2025**
(21) Application number: 22756597.5
(22) Date of filing: 22.02.2022
(51) Int. Cl.: C09K 11/06, C07B 59/00, C07D 405/04, C07D 405/14, C07D 409/04, C07D 409/14, H10K 50/12, H10K 85/60, H10K 50/11

(54) **NOVEL COMPOUND AND ORGANIC LIGHT EMITTING DEVICE COMPRISING SAME**
NEUARTIGE VERBINDUNG UND ORGANISCHE LICHTEMITTIERENDE VORRICHTUNG DAMIT
NOUVEAU COMPOSÉ ET DISPOSITIF ÉLECTROLUMINESCENT ORGANIQUE LE COMPRENANT

(30) Priority: 22.02.2021 KR 20210023632; 21.02.2022 KR 20220022273
(43) Date of publication of application: 04.10.2023
(73) Proprietor: LG Chem, Ltd., Seoul 07336 (KR)
(72) Inventor: JUNG, Min Woo, Daejeon 34122 (KR); LEE, Dong Hoon, Daejeon 34122 (KR); SUH, Sang Duk, Daejeon 34122 (KR); LEE, Jungha, Daejeon 34122 (KR); HAN, Su Jin, Daejeon 34122 (KR); PARK, Seulchan, Daejeon 34122 (KR); HWANG, Sunghyun, Daejeon 34122 (KR)
(74) Representative: Hoffmann Eitle
(86) International application number: PCT/KR2022/002559
(87) International publication number: WO 2022/177399

(56) References cited:
- WO-A1-2019/066250
- KR-A- 20140 058 292
- KR-A- 20190 000 185
- KR-A- 20190 038 254
- KR-A- 20190 123 695
- KR-A- 20200 133 423
- US-A1- 2017 054 087

## Description

### [TECHNICAL FIELD]

### Cross-reference to Related Application(s)

This application claims the benefit of Korean Patent Applications No. 10-2021-0023632 filed on February 22, 2021 and No. 10-2022-0022273 filed on February 21, 2022 in the Korean Intellectual Property Office.

The present disclosure relates to a novel compound and an organic light emitting device including the same.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

### [Prior Art Literature]

### [Patent Literature]

(Patent Literature 0001) Korean Unexamined Patent Publication No. 10-2000-0051826
(Patent Literature 0002) International Patent Publication WO 2019/066250 A1; Compound for organic electronic element, organic electronic element using same, and electronic device comprising same.

### [DETAILED DESCRIPTION OF THE INVENTION]

### [Technical Problem]

The present disclosure relates to a novel compound and an organic light emitting device including the same.

### [Technical Solution]

The invention is set out in the appended set of claims. In the present disclosure, there is provided a compound represented by the following Chemical Formula 1: 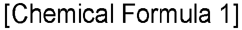 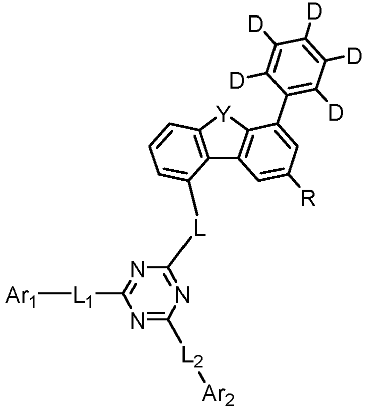 in the Chemical Formula 1,
Y is O or S,
D is deuterium,
L, L₁ and L₂ are each independently a single bond, or C₆₋₂₀ arylene unsubstituted or substituted with deuterium,
Ar₁ and Ar₂are each independently C₆₋₂₀ aryl unsubstituted or substituted with at least one substituent selected from the group consisting of deuterium, methyl and phenyl; unsubstituted dibenzofuranyl; unsubstituted dibenzothiophenyl; or C₂₋₂₀ heteroaryl containing N unsubstituted or substituted with at least one substituent selected from the group consisting of deuterium, methyl and phenyl, and
R is hydrogen; or C₆₋₆₀ aryl unsubstituted or substituted with deuterium.

In addition, there is also provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

### [ADVANTAGEOUS EFFECTS]

The compound represented by the Chemical Formula 1 may be used as a material for an organic material layer of an organic light emitting device, and may improve efficiency, low driving voltage, and/or lifespan of the organic light emitting device.

### [BRIEF DESCRIPTION OF THE DRAWINGS]

FIG. 1 shows an example of an organic light emitting device including a substrate 1, an anode 2, a light emitting layer 3, and a cathode 4.
FIG. 2 shows an example of an organic light emitting device including a substrate 1, an anode 2, a hole injection layer 5, a hole transport layer 6, an electron blocking layer 7, a light emitting layer 3, an electron transport layer 8, an electron injection layer 9, and a cathode 4.

### [DETAILED DESCRIPTION OF THE EMBODIMENTS]

Hereinafter, embodiments of the present disclosure will be described in more detail to facilitate understanding of the invention.

### (Definition of Terms)

As used herein, the notation 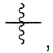 or 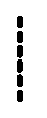 means a bond linked to another substituent group.

As used herein, the term "substituted or unsubstituted" means being unsubstituted or substituted with one or more substituents selected from the group consisting of deuterium; a halogen group; a cyano group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl group containing at least one of **N,** O and S atoms, or being unsubstituted or substituted with a substituent in which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected. For example, the term "substituted or unsubstituted" may be understood to mean "unsubstituted or substituted with at least one substituent, e.g., 1 to 5 substituents, selected from the group consisting of deuterium, halogen, C₁₋₁₀ alkyl, C₁₋₁₀ alkoxy and C₆₋₂₀ aryl". Also, in the present disclosure, the term "substituted with at least one substituent" may be understood to mean "substituted with 1 to 5 substituents"; or "substituted with 1 or 2 substituents".

In the present disclosure, the carbon number of a carbonyl group is not particularly limited, but is preferably 1 to 40. Specifically, the carbonyl group may be a substituent having the following structural formulae, but is not limited thereto.

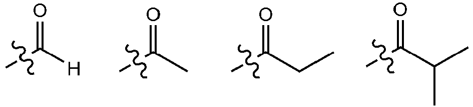

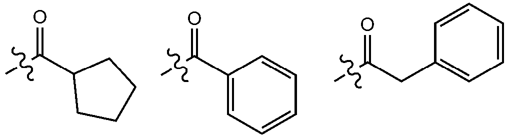

In the present disclosure, an ester group may have a structure in which oxygen of the ester group is substituted by a straight-chain, branched-chain, or cyclic alkyl group having 1 to 25 carbon atoms, or an aryl group having 6 to 25 carbon atoms. Specifically, the ester group may be a substituent having the following structural formulae, but is not limited thereto.

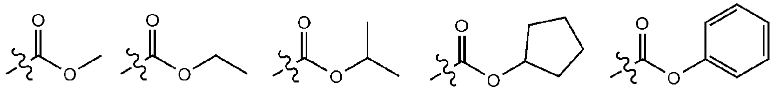

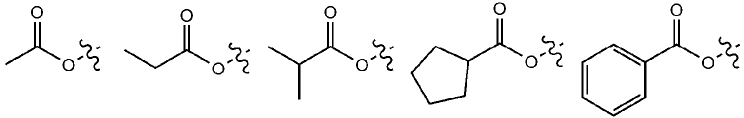

In the present disclosure, the carbon number of an imide group is not particularly limited, but is preferably 1 to 25. Specifically, the imide group may be a substituent having the following structural formulae, but is not limited thereto.

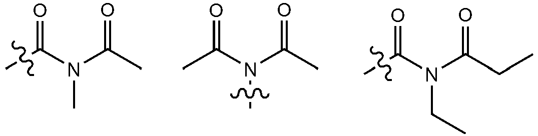

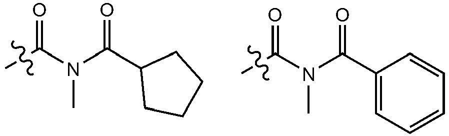

**In** the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

**In** the present disclosure, a boron group specifically includes a trimethylboron group, a triethylboron group, a t-butyldimethylboron group, a triphenylboron group, a phenylboron group and the like, but is not limited thereto.

In the present disclosure, examples of a halogen group include fluoro, chloro, bromo, or iodo.

In the present disclosure, the alkyl group may be straight-chain, or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. Specific examples of the alkyl group include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, 1-ethyl-propyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, isohexyl, 1-methylhexyl, 2-methylhexyl, 3-methylhexyl, 4-methylhexyl, 5-methylhexyl, cyclopentylmethyl, cyclohexylmethyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, and the like, but are not limited thereto.

**In** the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

**In** the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group having aromaticity. According to one embodiment, the carbon number of the aryl group is 6 to 30. According to one embodiment, the carbon number of the aryl group is 6 to 20. The monocyclic aryl group includes a phenyl group, a biphenyl group, a terphenyl group and the like, but is not limited thereto. The polycyclic aryl group includes a naphthyl group, an anthracenyl group, a phenanthrenyl group, a triphenylenyl group, a pyrenyl group, a perylenyl group, a chrysenyl group, or the like, but is not limited thereto.

In the present disclosure, the heteroaryl is heteroaryl containing at least one heteroatom of O, N, Si and S as a heterogeneous element, and the carbon number thereof is not particularly limited, but is preferably 2 to 60. Examples of the heteroaryl include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, the arylamine group, and the arylsilyl group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can apply the aforementioned description of the heteroaryl. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heteroaryl can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heteroaryl can be applied, except that the heterocycle is not a monovalent group but formed by combining two substituent groups.

### (Compound)

Meanwhile, the present disclosure provides a compound represented by the Chemical Formula 1.

Specifically, the compound represented by the Chemical Formula 1 has a structure having a dibenzofuran/dibenzothiophene core in which a phenyl group (phenyl-D5) substituted with five deuteriums is substituted at position 6 of the core and a triazinyl group is further substituted at position 1 of the core. In addition, the compound may have a structure in which the carbon at position 8 of the dibenzofuran/dibenzothiophene is unsubstituted or substituted with deuterium-substituted aryl.

In particular, the compound having a structure in which a triazinyl group is substituted at position 1 and a phenyl group substituted with 5 deuteriums is substituted at position 6 of the dibenzofuran/dibenzothiophene core has a property that a bond energy of the C-D bond is greater than that of the C-H bond. Therefore, it has a stronger bond energy in the molecule compared to a compound not having a phenyl group substituted with deuterium, thereby exhibiting improved material stability. In addition, since deuterium is not directly substituted in the dibenzofuran/dibenzothiophene core, the above compound may exhibit higher electronic stability than a compound in which deuterium is directly substituted in the core.

Accordingly, the organic light emitting device employing the above compound may have significantly improved lifespan.

Meanwhile, in the above compound, Ar₁ and Ar₂, which are substituents of the triazinyl group, are any one substituent selected from the group consisting of:
1) substituted or unsubstituted C₆₋₆₀ aryl;
2) unsubstituted C₄₋₆₀ heteroaryl containing O;
3) unsubstituted C₄₋₆₀ heteroaryl containing S;
4) substituted or unsubstituted C₂₋₆₀ heteroaryl containing N; and
5) substituted or unsubstituted C₂₋₆₀ heteroaryl containing at least two heteroatoms selected from the group consisting of N, O and S

Herein, "C₄₋₆₀ heteroaryl containing O" refers to a monovalent heterocyclic group having 4 to 60 carbon atoms while containing at least one O atom, which is a heteroatom, and examples thereof include thiofuranyl, dibenzofuranyl, benzonaphthofuranyl and dinaphthofuranyl.

In addition, "C₄₋₆₀ heteroaryl containing S" refers to a monovalent heterocyclic group having 4 to 60 carbon atoms while containing at least one S atom, which is a heteroatom, and examples thereof include thiophenyl, benzothiophenyl, dibenzothiophenyl, benzonaphthothiophenyl and dinaphthothiophenyl.

In addition, "C₂₋₆₀ heteroaryl containing N" refers to a monovalent heterocyclic group having 2 to 60 carbon atoms while containing at least one N atom, which is a heteroatom, and examples thereof include imidazolyl, pyrrolyl, pyridinyl, pyrimidinyl, triazinyl, pyrazinyl, quinolinyl, isoquinolinyl, quinazolinyl, indolyl, carbazolyl and benzocarbazolyl.

In addition, "C₂₋₆₀ heteroaryl containing at least two heteroatoms selected from the group consisting of N, O and S" refers to a heterocyclic group having 2 to 60 carbon atoms while containing at least two of N, O and S atoms, which are heteroatoms, and examples thereof include thiazolyl, oxazolyl, benzoxazolyl, isoxazolyl, phenothiazinyl and thiadiazolyl.

In other words, in the Chemical Formula 1, Ar₁ and Ar₂ do not include C₄₋₆₀ heteroaryl containing O substituted with a substituent and C₄₋₆₀ heteroaryl containing S substituted with a substituent. This is because the compound may exhibit higher electronic stability when used as a host material of the light emitting layer compared to the compound in which Ar₁ and/or Ar₂ is a heteroaryl group containing O or S substituted with a substituent such as a phenyl group.

Specifically, in the above Chemical Formula 1, Ar₁ and Ar₂ are each independently C₆₋₂₀ aryl unsubstituted or substituted with at least one substituent selected from the group consisting of deuterium, methyl and phenyl; unsubstituted dibenzofuranyl; unsubstituted dibenzothiophenyl; or C₂₋₂₀ heteroaryl containing N unsubstituted or substituted with at least one substituent selected from the group consisting of deuterium, methyl and phenyl.

More specifically, Ar₁ and Ar₂ may each independently be phenyl, biphenylyl, terphenylyl, naphthyl, phenanthryl, fluorenyl, dibenzofuranyl, dibenzothiophenyl, or carbazolyl, and
the phenyl, biphenylyl, terphenylyl, naphthyl, phenanthryl, fluorenyl, and carbazolyl may each independently be unsubstituted or substituted with at least one substituent, preferably one to five substituents, selected from the group consisting of deuterium, methyl and phenyl.

For example, Ar₁ and Ar₂ may each independently be any one selected from the group consisting of the following, but are not limited thereto:

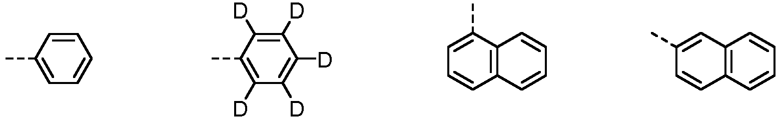

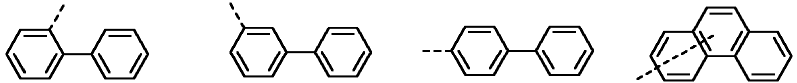

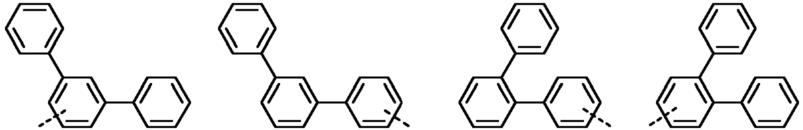

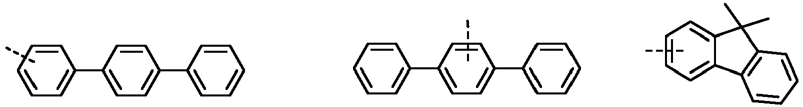

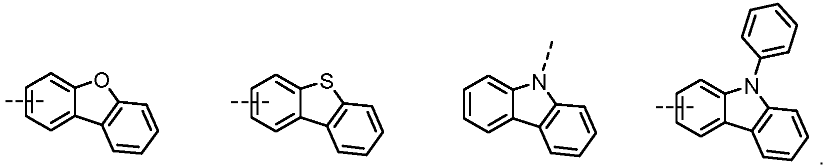

Herein, Ar₁ and Ar₂ may be the same as each other. Alternatively, Ar₁ and Ar₂ may be different from each other.

In one embodiment, at least one of Ar₁ and Ar₂ may be C₆₋₁₂ aryl unsubstituted or substituted with deuterium.

For example, at least one of Ar₁ and Ar₂ may be

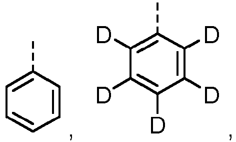

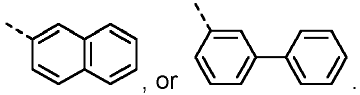

In the above Chemical Formula 1,, L, L₁ and L₂ are a single bond, or C₆₋₂₀ arylene unsubstituted or substituted with deuterium.

Specifically, L may be a single bond.

In addition, L₁ and L₂may each independently be a single bond, or phenylene. In other words, L₁ and L₂ may each independently be a single bond, 1,2-phenylene, 1,3-phenylene, or 1,4-phenylene.

For example, both of L₁ and L₂ are a single bond; or
one of L₁ and L₂ is a single bond, and the other is 1,2-phenylene, 1,3-phenylene, or 1,4-phenylene.

Herein, L₁ and L₂ may be the same as each other. Alternatively, L₁ and L₂ may be different from each other.

In one embodiment, R may be hydrogen; or C₆₋₂₀ aryl unsubstituted or substituted with deuterium.

More specifically, R may be hydrogen; phenyl unsubstituted or substituted with deuterium; biphenylyl unsubstituted or substituted with deuterium; or naphthyl unsubstituted or substituted with deuterium.

For example, R may be hydrogen,

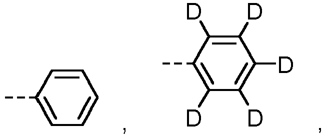

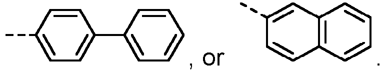

In one embodiment, when R is not hydrogen, that is, when R is C₆₋₆₀ aryl unsubstituted or substituted with deuterium, R may be the same as Ar₁ or Ar₂.

In one embodiment, the compound may be represented by any one of the following Chemical Formulae 1-1 to 1-5: 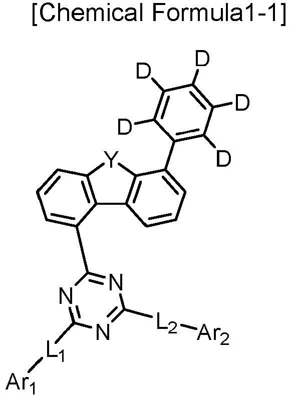 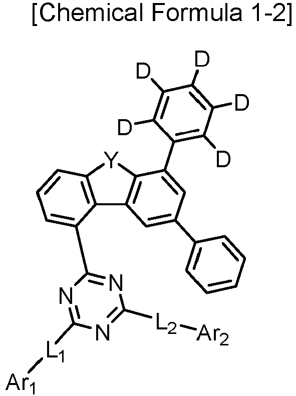 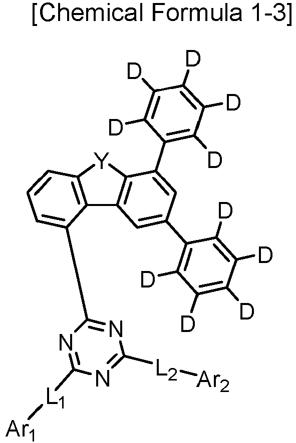 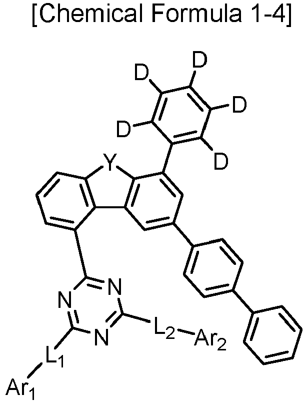 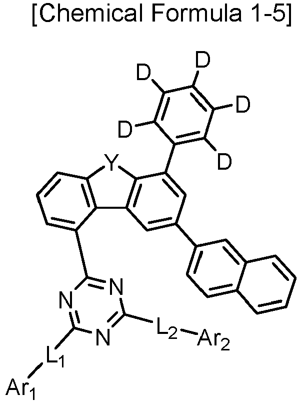 in the Chemical Formulae 1-1 to 1-5,
Y, L₁, L₂, Ar₁ and Ar₂ are as defined in the Chemical Formula 1.

Meanwhile, representative examples of the compound represented by the Chemical Formula 1 are as follows:

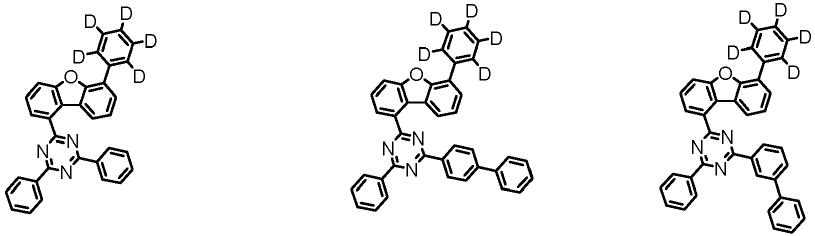

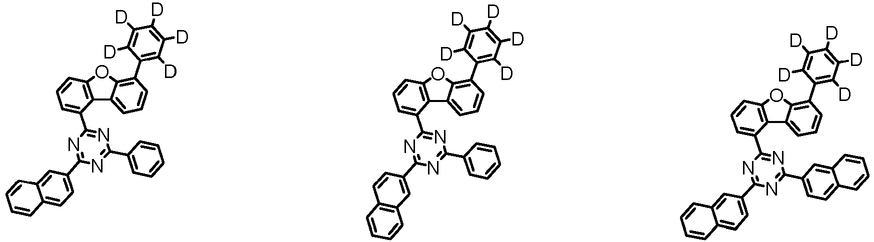

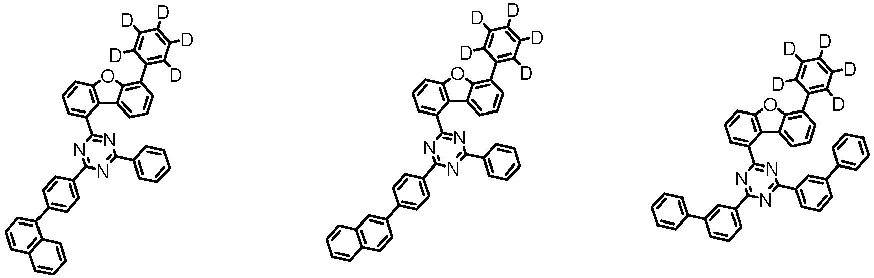

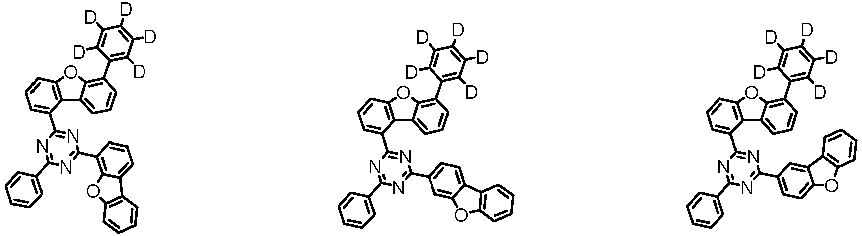

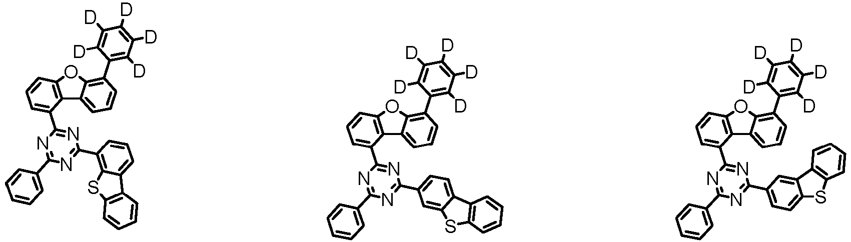

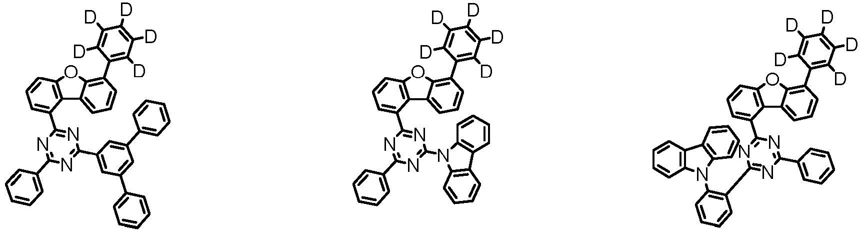

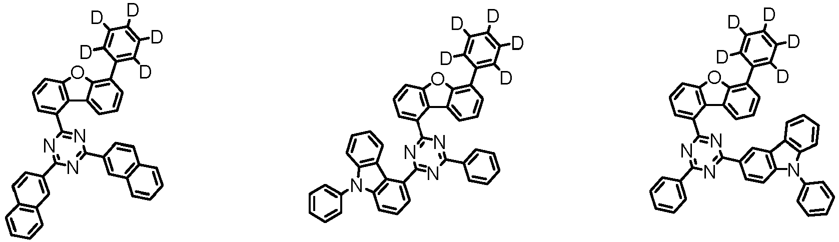

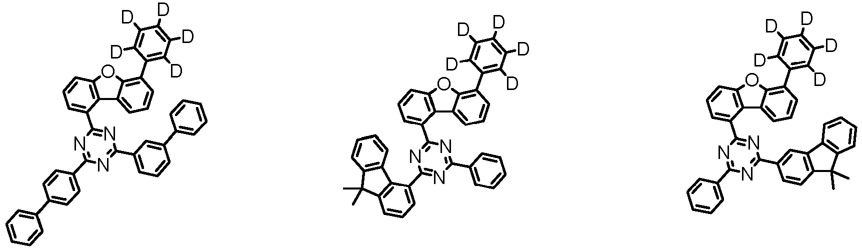

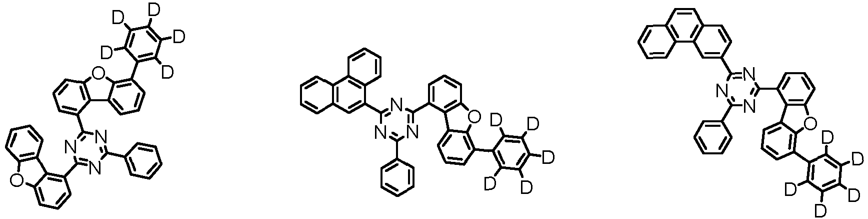

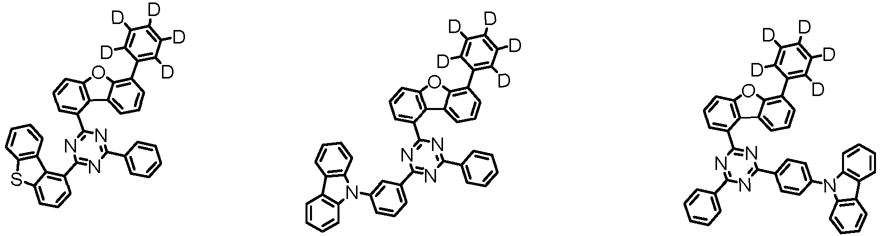

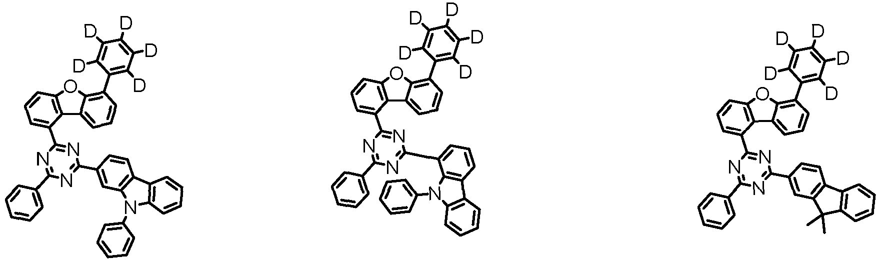

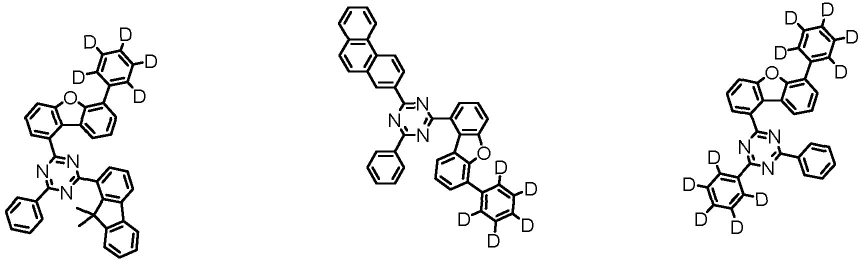

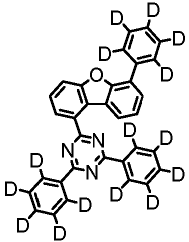

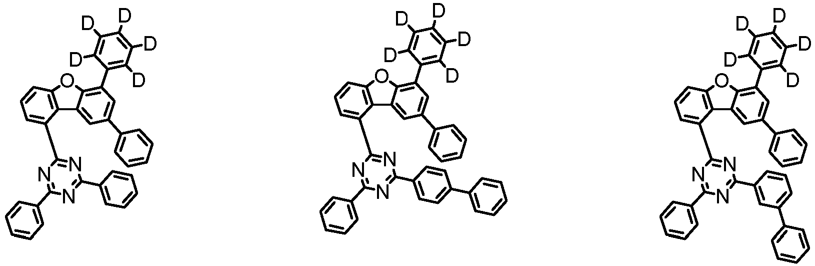

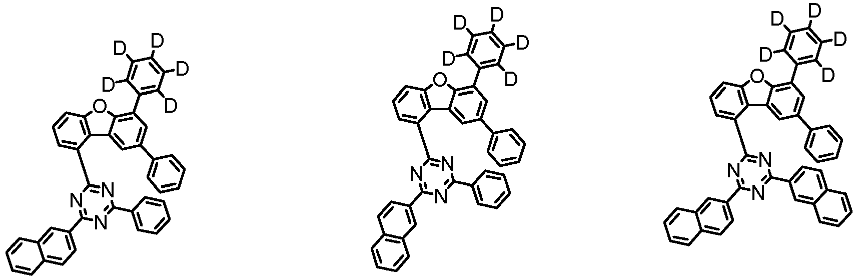

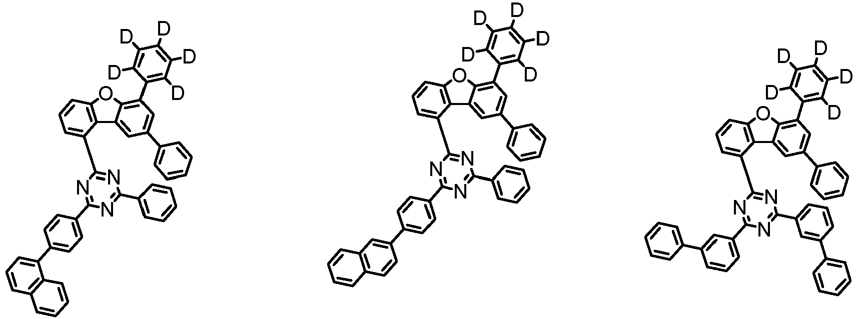

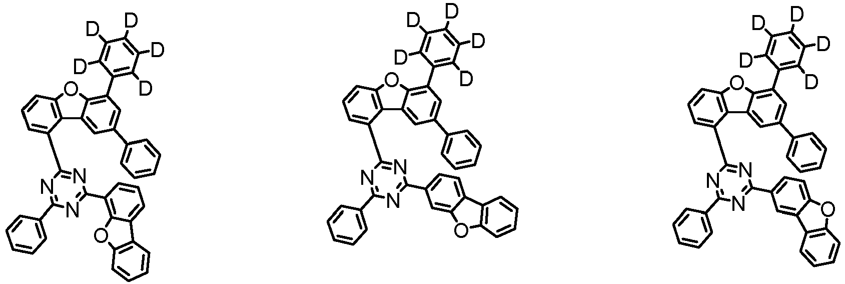

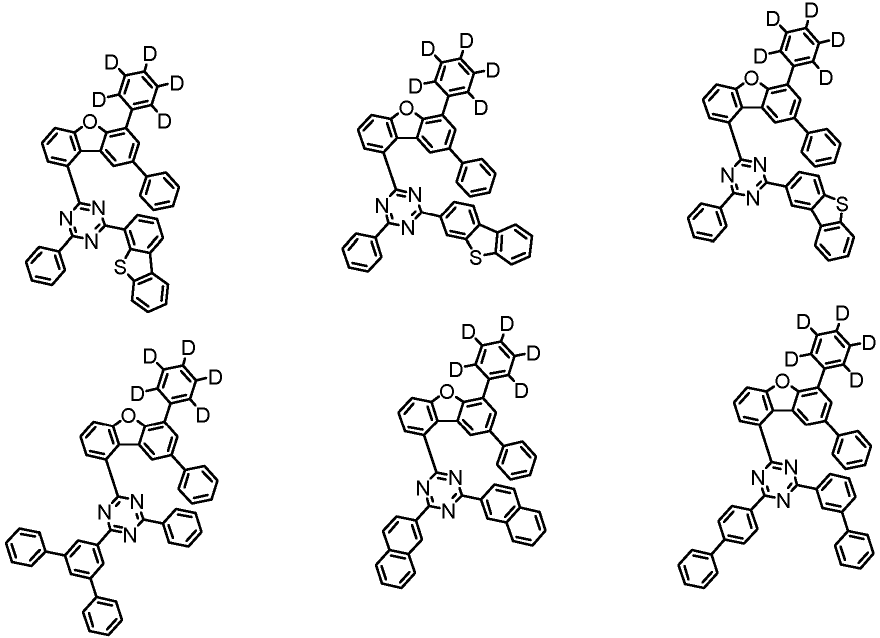

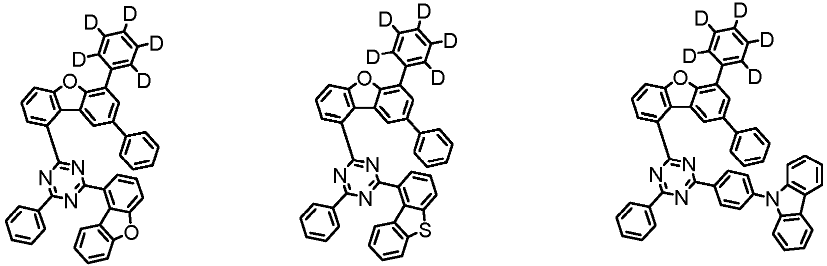

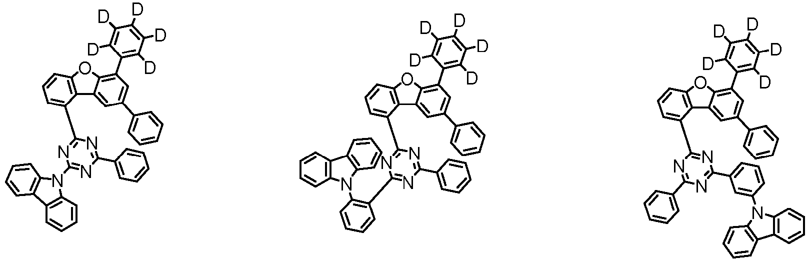

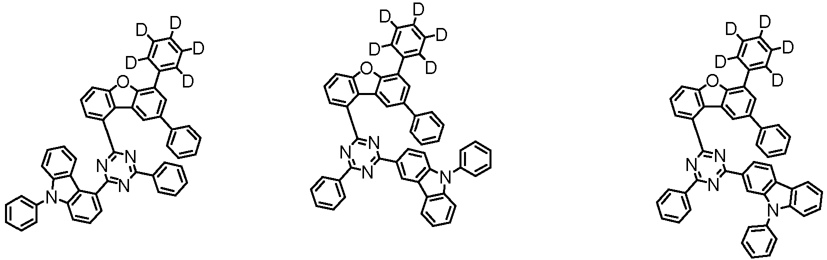

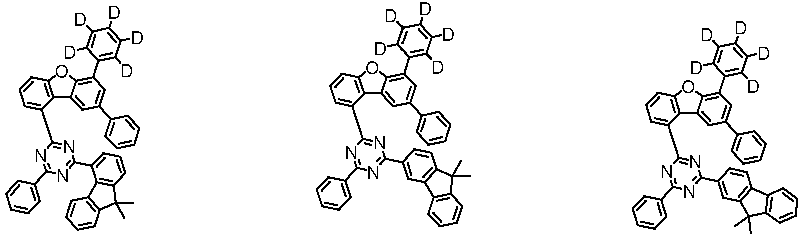

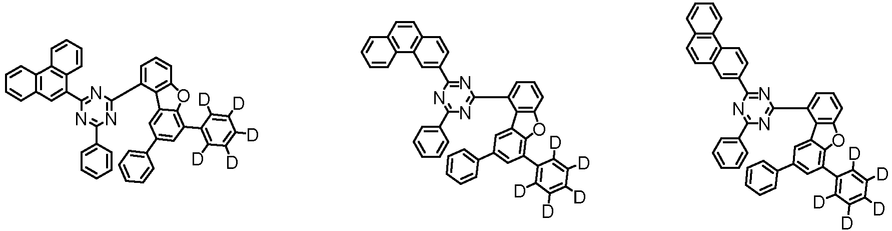

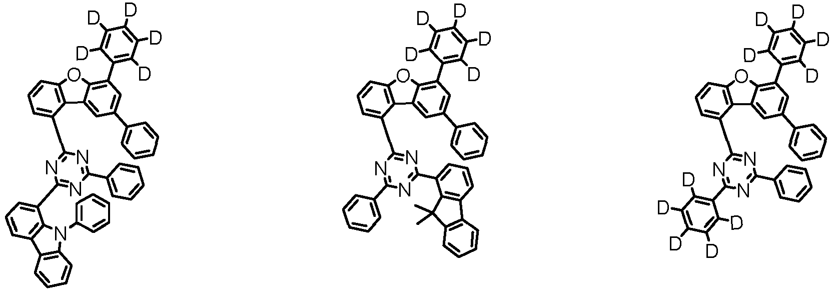

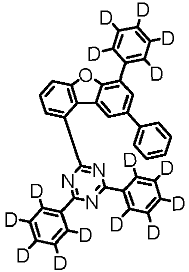

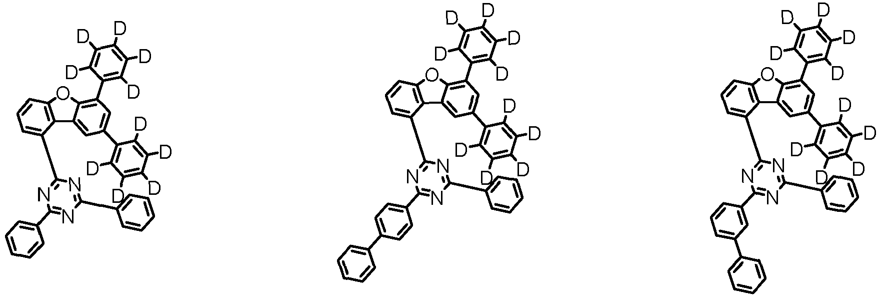

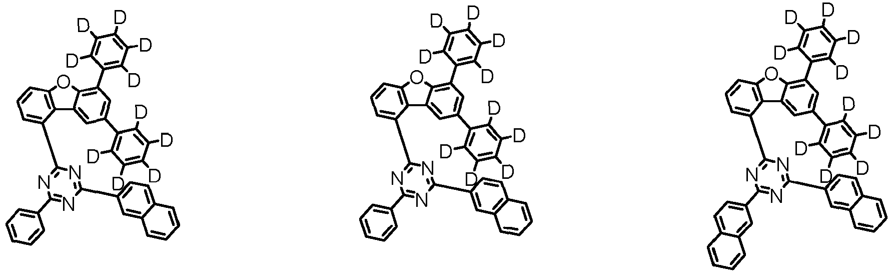

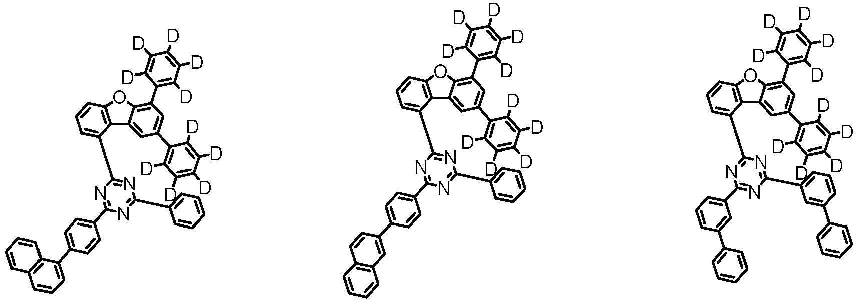

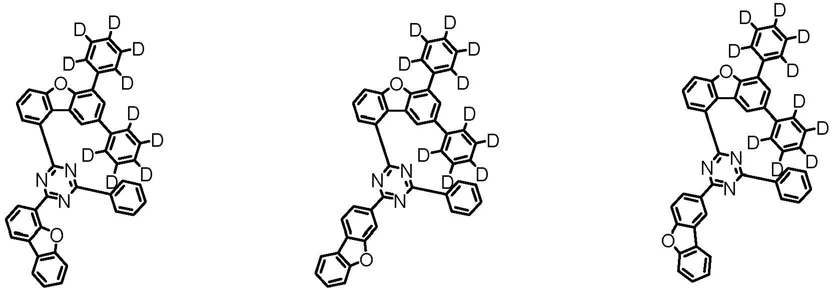

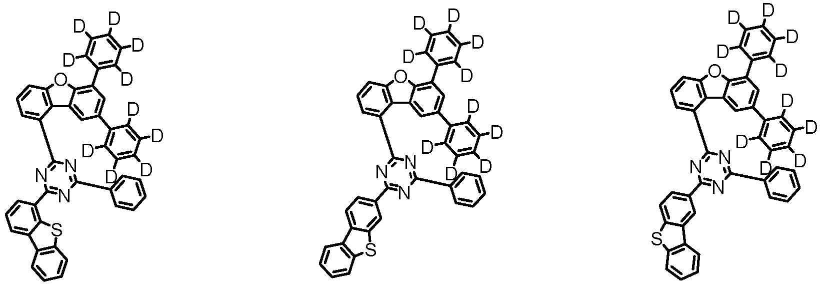

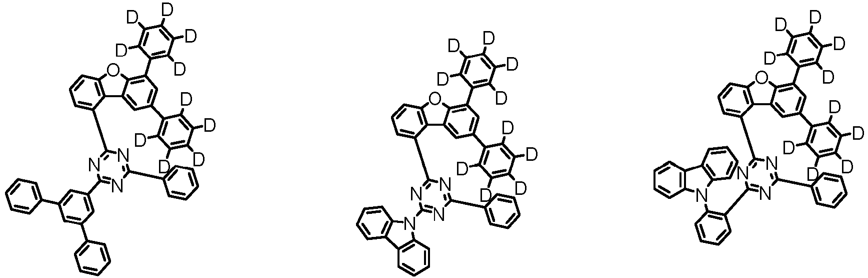

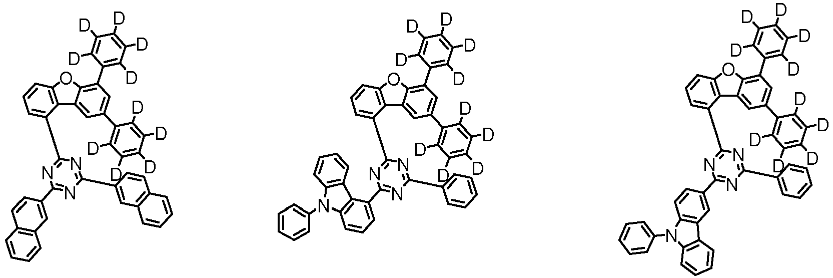

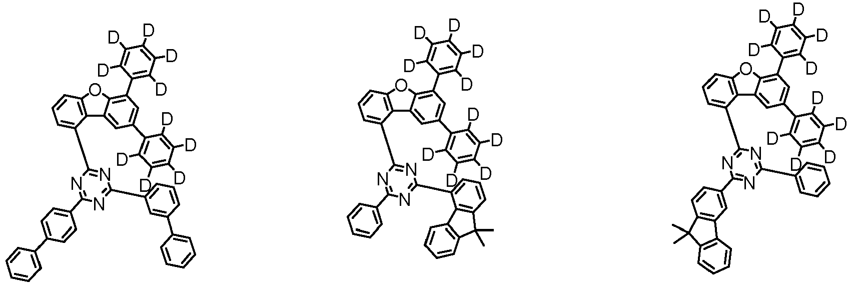

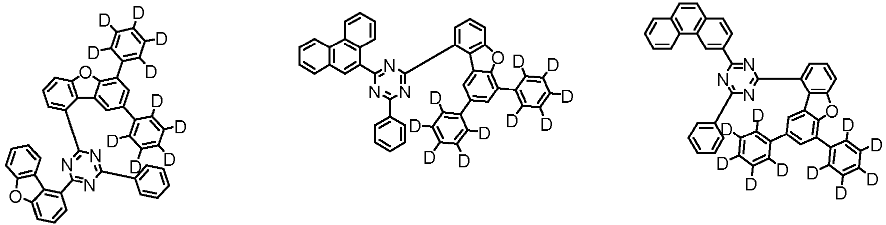

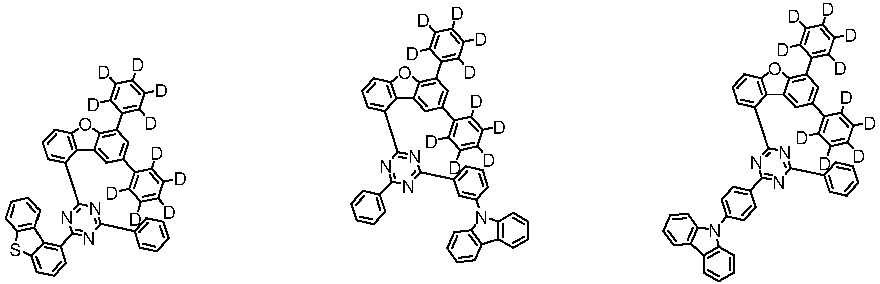

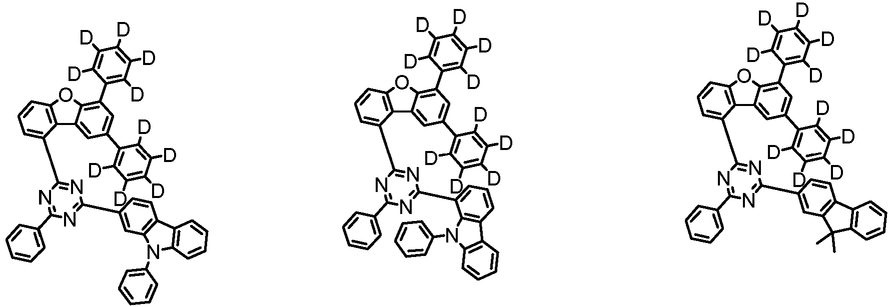

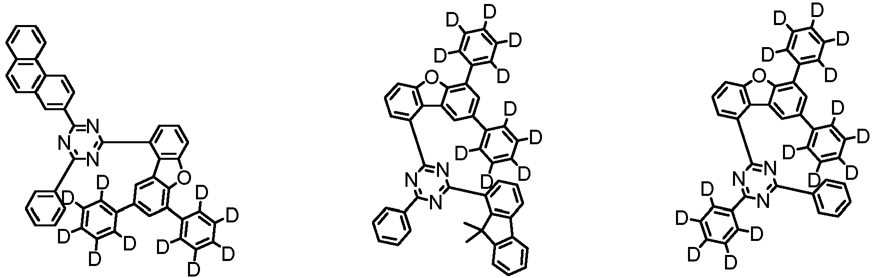

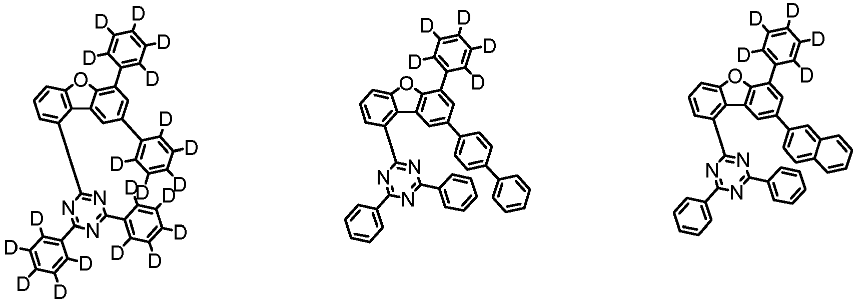

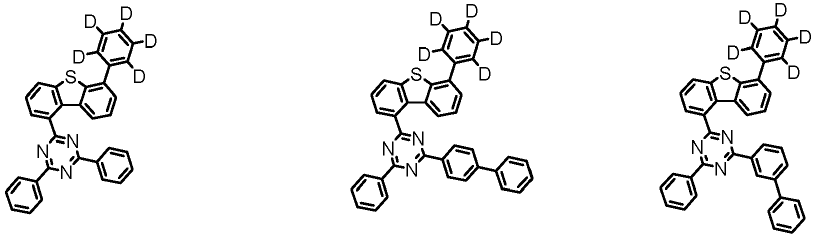

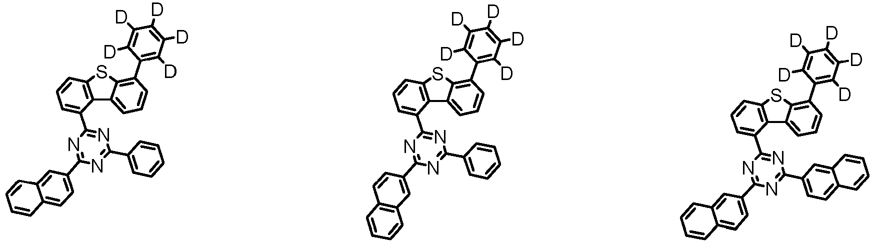

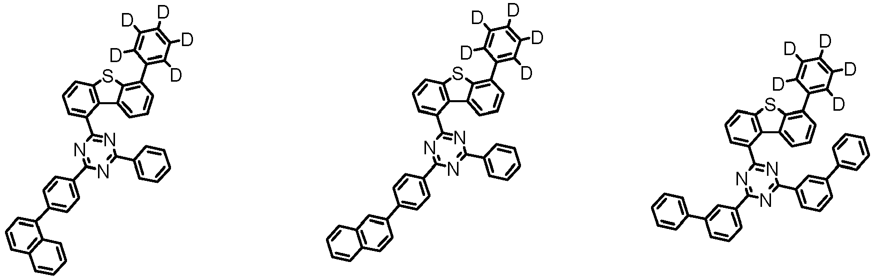

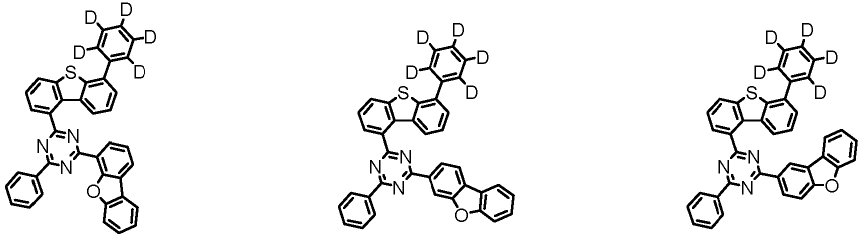

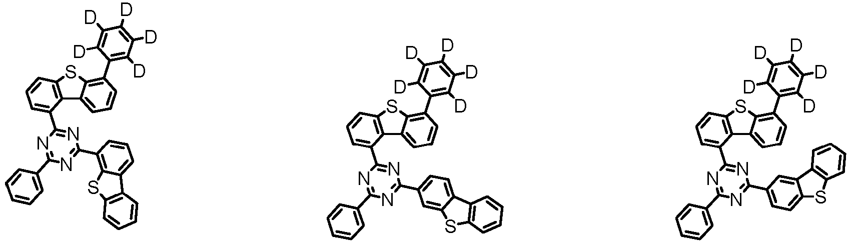

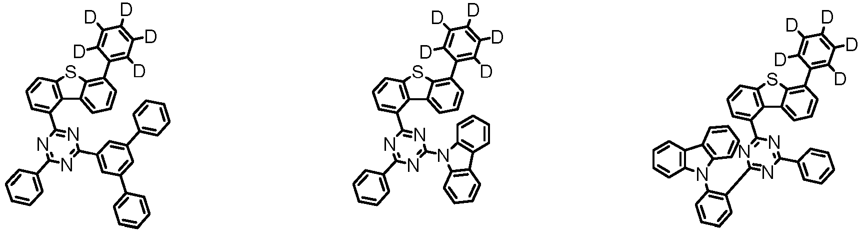

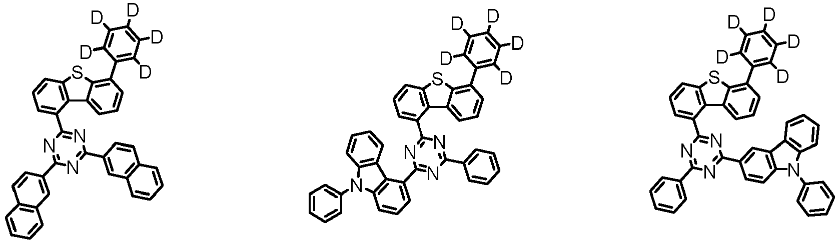

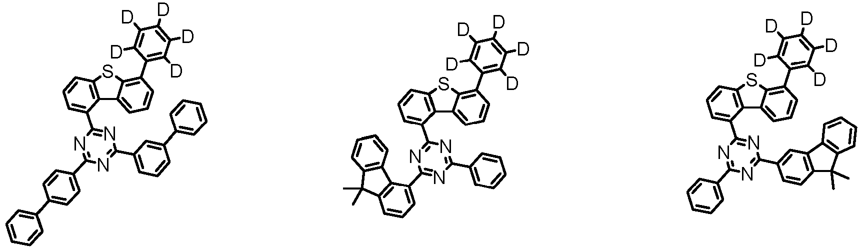

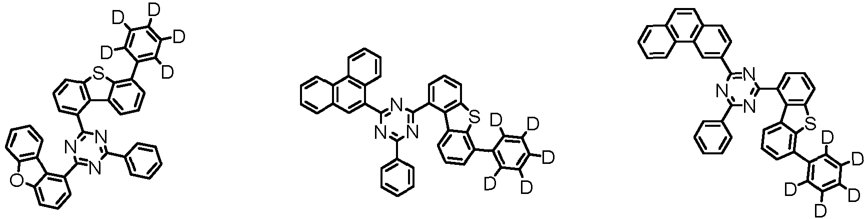

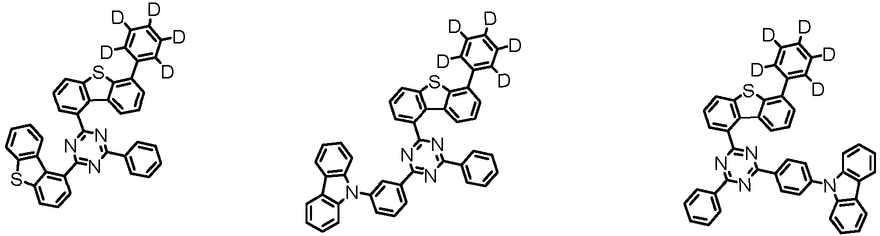

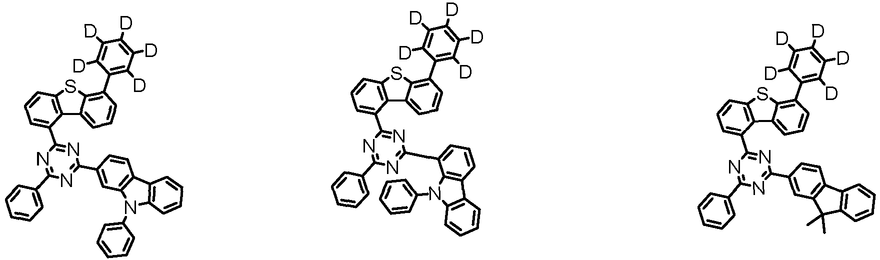

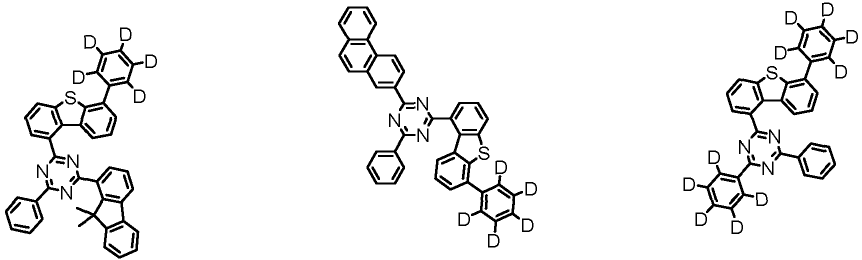

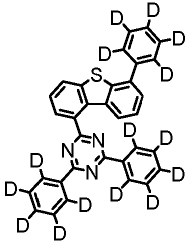

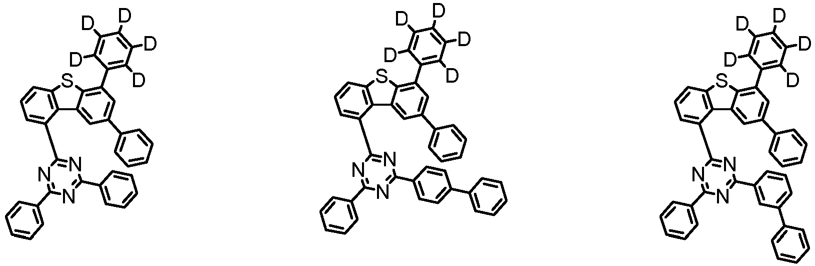

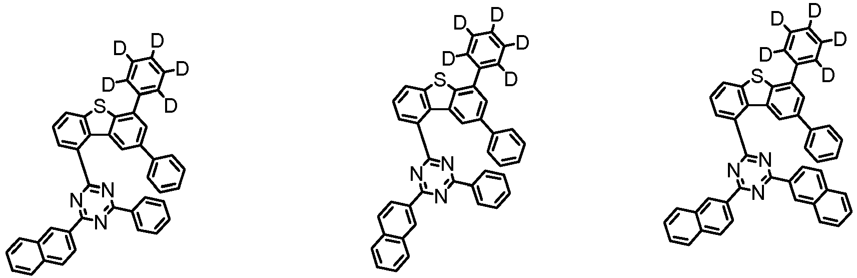

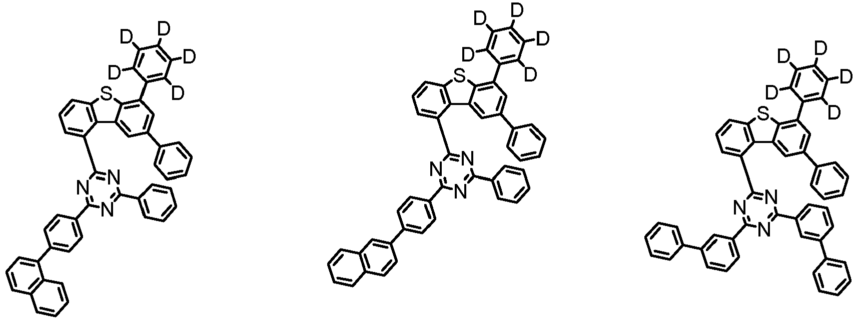

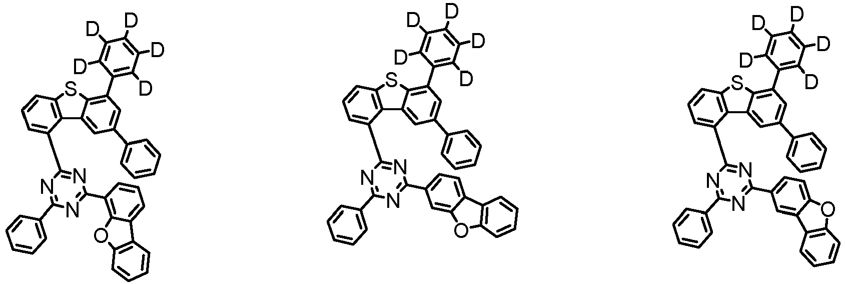

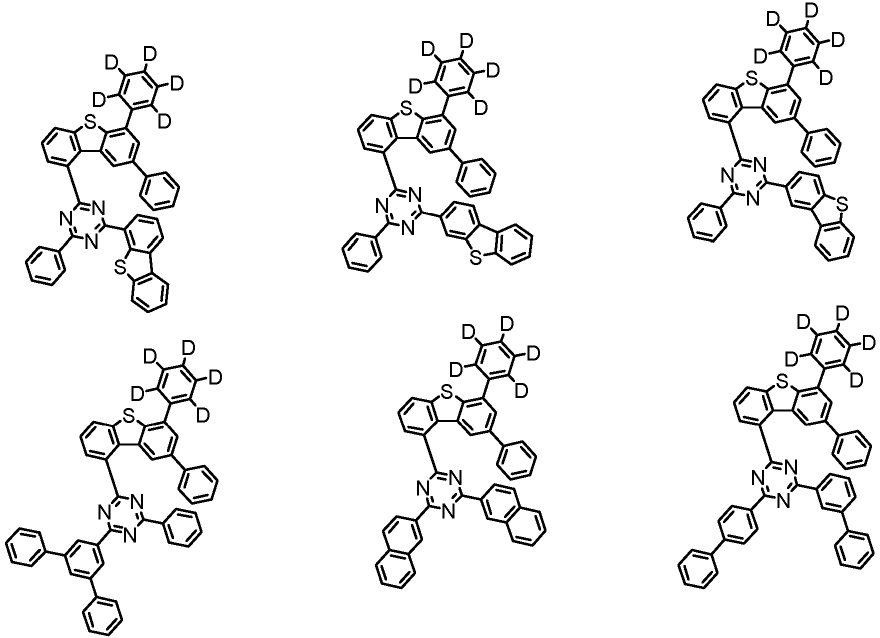

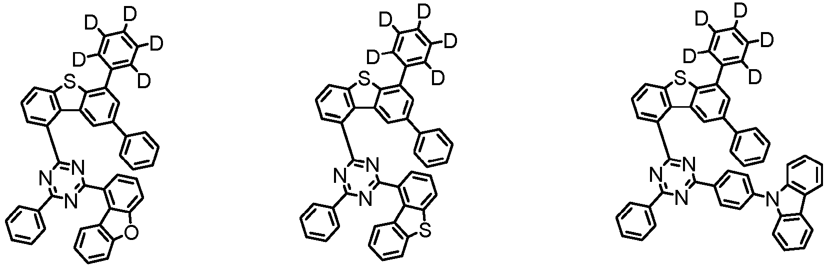

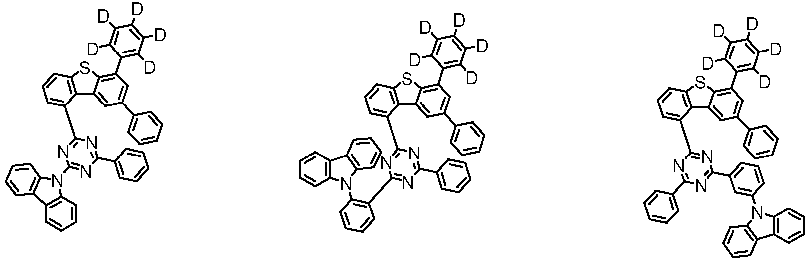

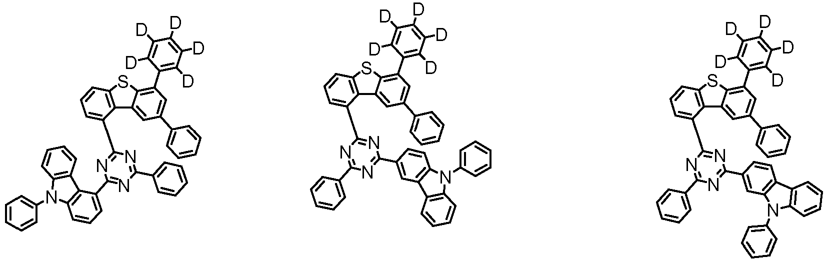

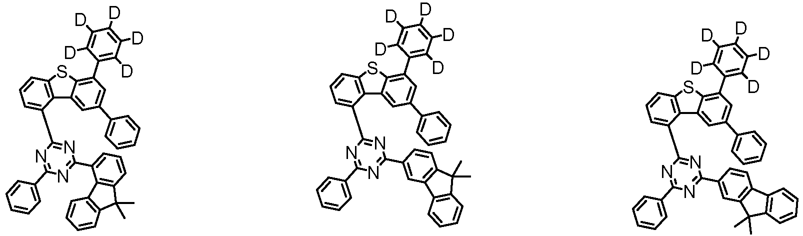

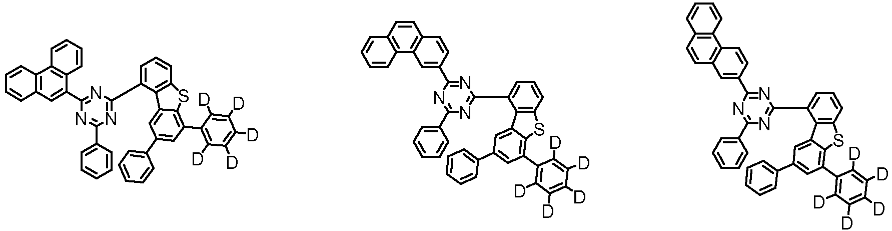

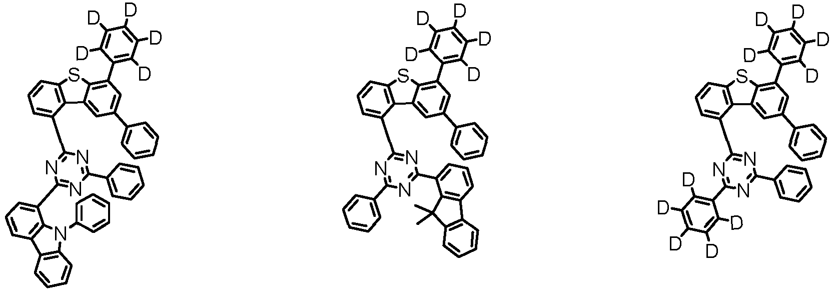

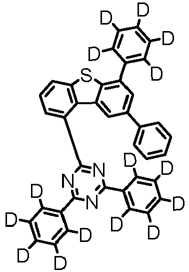

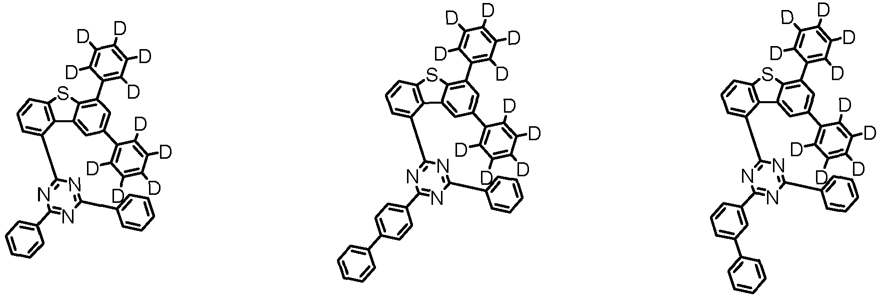

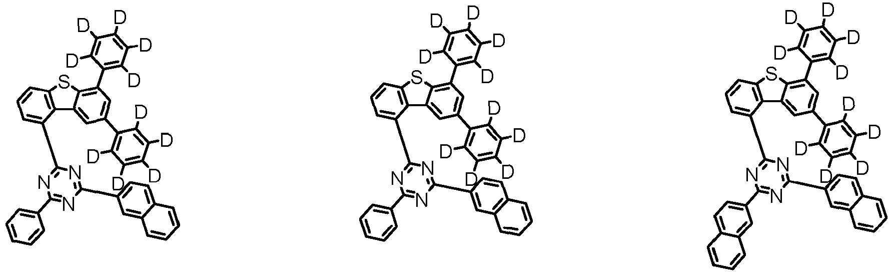

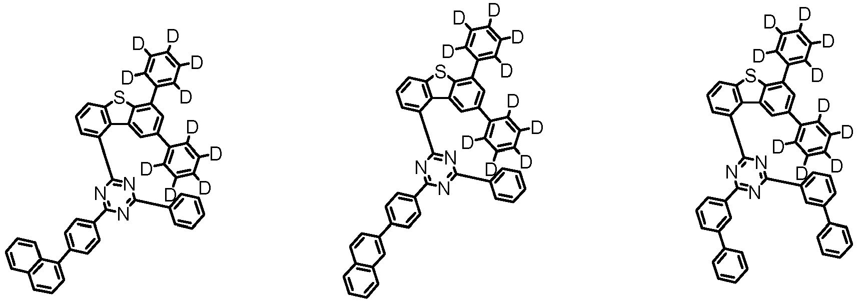

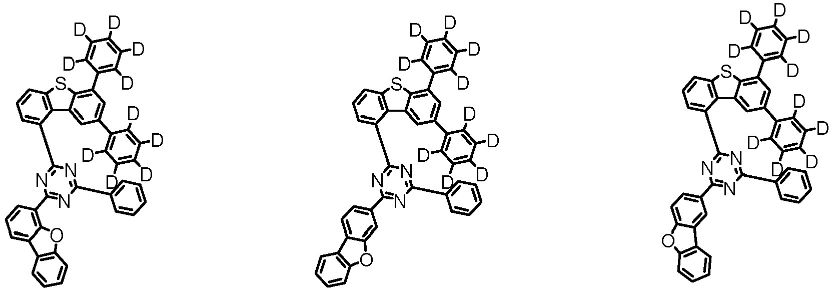

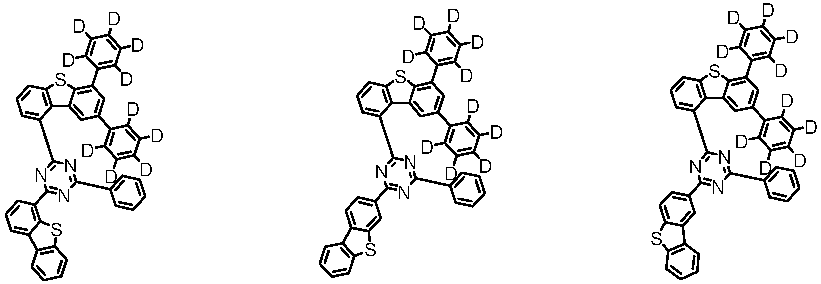

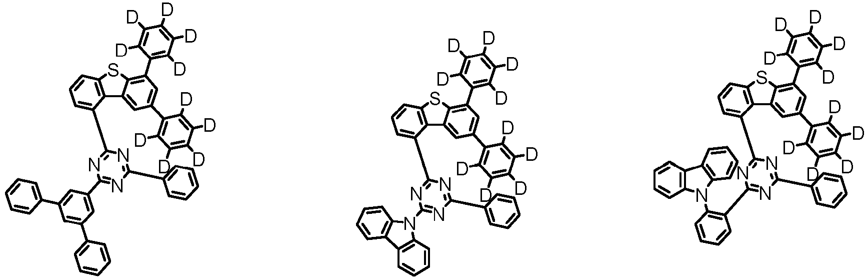

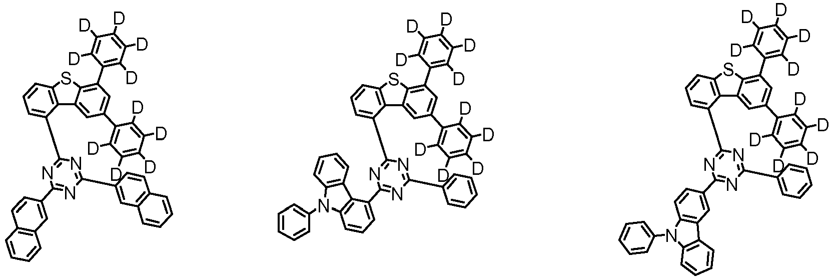

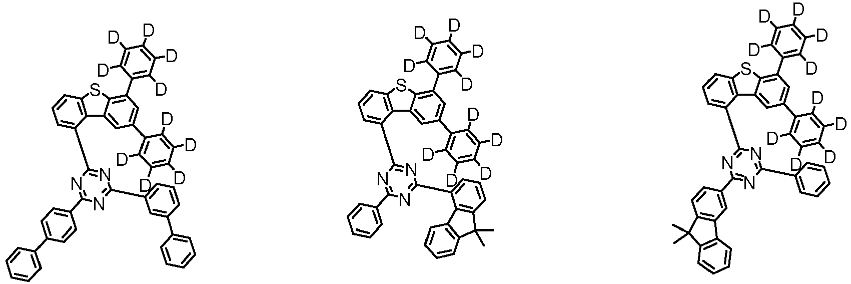

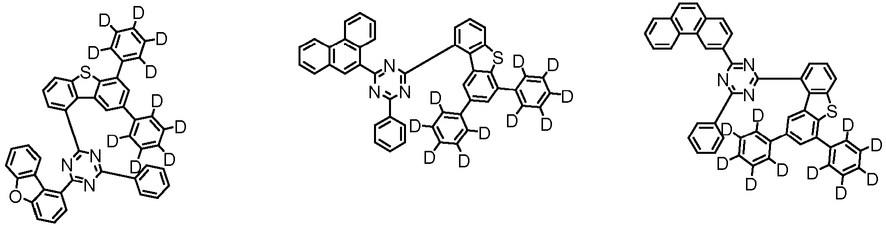

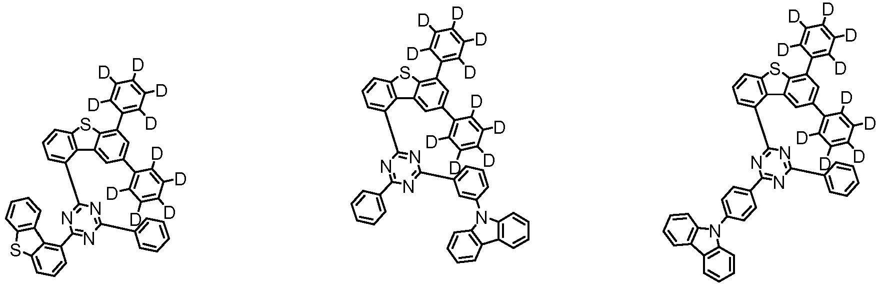

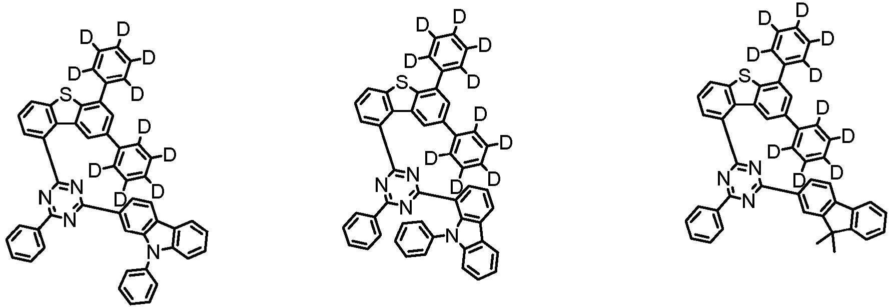

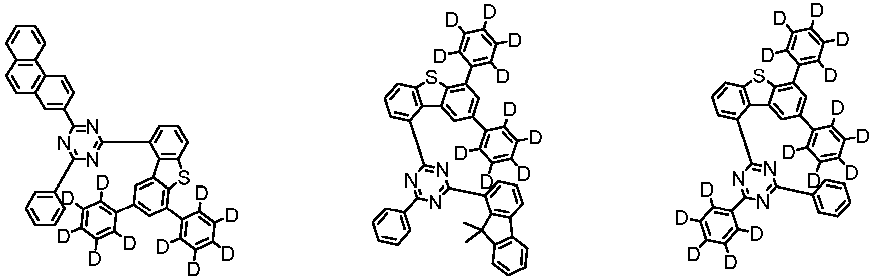

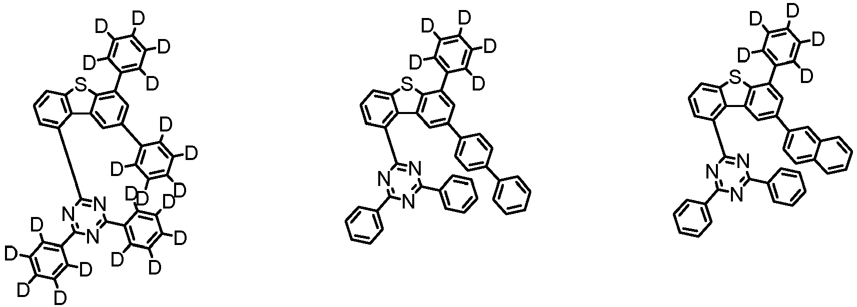

Meanwhile, the compound represented by the Chemical Formula 1 may be prepared by, for example, a preparation method as shown in Reaction Scheme 1 below. 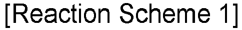 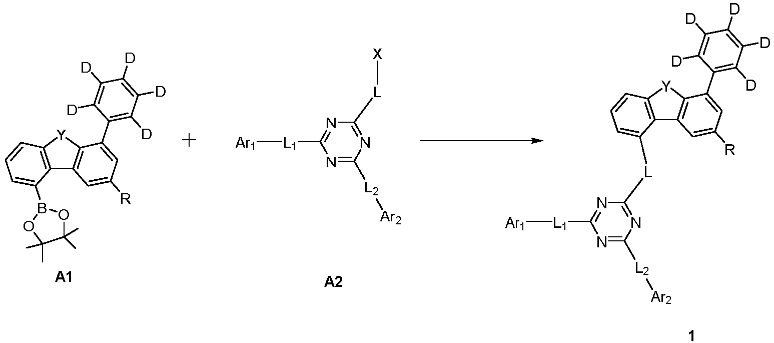 in the Reaction Scheme 1, X is halogen, preferably bromo, or chloro, and the definitions of other substituents are the same as described above.

Specifically, the compound represented by the Chemical Formula 1 may be prepared by Suzuki-coupling reaction of reactants A1 and A2. The Suzuki-coupling reaction is preferably performed in the presence of a palladium catalyst and a base, and the reactive group for the Suzuki-coupling reaction may be appropriately changed. The preparation method may be more specifically described in Synthesis Examples described below.

### (Organic light emitting device)

Meanwhile, according to another aspect of the present disclosure, there is provided an organic light emitting device comprising the above-mentioned compound represented by the Chemical Formula 1. As an example, there is provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure including a hole injection layer, a hole transport layer, a light emitting layer, an electron transport layer, an electron injection layer, and the like as the organic material layer. However, the structure of the organic light emitting device is not limited thereto, and it may include a smaller number of organic material layers.

In one embodiment, the organic material layer may include a light emitting layer, wherein the organic material layer including the above compound may be a light emitting layer.

In another embodiment, the organic material layer may include a hole injection layer, a hole transport layer, a light emitting layer, an electron transport layer and an electron injection layer, wherein the organic material layer including the above compound may be a light emitting layer or an electron transport layer.

In another embodiment, the organic material layer may include a hole injection layer, a hole transport layer, an electron blocking layer, a light emitting layer, an electron transport layer and an electron injection layer, wherein the organic material layer including the above compound may be a light emitting layer or an electron transport layer.

In another embodiment, the organic material layer may include a hole injection layer, a hole transport layer, an electron blocking layer, a light emitting layer, a hole blocking layer, an electron transport layer and an electron injection layer, wherein the organic material layer including the above compound may be a light emitting layer or an electron transport layer.

The organic material layer of the organic light emitting device of the present disclosure may have a single-layer structure, or it may have a multilayered structure in which two or more organic material layers are stacked. For example, the organic light emitting device of the present disclosure may have a structure further including a hole injection layer and a hole transport layer provided between the first electrode and the light emitting layer, and an electron transport layer and an electron injection layer provided between the light emitting layer and the second electrode, in addition to the light emitting layer. However, the structure of the organic light emitting device is not limited thereto, and it may include a smaller number of organic layers or a larger number of organic material layers.

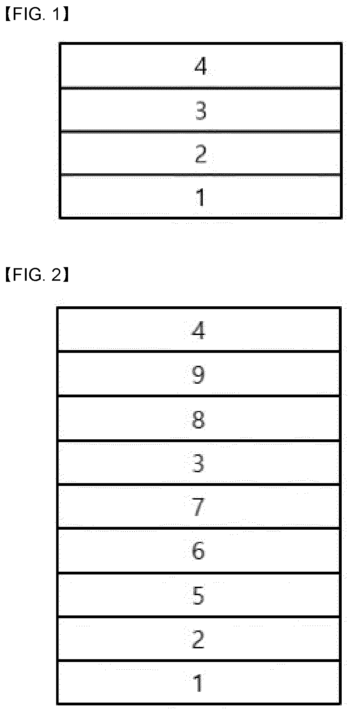

Further, the organic light emitting device according to the present disclosure may be a normal type organic light emitting device in which an anode, one or more organic material layers and a cathode are sequentially stacked on a substrate when the first electrode is an anode and the second electrode is a cathode. Further, the organic light emitting device according to the present disclosure may be an inverted type organic light emitting device in which a cathode, one or more organic material layers and an anode are sequentially stacked on a substrate when the first electrode is a cathode and the second electrode is an anode. For example, the structure of an organic light emitting device according to an embodiment of the present disclosure is illustrated in FIGS. 1 and 2.

FIG. 1 shows an example of an organic light emitting device including a substrate 1, an anode 2, a light emitting layer 3, and a cathode 4. In such a structure, the compound represented by the Chemical Formula 1 may be included in the light emitting layer.

FIG. 2 shows an example of an organic light emitting device including a substrate 1, an anode 2, a hole injection layer 5, a hole transport layer 6, an electron blocking layer 7, a light emitting layer 3, an electron transport layer 8, an electron injection layer 9, and a cathode 4. In such a structure, the compound represented by the Chemical Formula 1 may be included in the light emitting layer.

The organic light emitting device according to the present disclosure may be manufactured using materials and methods known in the art, except that at least one layer of the organic material layers includes the compound represented by Chemical Formula 1. Moreover, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device may be manufactured by sequentially depositing a cathode material, an organic material layer and an anode material on a substrate.

Further, the compound represented by Chemical Formula 1 may be formed into an organic material layer by a solution coating method as well as a vacuum deposition method at the time of manufacturing an organic light emitting device. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

For example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

In addition, the hole injection layer is a layer for injecting holes from the electrode, and the hole injection material is preferably a compound which has a capability of transporting the holes, thus has a hole injecting effect in the anode and an excellent hole-injecting effect to the light emitting layer or the light emitting material, prevents excitons produced in the light emitting layer from moving to an electron injection layer or the electron injection material, and is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

In addition, the hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

In addition, the electron blocking layer is a layer which is formed on the hole transport layer, is preferably provided in contact with the light emitting layer, and thus serves to control hole mobility, to prevent excessive movement of electrons, and to increase the probability of hole-electron bonding, thereby improving the efficiency of the organic light emitting device. The electron blocking layer includes an electron blocking material, and an arylamine-based organic material may be used as the electron blocking material, but is not limited thereto.

In addition, the light emitting layer may include a host material and a dopant material. As the host material, the compound represented by the Chemical Formula 1 may be used. In addition, the host material may further include a fused aromatic ring derivative or a heterocycle-containing compound in addition to the compound represented by the Chemical Formula 1. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

In one embodiment, the light emitting layer may further include a compound represented by the following Chemical Formula 2 in addition to the compound represented by the Chemical Formula 1: 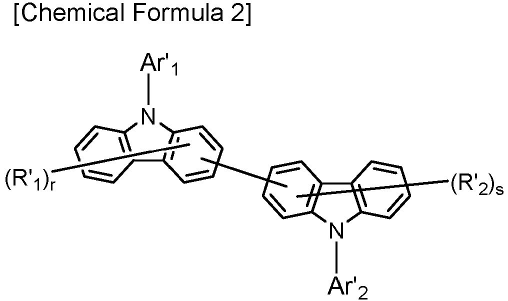 in the Chemical Formula 2,
Ar'₁ and Ar'₂ are each independently substituted or unsubstituted C₆₋₆₀ aryl; or substituted or unsubstituted C₂₋₆₀ heteroaryl containing at least one heteroatom selected from the group consisting of N, O and S, and
R'₁ and R'₂ are each independently hydrogen; deuterium; C₁₋₆₀ alkyl; C₆₋₆₀ aryl; or C₂₋₆₀ heteroaryl containing at least one heteroatom selected from the group consisting of N, O and S; and
r and s are each independently an integer of 0 to 7.

When the organic light emitting device further includes a compound represented by the Chemical Formula 2 capable of efficiently transferring holes to the dopant material as a host material of the light emitting layer, the probability of hole-electron recombination in the light emitting layer together with the compound represented by the Chemical Formula 1 may increase, thereby improving the efficiency and lifespan of the organic light emitting device.

According to one embodiment, the compound represented by the Chemical Formula 2 may be represented by the Chemical Formula 2': 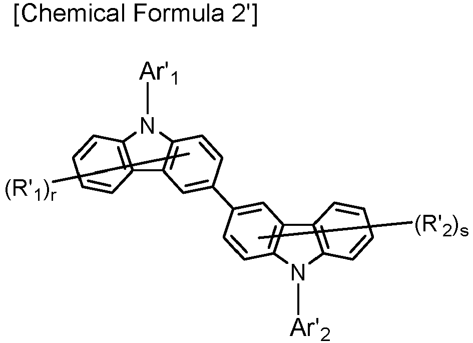 in the Chemical Formula 2',
Ar'₁, Ar'₂, R'₁, R'₂, r and s are as defined in the Chemical Formula 2.

In addition, in the Chemical Formula 2, Ar'₁ and Ar'₂ may each independently be C₆₋₂₀ aryl; or C₂₋₂₀ heteroaryl containing at least one heteroatom selected from the group consisting of N, O and S,
wherein Ar'₁ may be unsubstituted or substituted with at least one substituent selected from the group consisting of deuterium and C₆₋₂₀ aryl.

For example, Ar'₁ and Ar'₂ may each independently be phenyl, biphenylyl, terphenylyl, naphthyl, dibenzofuranyl, or dibenzothiophenyl,
wherein Ar'₁ may be unsubstituted or substituted with at least one substituent selected from the group consisting of deuterium and C₆₋₂₀ aryl.

Herein, at least one of Ar'₁ and Ar'₂ may be phenyl or biphenylyl.

In addition, in the Chemical Formula 2, R'₁ and R'₂ may each independently be hydrogen, deuterium, or C₆₋₂₀ aryl.

For example, R'₁ and R'₂ may each independently be hydrogen, deuterium, or phenyl, but are not limited thereto.

In addition, r and s, each representing the number of R'₁ and R'₂, may independently be 0, 1, 2, 3, 4, 5, 6, or 7.

More specifically, r and s may each independently be 0, 1, or 7.

For example, r+s may be 0 or 1.

Representative examples of the compound represented by the Chemical Formula 2 are as follows:

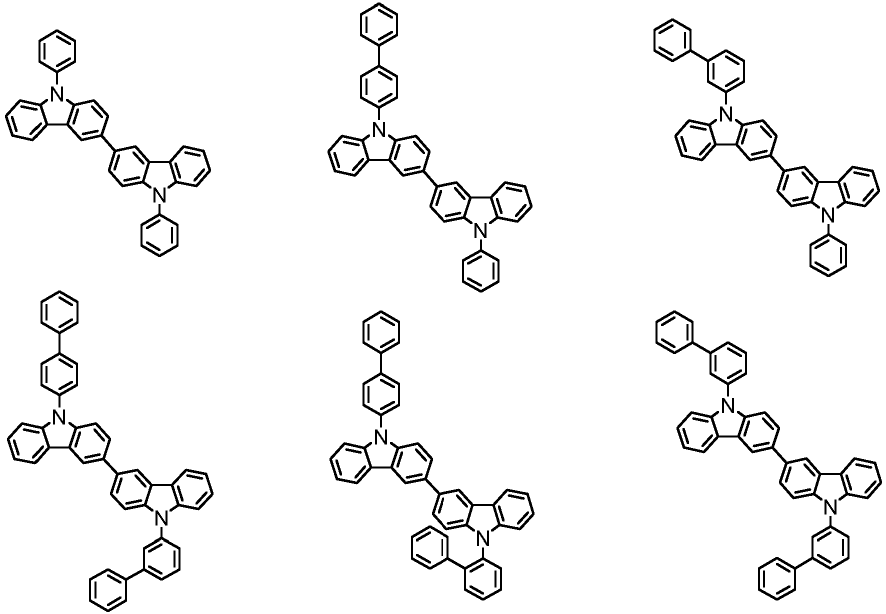

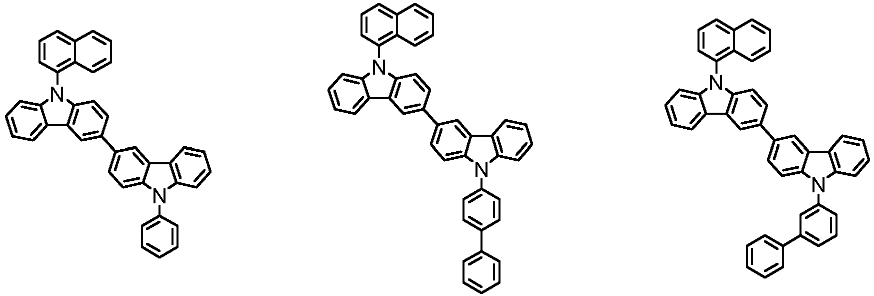

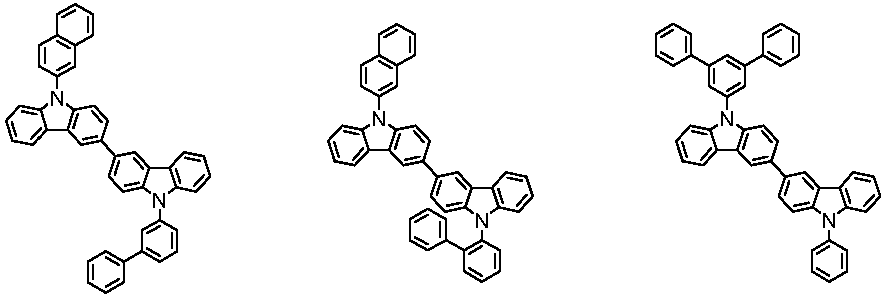

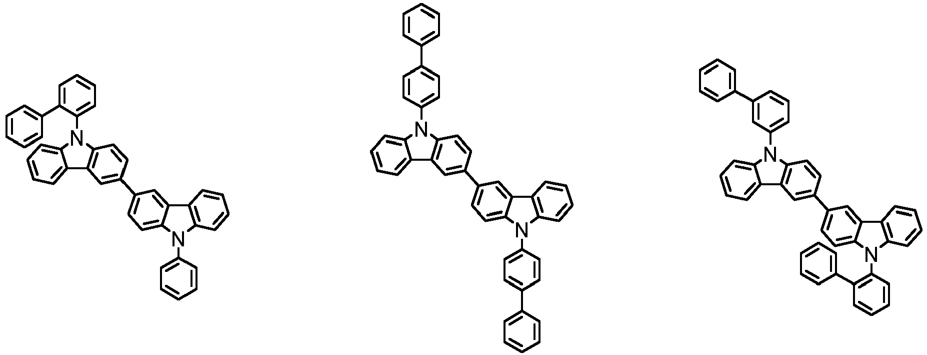

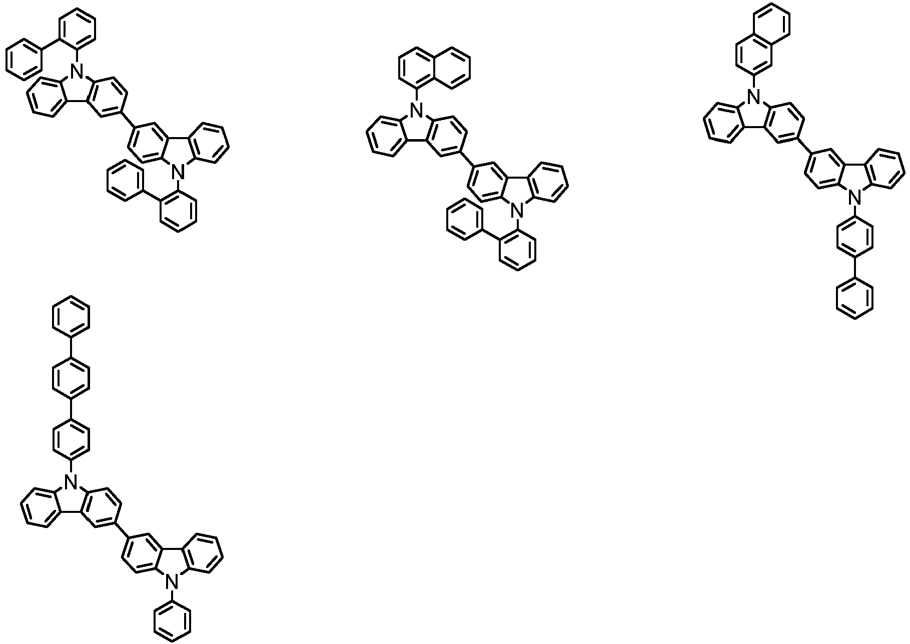

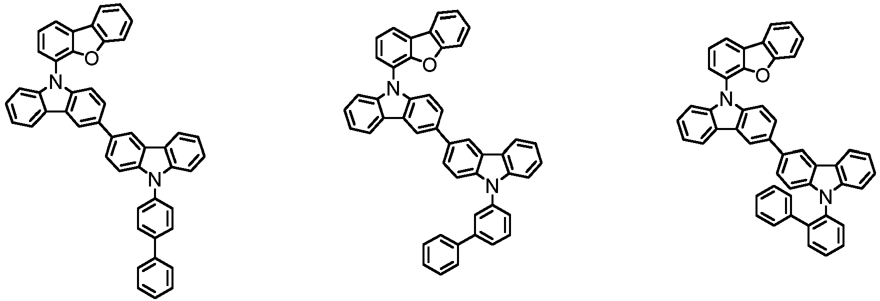

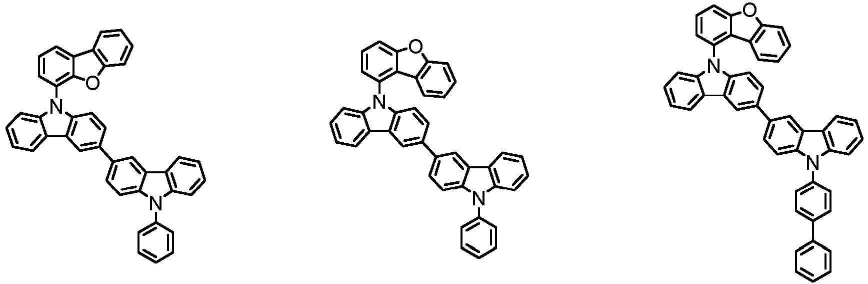

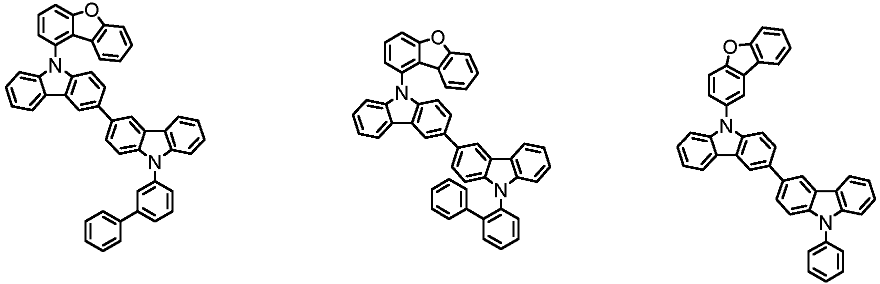

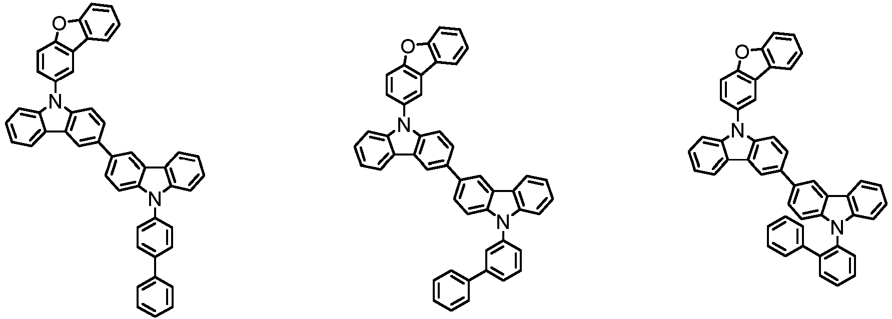

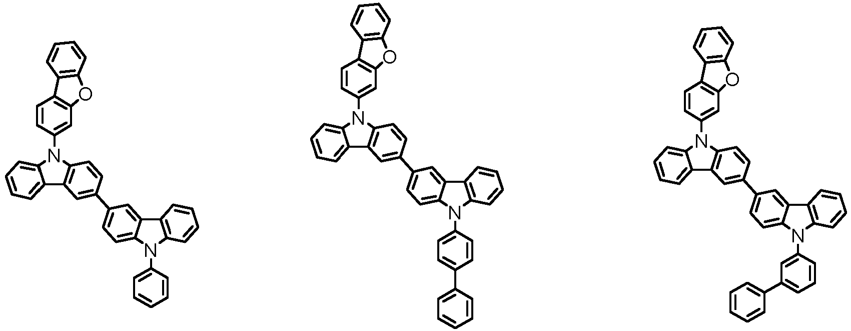

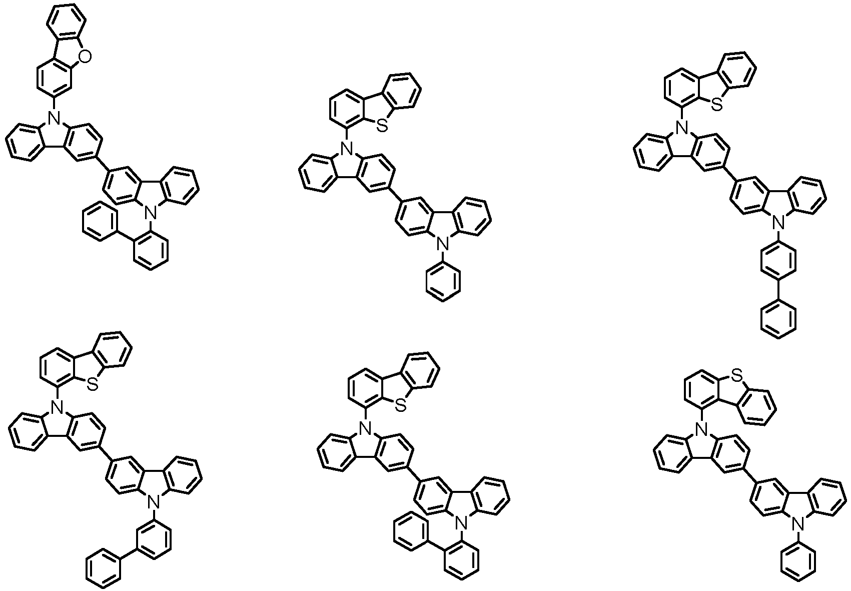

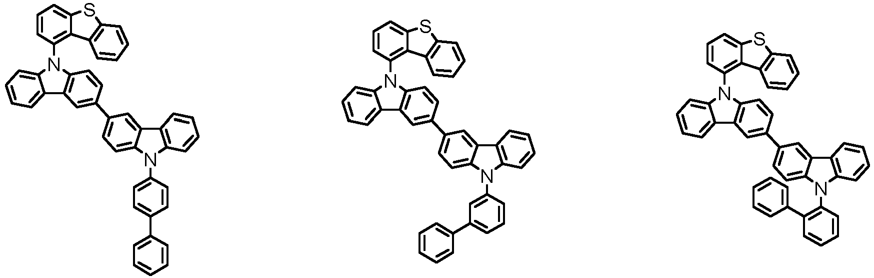

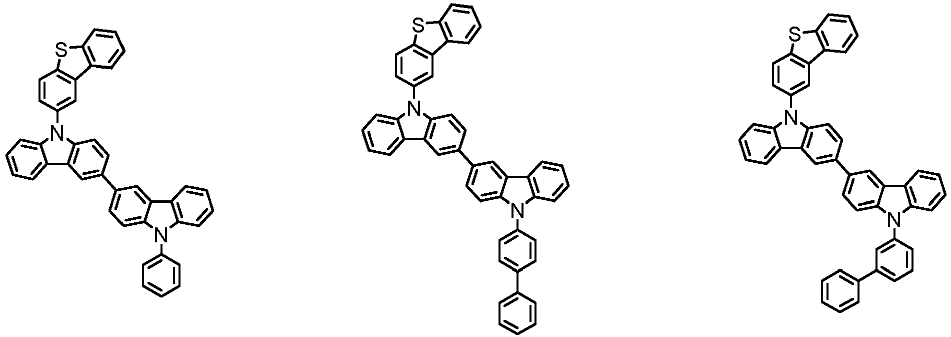

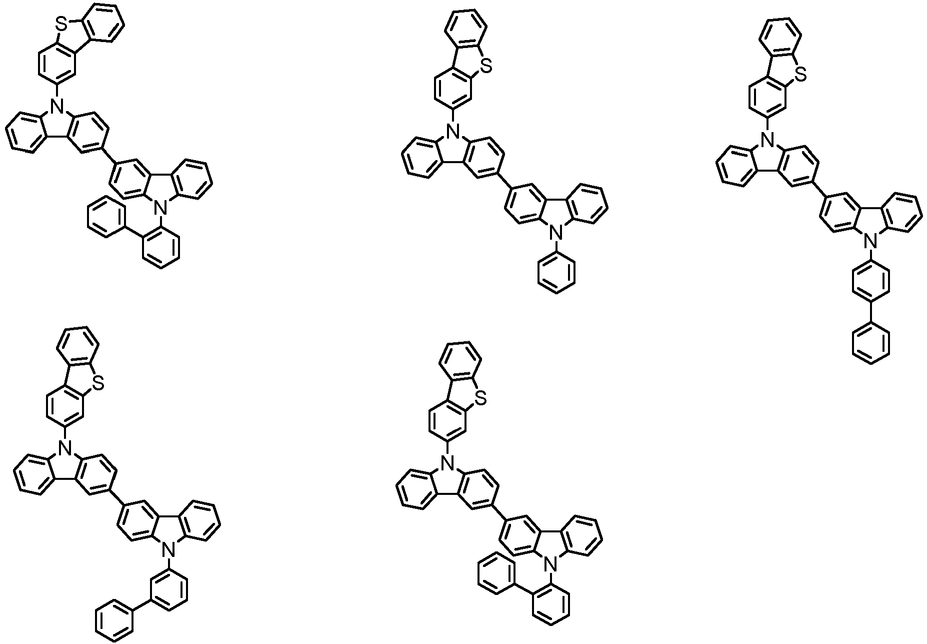

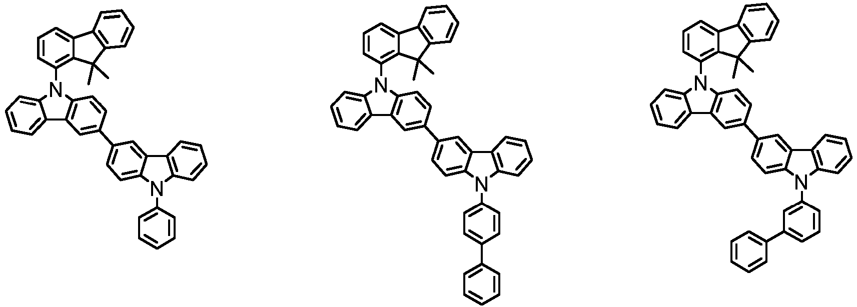

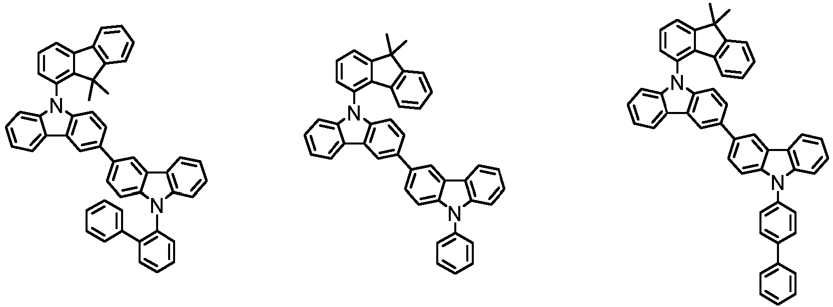

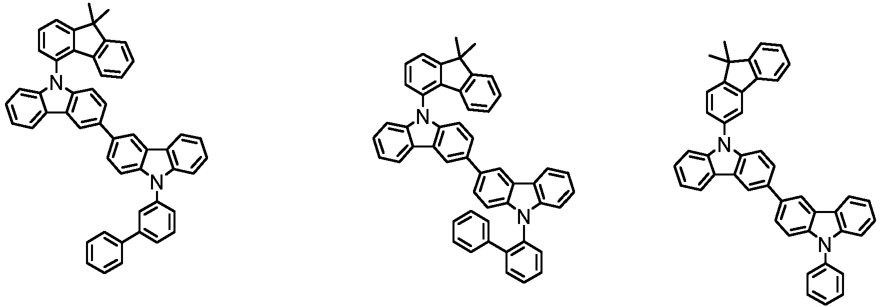

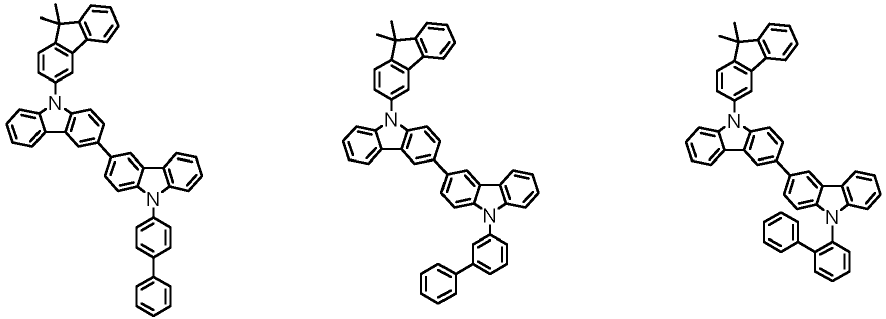

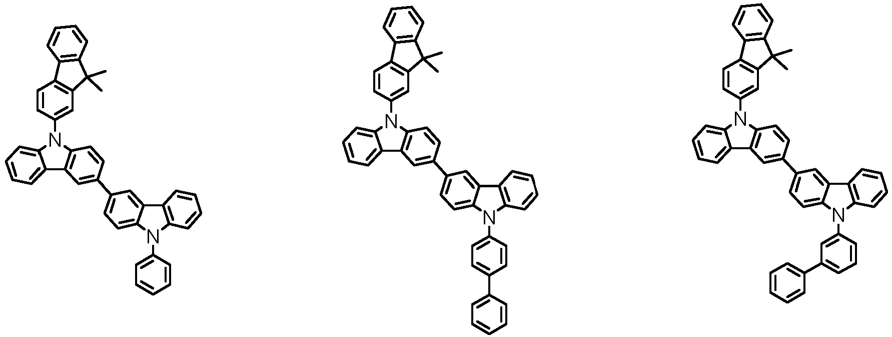

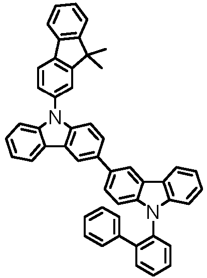

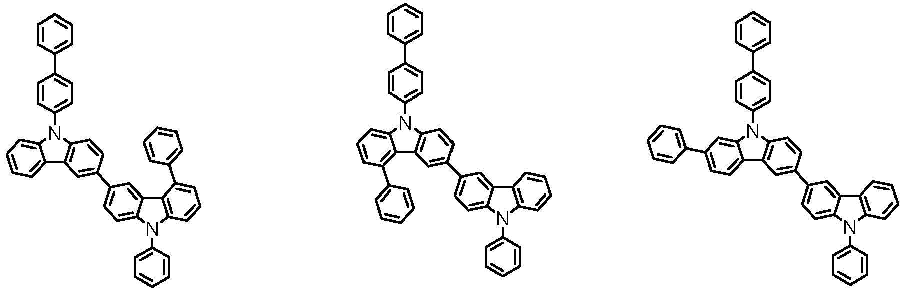

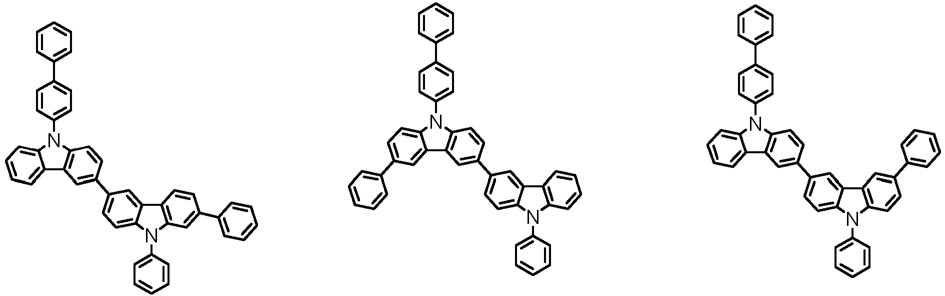

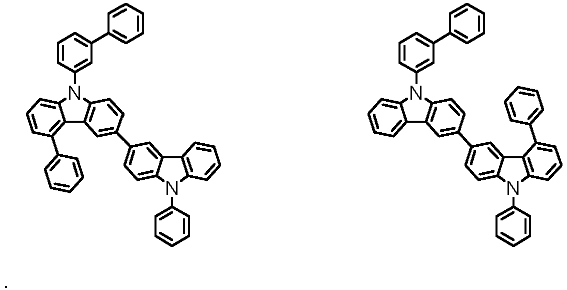

These two host materials, the compound represented by the Chemical Formula 1 and the compound represented by the Chemical Formula 2, may be included in the light emitting layer at a weight ratio of 10:90 to 90:10, for example, 50:50.

The dopant material includes an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

In addition, the hole blocking layer means a layer which is formed on the light emitting layer, is preferably provided in contact with the light emitting layer, and thus serves to control electron mobility, to prevent excessive movement of holes, and to increase the probability of hole-electron bonding, thereby improving the efficiency of the organic light emitting device. The hole blocking layer includes a hole blocking material, and as an example of such a hole blocking material, compounds introduced with electron attracting groups, such as azine-based derivatives including triazine; triazole derivatives; oxadiazole derivatives; phenanthroline derivatives; phosphine oxide derivatives may be used, but is not limited thereto.

In addition, the electron transport layer is a layer which receives electrons from an electron injection layer and transports the electrons to a light emitting layer, and is formed on the light emitting layer or the hole blocking layer. The electron transport layer includes an electron transport material, and a material having large mobility for electrons is suitable. Specific examples thereof include: an Al complex of 8-hydroxyquinoline; a complex including Alq₃; an organic radical compound; a hydroxyflavone-metal complex; a triazine derivative, and the like, but are not limited thereto. Alternatively, it may be used together with fluorenone, anthraquinodimethane, diphenoquinone, thiopyran dioxide, oxazole, oxadiazole, triazole, imidazole, perylenetetracarboxylic acid, fluorenylidene methane, anthrone, or derivatives thereof, a metal complex compound, a nitrogen-containing 5-membered ring derivative, or the like, but are not limited thereto.

In addition, the electron injection layer is a layer which injects electrons from an electrode, and is formed on the electron transport layer. Specific examples of the electron injection material included in the electron injection layer may include LiF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-hydroxyquinolinato)chlorogallium, bis(2-methyl-8-hydroxyquinolinato)(o-cresolato)gallium, bis(2-methyl-8-hydroxyquinolinato)(1-naphtholato)aluminum, bis(2-methyl-8-hydroxyquinolinato)(2-naphtholato)gallium, and the like, but are not limited thereto.

The organic light emitting device according to the present disclosure may be a bottom emission device, a top emission device, or a double-sided emission device, and in particular, may be a bottom emission device requiring relatively high luminous efficiency.

In addition, the compound represented by Chemical Formula 1 may be included in an organic solar cell or an organic transistor in addition to an organic light emitting device.

The preparation of the compound represented by the Chemical Formula 1 and the organic light emitting device including the same will be described in detail in the following examples. However, these examples are presented for illustrative purposes only, and are not intended to limit the scope of the present disclosure.

### Preparation Example 1: Preparation of Compound A-4

### 1) Preparation of Compound A-1

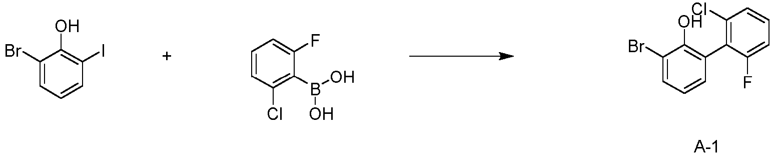

2-bromo-6-iodophenol (100 g, 336.1 mmol) and (2-chloro-6-fluorophenyl)boronic acid (58.5 g, 336.1 mmol) were added to 2000 ml of tetrahydrofuran under a nitrogen atmosphere, and the mixture was stirred and refluxed. Then, potassium carbonate (139.4 g, 1008.4 mmol) was dissolved in 139 ml of water, and then added thereto. Thereafter, it was stirred sufficiently, followed by adding tetrakis(triphenylphosphino)palladium (11.6 g, 10.1 mmol). After 2 hours of reaction, it was cooled to room temperature and the resulting solid was filtered. Then, the resulting solid was dissolved in 5041 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound A-1 in the form of white solid (59.5g, 59%, MS: [M+H]⁺ = 300.9).

### 2) Preparation of Compound A-2

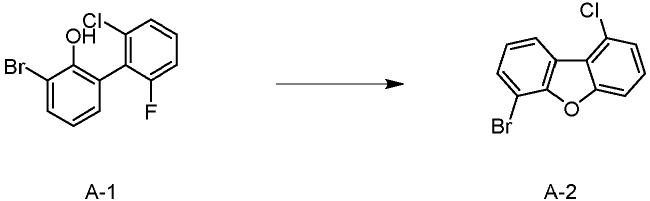

A-1 (50 g, 166.7 mmol) was added to 250 mL of dimethylformamide, followed by adding potassium carbonate. Then, the mixture was stirred and heated to 140 °C. After 3 hours of reaction, it was cooled to room temperature and then water was added thereto. And, the resulting solid was filtered. Then, the resulting solid was dissolved again in 497 mL of chloroform, and washed twice with water. Thereafter, the organic layer was separated, treated with anhydrous magnesium sulfate, stirred, then filtered, and the filtrate was distilled under reduced pressure. The concentrated compound was purified by silica column using chloroform and ethyl acetate to prepare Compound A-2 in the form of white solid (29.3 g, 59%, MS: [M+H]⁺ = 298.9).

### 3) Preparation of Compound A-3

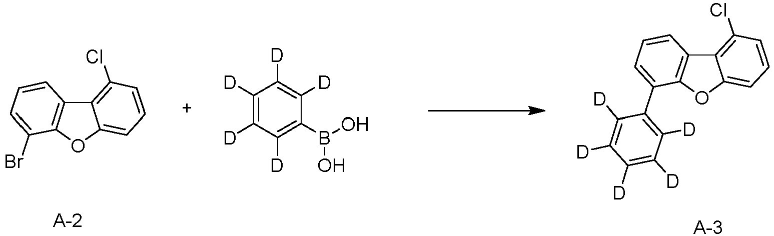

A-2 (50 g, 178.6 mmol) and phenylboronic acid-D5 (22.7 g, 178.6 mmol) were added to 1000 ml of tetrahydrofuran under a nitrogen atmosphere, and the mixture was stirred and refluxed. Then, potassium carbonate (74.1 g, 535.8 mmol) was dissolved in 74 ml of water, and then added thereto. Thereafter, it was stirred sufficiently, followed by adding tetrakis(triphenylphosphino)palladium (6.2 g, 5.4 mmol). After 3 hours of reaction, cooling was performed to room temperature. Then, the organic layer was separated from the water layer, and then the organic layer was distilled. Then, this was dissolved again in 1011 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound A-3 in the form of yellow solid (26.3 g, 52%, MS: [M+H]⁺ = 284.1).

### 4) Preparation of Compound A-4

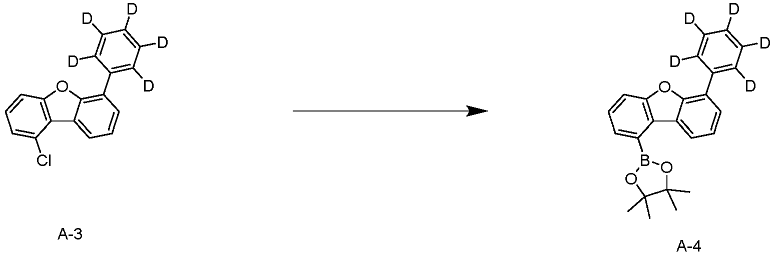

A-3 (50 g, 139.2 mmol) and bis(pinacolato)diboron (38.9 g, 153.2 mmol) were added to 1000 ml of 1,4-dioxane under a nitrogen atmosphere, and the mixture was stirred and refluxed. Then, potassium acetate (40.1 g, 417.7 mmol) was added thereto and stirred sufficiently, followed by adding palladium dibenzylideneacetone palladium (2.4 g, 4.2 mmol) and tricyclohexylphosphine (2.3 g, 8.4 mmol). After 3 hours of reaction, cooling was performed to room temperature. Thereafter, the organic layer was filtered to remove salt, and then the filtered organic layer was distilled. Then, this was dissolved again in 628 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound A-4 in the form of grey solid (52.8 g, 84%, MS: [M+H]⁺ = 452.2).

### Preparation Example 2: Preparation of Compound B-4

### 1) Preparation of Compound B-2

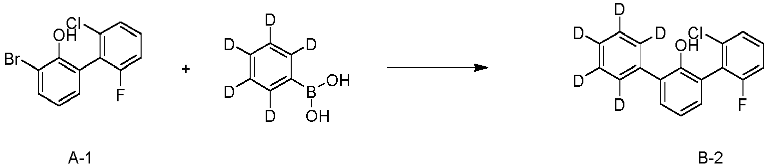

A-1 (50 g, 166.7 mmol) and phenylboronic acid-D5 (21.2 g, 166.7 mmol) were added to 1000 ml of tetrahydrofuran under a nitrogen atmosphere, and the mixture was stirred and refluxed. Then, potassium carbonate (69.1 g, 500.1 mmol) was dissolved in 69 ml of water, and then added thereto. Thereafter, it was stirred sufficiently, followed by adding tetrakis(triphenylphosphino)palladium (5.8 g, 5 mmol). After 3 hours of reaction, cooling was performed to room temperature. Then, the organic layer was separated from the water layer, and then the organic layer was distilled. Then, this was dissolved again in 1011 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound B-2 in the form of yellow solid (26.8 g, 53%, MS: [M+H]⁺ = 304.1).

### 2) Preparation of Compound B-3

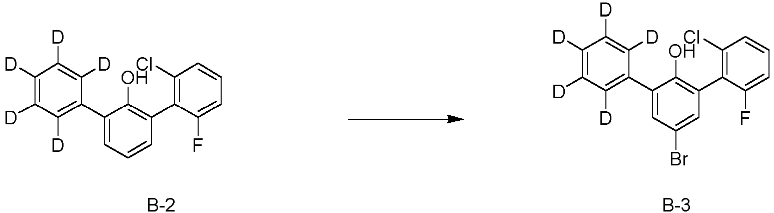

B-2 (50 g, 165 mmol) and N-Bromosuccinimide (32.3 g, 181.5 mmol) were added to 250 mL of dimethylformamide under a nitrogen atmosphere, reacted for 3 hours, and then cooled in an ice bath while adding water. Then, the resulting solid was filtered. Then, this was dissolved again in 596 mL of chloroform, and washed twice with water. Thereafter, the organic layer was separated, treated with anhydrous magnesium sulfate, stirred, then filtered, and the filtrate was distilled under reduced pressure. The concentrated compound was purified by silica column using chloroform and ethyl acetate to prepare Compound B-3 in the form of white solid (37.5 g, 63%, MS: [M+H]⁺ = 362).

### 3) Preparation of Compound B-4

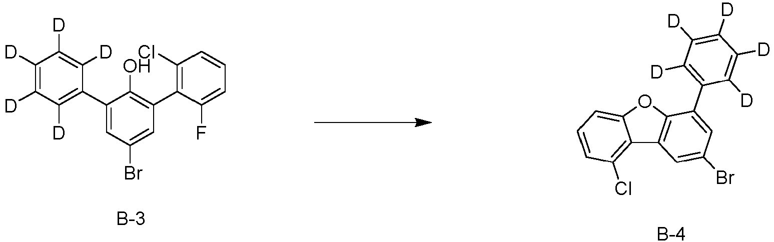

B-3 (50 g, 131.2 mmol) was added to 250 mL of dimethylformamide, followed by adding potassium carbonate. Then, the mixture was stirred and heated to 140 °C. After 4 hours of reaction, it was cooled to room temperature and then water was added thereto. And, the resulting solid was filtered. Then, this was dissolved again in 474 mL of chloroform, and washed twice with water. Thereafter, the organic layer was separated, treated with anhydrous magnesium sulfate, stirred, then filtered, and the filtrate was distilled under reduced pressure. The concentrated compound was purified by silica column using chloroform and ethyl acetate to prepare Compound B-4 in the form of white solid (33.2 g, 70%, MS: [M+H]⁺ = 362).

### Preparation Example 3: Preparation of Compound C-2

### 1) Preparation of Compound C-1

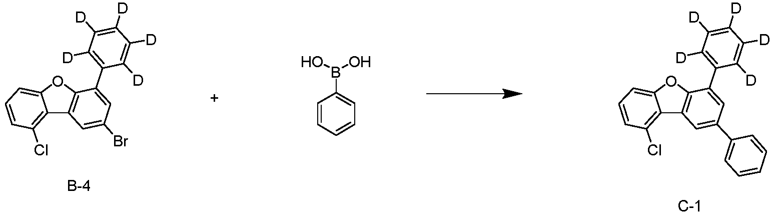

**B-4** (50 g, 138.5 mmol) and phenylboronic acid (16.9 g, 138.5 mmol) were added to 1000 ml of tetrahydrofuran under a nitrogen atmosphere, and the mixture was stirred and refluxed. Then, potassium carbonate (57.4 g, 415.5 mmol) was dissolved in 57 ml of water, and then added thereto. Thereafter, it was stirred sufficiently, followed by adding tetrakis(triphenylphosphino)palladium (4.8 g, 4.2 mmol). After 1 hour of reaction, cooling was performed to room temperature. Then, the organic layer was separated from the water layer, and then the organic layer was distilled. Then, this was dissolved again in 995 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound C-1 in the form of white solid (30.8 g, 62%, MS: [M+H]⁺ = 360.1).

### 2) Preparation of Compound C-2

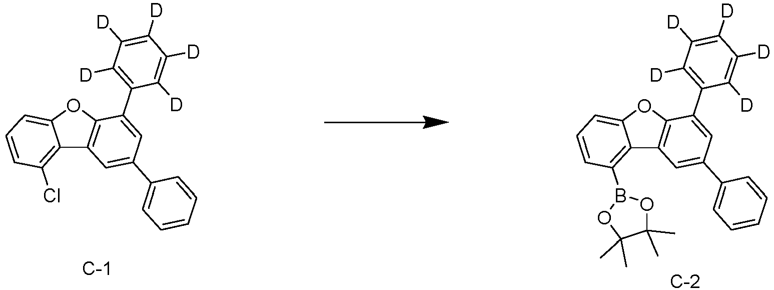

C-1 (50 g, 139.2 mmol) and bis(pinacolato)diboron (38.9 g, 153.2 mmol) were added to 1000 ml of 1,4-dioxane under a nitrogen atmosphere, and the mixture was stirred and refluxed. Then, potassium acetate (40.1 g, 417.7 mmol) was added thereto and stirred sufficiently, followed by adding palladium dibenzylideneacetone palladium (2.4 g, 4.2 mmol) and tricyclohexylphosphine (2.3 g, 8.4 mmol). After 7 hours of reaction, cooling was performed to room temperature. Thereafter, the organic layer was filtered to remove salt, and then the filtered organic layer was distilled. Then, this was dissolved again in 628 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound C-2 in the form of grey solid (50.9 g, 81%, MS: [M+H]⁺ = 452.2).

### Preparation Example 4: Preparation of Compound D-2

### 1) Preparation of Compound D-1

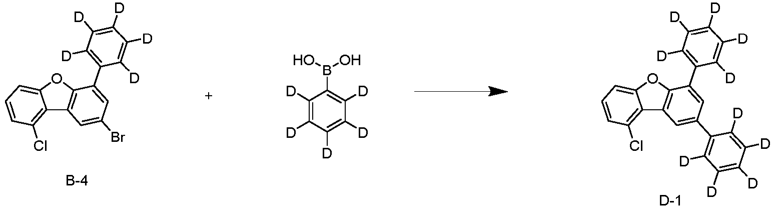

**B-4** (50 g, 138.5 mmol) and phenylboronic acid-D5 (17.6 g, 138.5 mmol) were added to 1000 ml of tetrahydrofuran under a nitrogen atmosphere, and the mixture was stirred and refluxed. Then, potassium carbonate (57.4 g, 415.5 mmol) was dissolved in 57 ml of water, and then added thereto. Thereafter, it was stirred sufficiently, followed by adding tetrakis(triphenylphosphino)palladium (4.8 g, 4.2 mmol). After 1 hour of reaction, cooling was performed to room temperature. Then, the organic layer was separated from the water layer, and then the organic layer was distilled. Then, this was dissolved again in 1009 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound D-1 in the form of yellow solid (29.3 g, 58%, MS: [M+H]⁺ = 365.1).

### 2) Preparation of Compound D-2

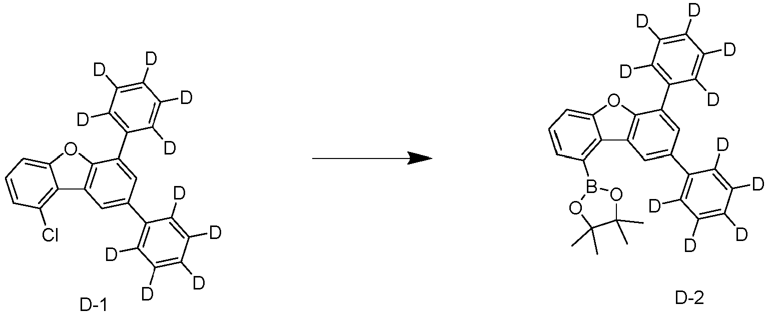

D-1 (50 g, 139.2 mmol) and bis(pinacolato)diboron (38.9 g, 153.2 mmol) were added to 1000 ml of 1,4-dioxane under a nitrogen atmosphere, and the mixture was stirred and refluxed. Then, potassium acetate (40.1 g, 417.7 mmol) was added thereto and stirred sufficiently, followed by adding palladium dibenzylideneacetone palladium (2.4 g, 4.2 mmol) and tricyclohexylphosphine (2.3 g, 8.4 mmol). After 7 hours of reaction, cooling was performed to room temperature. Thereafter, the organic layer was filtered to remove salt, and then the filtered organic layer was distilled. Then, this was dissolved again in 635 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound D-2 in the form of grey solid (50.8 g, 80%, MS: [M+H]⁺ = 457.2).

### Preparation Example 5: Preparation of Compound E-2

### 1) Preparation of Compound E-1

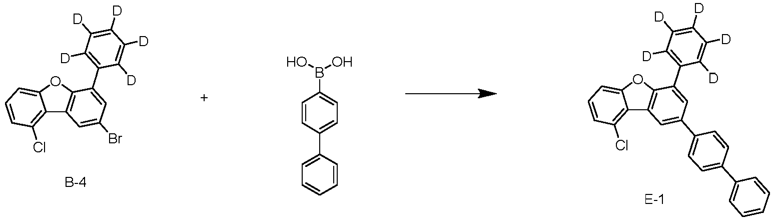

**B-4** (50 g, 114.9 mmol) and (1,1'-biphenyl)-4-ylboronic acid (32.1 g, 126.4 mmol) were added to 1000 mL of 1,4-dioxane under a nitrogen atmosphere, and the mixture was stirred and refluxed. Then, potassium acetate (33.1 g, 344.7 mmol) was added thereto and stirred sufficiently, followed by adding palladium dibenzylideneacetone palladium (2 g, 3.4 mmol) and tricyclohexylphosphine (1.9 g, 6.9 mmol). After 4 hours of reaction, cooling was performed to room temperature. Thereafter, the organic layer was filtered to remove salt, and then the filtered organic layer was distilled. Then, this was dissolved again in 500 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound E-1 in the form of grey solid (25.5 g, 51%, MS: [M+H]⁺ = 436.1).

### 2) Preparation of Compound E-2

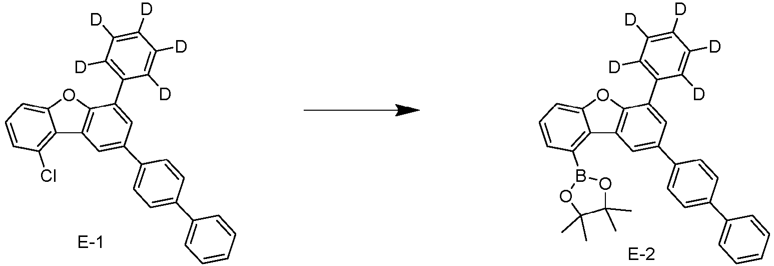

E-1 (50 g, 114.9 mmol) and bis(pinacolato)diboron (32.1 g, 126.4 mmol) were added to 1000 mL of 1,4-dioxane under a nitrogen atmosphere, and the mixture was stirred and refluxed. Then, potassium acetate (33.1 g, 344.7 mmol) was added thereto and stirred sufficiently, followed by adding palladium dibenzylideneacetone palladium (2 g, 3.4 mmol) and tricyclohexylphosphine (1.9 g, 6.9 mmol). After 3 hours of reaction, cooling was performed to room temperature. Thereafter, the organic layer was filtered to remove salt, and then the filtered organic layer was distilled. Then, this was dissolved again in 606 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound E-2 in the form of grey solid (41.8g, 69%, MS: [M+H]⁺ = 528.3).

### Preparation Example 6: Preparation of Compound F-2

### 1) Preparation of Compound F-1

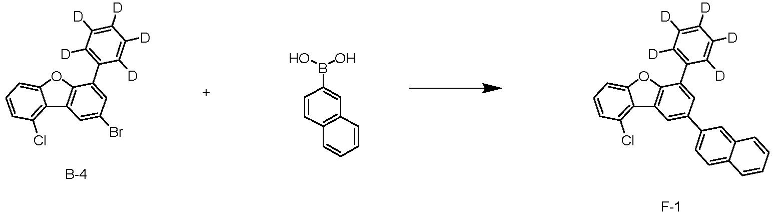

**B-4** (50 g, 138.5 mmol) and naphthalen-2-ylboronic acid (23.8 g, 138.5 mmol) were added to 1000 mL of tetrahydrofuran under a nitrogen atmosphere, and the mixture was stirred and refluxed. Then, potassium carbonate (57.4 g, 415.5 mmol) was dissolved in 57 mL of water, and then added thereto. Thereafter, it was stirred sufficiently, followed by adding tetrakis(triphenylphosphino)palladium (4.8 g, 4.2 mmol). After 2 hours of reaction, cooling was performed to room temperature. Then, the organic layer was separated from the water layer, and then the organic layer was distilled. Then, this was dissolved again in 1133 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound F-1 in the form of white solid (40.2 g, 71%, MS: [M+H]⁺ = 410.1).

### 2) Preparation of Compound F-2

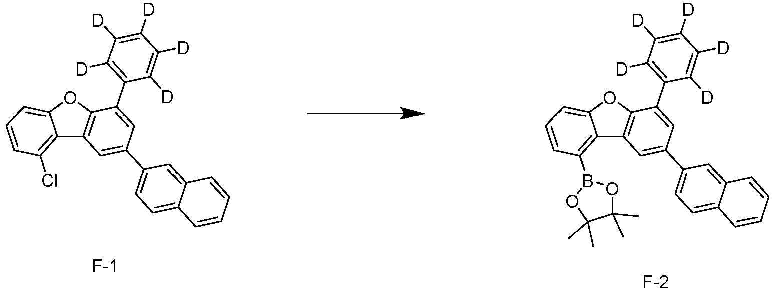

F-1 (50 g, 122.2 mmol) and bis(pinacolato)diboron (34.2 g, 134.4 mmol) were added to 1000 mL of 1,4-dioxane under a nitrogen atmosphere, and the mixture was stirred and refluxed. Then, potassium acetate (35.2 g, 366.6 mmol) was added thereto and stirred sufficiently, followed by adding palladium dibenzylideneacetone palladium (2.1 g, 3.7 mmol) and tricyclohexylphosphine (2.1 g, 7.3 mmol). After 5 hours of reaction, cooling was performed to room temperature. Thereafter, the organic layer was filtered to remove salt, and then the filtered organic layer was distilled. Then, this was dissolved again in 613 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound F-2 in the form of grey solid (47.2 g, 77%, MS: [M+H]⁺ = 502.3).

### Preparation Example 7: Preparation of Compound G-2

### 1) Preparation of Compound G-1

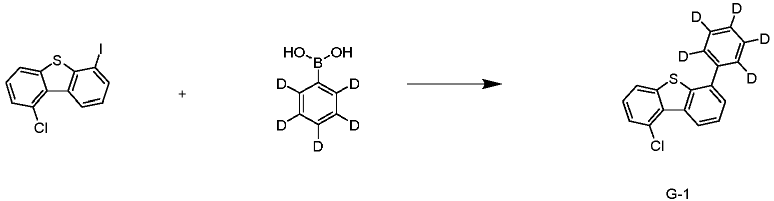

1-chloro-6-iododibenzo[b,d]thiophene (50 g, 145.4 mmol) and phenylboronic acid-D5 (18.5 g, 145.4 mmol) were added to 1000 mL of tetrahydrofuran under a nitrogen atmosphere, and the mixture was stirred and refluxed. Then, potassium carbonate (60.3 g, 436.2 mmol) was dissolved in 60 mL of water, and then added thereto. Thereafter, it was stirred sufficiently, followed by adding tetrakis(triphenylphosphino)palladium (5 g, 4.4 mmol). After 2 hours of reaction, cooling was performed to room temperature. Then, the organic layer was separated from the water layer, and then the organic layer was distilled. Then, this was dissolved again in 861 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound G-1 in the form of white solid (31 g, 72%, MS: [M+H]⁺ = 297.1).

### 2) Preparation of Compound G-2

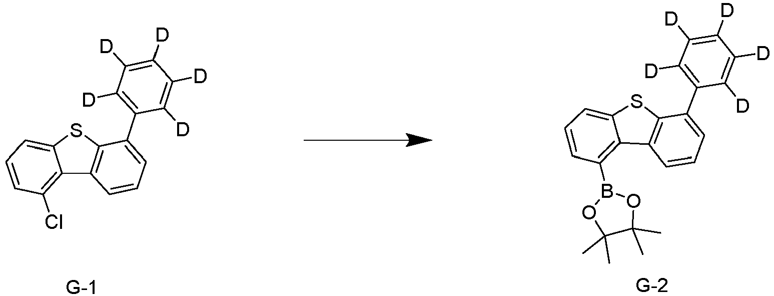

G-1 (50 g, 168.9 mmol) and bis(pinacolato)diboron (47.2 g, 185.8 mmol) were added to 1000 mL of 1,4-dioxane under a nitrogen atmosphere, and the mixture was stirred and refluxed. Then, potassium acetate (48.7 g, 506.7 mmol) was added thereto and stirred sufficiently, followed by adding palladium dibenzylideneacetone palladium (2.9 g, 5.1 mmol) and tricyclohexylphosphine (2.8 g, 10.1 mmol). After 6 hours of reaction, cooling was performed to room temperature. Thereafter, the organic layer was filtered to remove salt, and then the filtered organic layer was distilled. Then, this was dissolved again in 661 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound G-2 in the form of grey solid (34.4 g, 52%, MS: [M+H]⁺ = 392.2).

### Synthesis Example 1: Preparation of Compound 1

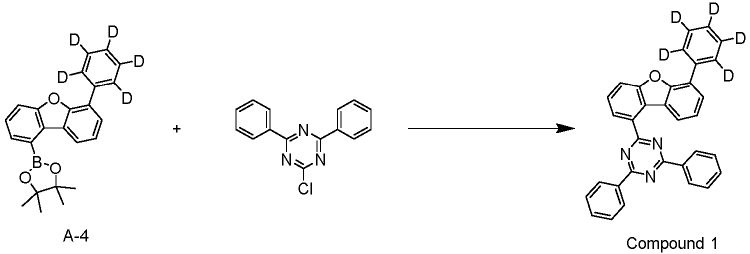

A-4 (20 g, 53.3 mmol) and 2-chloro-4,6-diphenyl-1,3,5-triazine (14.2 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 2 hours of reaction, it was cooled to room temperature and the resulting solid was filtered. Then, the resulting solid was dissolved in 1280 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 1 in the form of white solid (16.4 g, 64%, MS: [M+H]⁺ = 481.2).

### Synthesis Example 2: Preparation of Compound 2

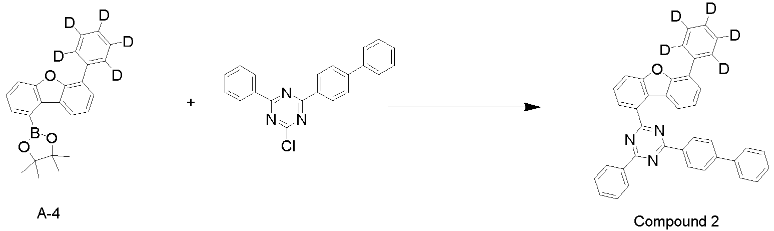

A-4 (20 g, 53.3 mmol) and 2-([1,1'-biphenyl]-4-yl)-4-chloro-6-phenyl-1,3,5-triazine (18.3 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 3 hours of reaction, it was cooled to room temperature and the resulting solid was filtered. Then, the resulting solid was dissolved in 1482 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 2 in the form of white solid (17.5 g, 59%, MS: [M+H]⁺ = 557.2).

### Synthesis Example 3: Preparation of Compound 3

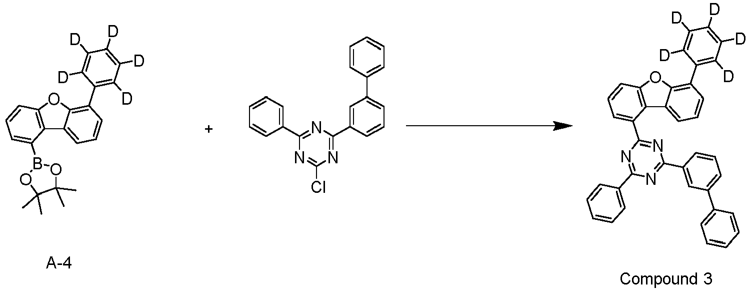

A-4 (20 g, 53.3 mmol) and 2-([1,1'-biphenyl]-3-yl)-4-chloro-6-phenyl-1,3,5-triazine (18.3 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 3 hours of reaction, it was cooled to room temperature and the resulting solid was filtered. Then, the resulting solid was dissolved in 1482 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 3 in the form of white solid (21.1 g, 71%, MS: [M+H]⁺ = 557.2).

### Synthesis Example 4: Preparation of Compound 4

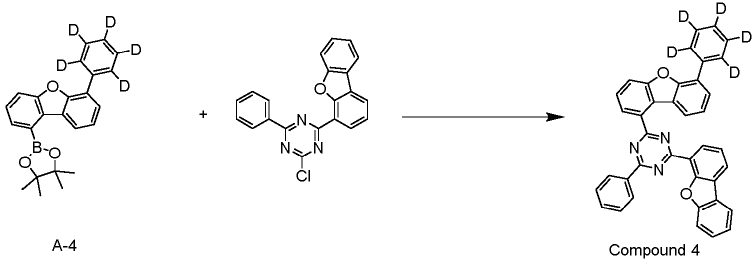

A-4 (20 g, 53.3 mmol) and 2-chloro-4-(dibenzo[b,d]furan-4-yl)-6-phenyl-1,3,5-triazine (19 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 1 hour of reaction, it was cooled to room temperature and the resulting solid was filtered. Then, the resulting solid was dissolved in 1520 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 4 in the form of white solid (17.3 g, 57%, MS: [M+H]⁺ = 571.2).

### Synthesis Example 5: Preparation of Compound 5

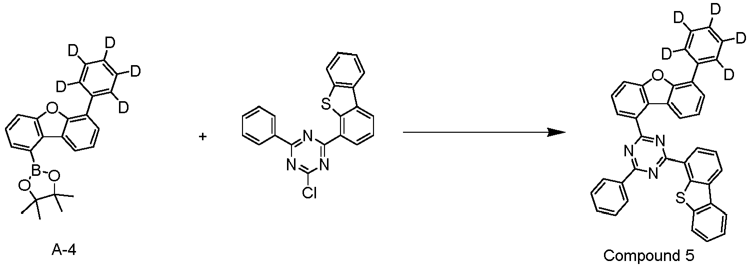

A-4 (20 g, 53.3 mmol) 2-chloro-4-(dibenzo[b,d]thiophen-4-yl)-6-phenyl-1,3,5-triazine (19.9 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 2 hours of reaction, it was cooled to room temperature and the resulting solid was filtered. Then, the resulting solid was dissolved in 1562 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 5 in the form of white solid (22.8 g, 73%, MS: [M+H]⁺ = 587.2).

### Synthesis Example 6: Preparation of Compound 6

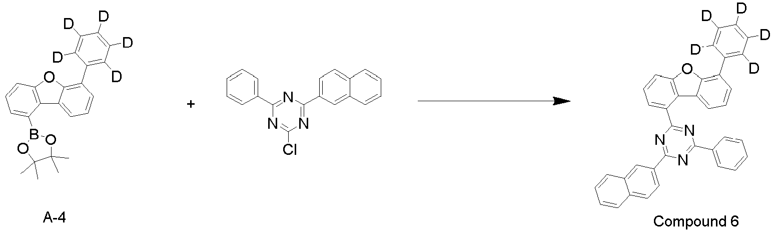

A-4 (20 g, 53.3 mmol) and 2-chloro-4-(naphthalen-2-yl)-6-phenyl-1,3,5-triazine (16.9 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 1 hour of reaction, it was cooled to room temperature and the resulting solid was filtered. Then, the resulting solid was dissolved in 1413 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 6 in the form of white solid (18.1 g, 64%, MS: [M+H]⁺ = 531.2).

### Synthesis Example 7: Preparation of Compound 7

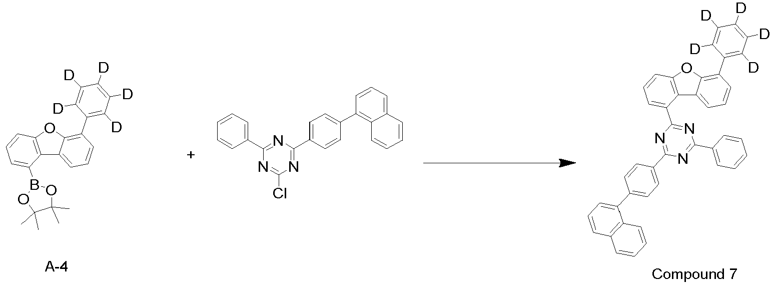

A-4 (20 g, 53.3 mmol) and 2-chloro-4-(4-(naphthalen-1-yl)phenyl)-6-phenyl-1,3,5-triazine (21 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 1 hour of reaction, it was cooled to room temperature and the resulting solid was filtered. Then, the resulting solid was dissolved in 1616 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 7 in the form of white solid (24.6 g, 76%, MS: [M+H]⁺ = 607.3).

### Synthesis Example 8: Preparation of Compound 8

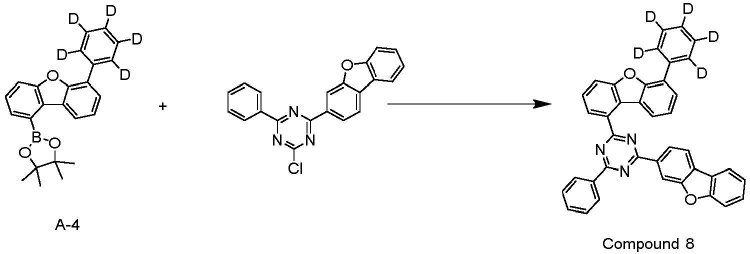

A-4 (20 g, 53.3 mmol) and 2-chloro-4-(dibenzo[b,d]furan-3-yl)-6-phenyl-1,3,5-triazine (19 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 2 hours of reaction, it was cooled to room temperature and the resulting solid was filtered. Then, the resulting solid was dissolved in 1520 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 8 in the form of white solid (21.6 g, 71%, MS: [M+H]⁺ = 571.2).

### Synthesis Example 9: Preparation of Compound 9

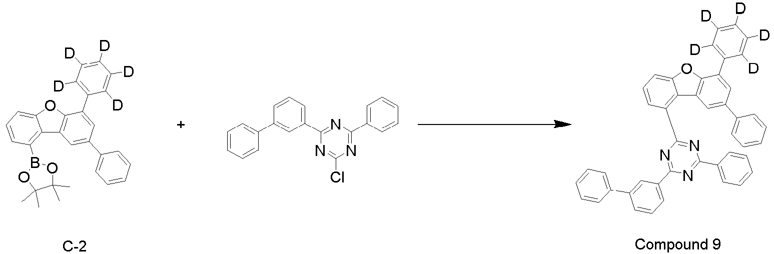

C-2 (20 g, 44.3 mmol) and 2-([1,1'-biphenyl]-3-yl)-4-chloro-6-phenyl-1,3,5-triazine (15.2 g, 44.3 mmol) were added to 400 mL of tetrahydrofuran under a nitrogen atmosphere, and the mixture was stirred and refluxed. Then, potassium carbonate (18.4 g, 133 mmol) was dissolved in 18 mL of water, and then added thereto. Thereafter, it was stirred sufficiently, followed by adding tetrakis(triphenylphosphino)palladium (1.5 g, 1.3 mmol). After 2 hours of reaction, it was cooled to room temperature and the resulting solid was filtered. Then, the resulting solid was dissolved in 1401 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 9 in the form of white solid (21 g, 75%, MS: [M+H]⁺ = 633.3).

### Synthesis Example 10: Preparation of Compound 10

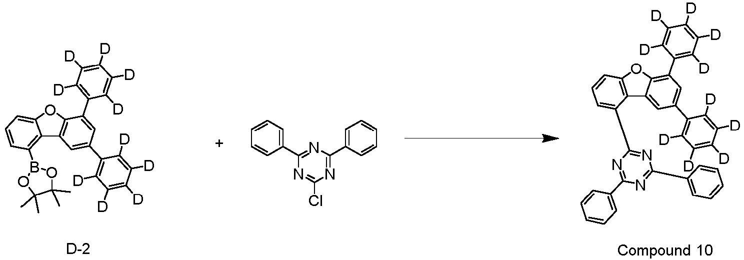

D-2 (20 g, 43.8 mmol) and 2-chloro-4,6-diphenyl-1,3,5-triazine (17.2 g, 43.8 mmol) were added to 400 mL of tetrahydrofuran under a nitrogen atmosphere, and the mixture was stirred and refluxed. Then, potassium carbonate (18.2 g, 131.5 mmol) was dissolved in 18 mL of water, and then added thereto. Thereafter, it was stirred sufficiently, followed by adding tetrakis(triphenylphosphino)palladium (1.5 g, 1.3 mmol). After 2 hours of reaction, it was cooled to room temperature and the resulting solid was filtered. Then, the resulting solid was dissolved in 1329 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 10 in the form of white solid (15.9 g, 60%, MS: [M+H]⁺ = 607.3).

### Synthesis Example 11: Preparation of Compound 11

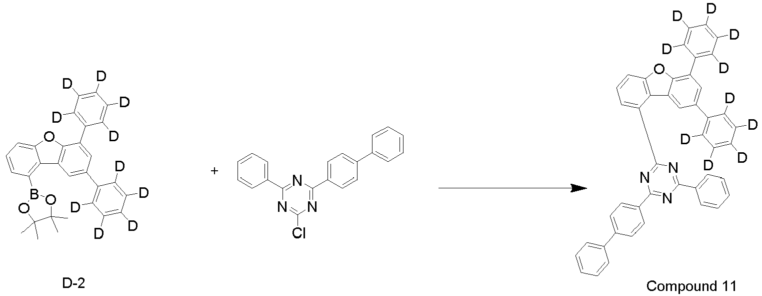

D-2(20 g, 53.3 mmol) and 2-([1,1'-biphenyl]-4-yl)-4-chloro-6-phenyl-1,3,5-triazine (18.3 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 3 hours of reaction, it was cooled to room temperature and the resulting solid was filtered. Then, the resulting solid was dissolved in 1698 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 11 in the form of white solid (26.8 g, 79%, MS: [M+H]⁺ = 638.3).

### Synthesis Example 12: Preparation of Compound 12

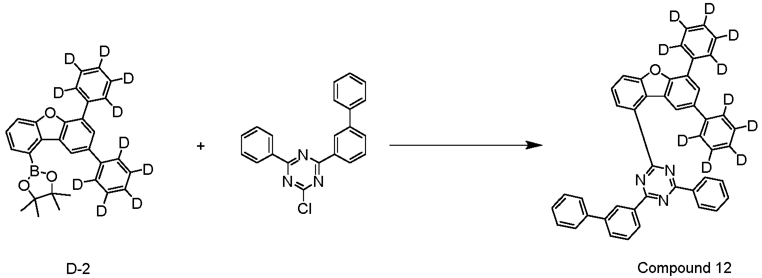

D-2 (20 g, 53.3 mmol) and 2-([1,1'-biphenyl]-3-yl)-4-chloro-6-phenyl-1,3,5-triazine (18.3 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 1 hour of reaction, it was cooled to room temperature and the resulting solid was filtered. Then, the resulting solid was dissolved in 1698 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 12 in the form of white solid (21.7 g, 64%, MS: [M+H]⁺ = 638.3).

### Synthesis Example 13: Preparation of Compound 13

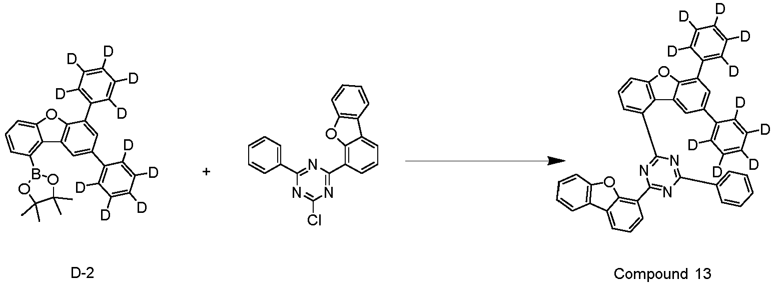

D-2 (20 g, 53.3 mmol) and 2-chloro-4-(dibenzo[b,d]furan-4-yl)-6-phenyl-1,3,5-triazine (18.3 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 3 hours of reaction, it was cooled to room temperature and the resulting solid was filtered. Then, the resulting solid was dissolved in 1736 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 13 in the form of white solid (20.8 g, 60%, MS: [M+H]⁺ = 652.3).

### Synthesis Example 14: Preparation of Compound 14

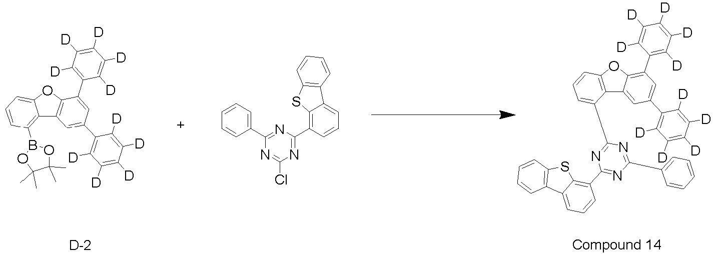

D-2 (20 g, 53.3 mmol) and 2-chloro-4-(dibenzo[b,d]thiophen-4-yl)-6-phenyl-1,3,5-triazine (19 g, 53.3 mmol) were added to 400 mL of tetrahydrofuran under a nitrogen atmosphere, and the mixture was stirred and refluxed. Then, potassium carbonate (22.1 g, 159.9 mmol) was dissolved in 22 mL of water, and then added thereto. Thereafter, it was stirred sufficiently, followed by adding tetrakis(triphenylphosphino)palladium (1.8 g, 1.6 mmol). After 2 hours of reaction, it was cooled to room temperature and the resulting solid was filtered. Then, the resulting solid was dissolved in 1778 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 14 in the form of white solid (24.9 g, 70%, MS: [M+H]⁺ = 668.3).

### Synthesis Example 15: Preparation of Compound 15

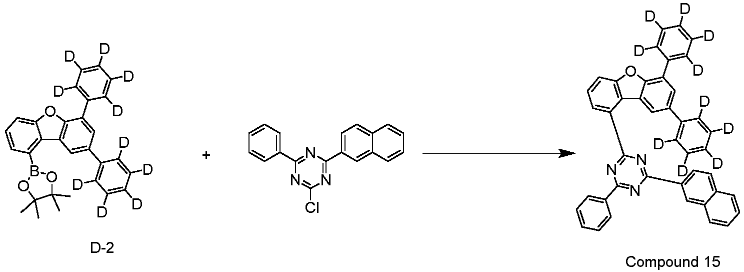

D-2 (20 g, 43.8 mmol) and 2-chloro-4-(naphthalen-2-yl)-6-phenyl-1,3,5-triazine (13.9 g, 43.8 mmol) were added to 400 mL of tetrahydrofuran under a nitrogen atmosphere, and the mixture was stirred and refluxed. Then, potassium carbonate (18.2 g, 131.5 mmol) was dissolved in 18 mL of water, and then added thereto. Thereafter, it was stirred sufficiently, followed by adding tetrakis(triphenylphosphino)palladium (1.5 g, 1.3 mmol). After 1 hour of reaction, it was cooled to room temperature and the resulting solid was filtered. Then, the resulting solid was dissolved in 1340 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 15 in the form of white solid (19.6 g, 73%, MS: [M+H]⁺ = 612.3).

### Synthesis Example 16: Preparation of Compound 16

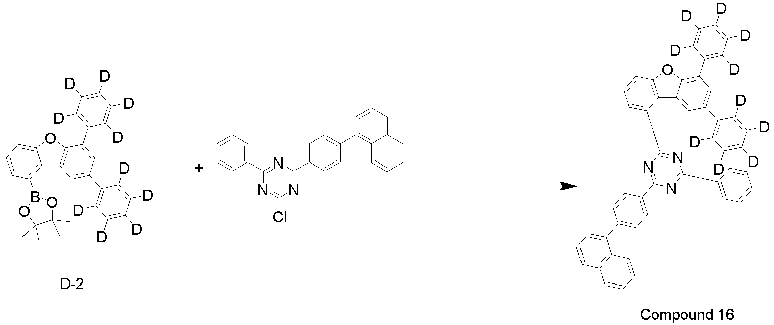

D-2 (20 g, 43.8 mmol) and 2-chloro-4-(4-(naphthalen-1-yl)phenyl)-6-phenyl-1,3,5-triazine (17.2 g, 43.8 mmol) were added to 400 mL of tetrahydrofuran under a nitrogen atmosphere, and the mixture was stirred and refluxed. Then, potassium carbonate (18.2 g, 131.5 mmol) was dissolved in 18 mL of water, and then added thereto. Thereafter, it was stirred sufficiently, followed by adding tetrakis(triphenylphosphino)palladium (1.5 g, 1.3 mmol). After 3 hours of reaction, it was cooled to room temperature and the resulting solid was filtered. Then, the resulting solid was dissolved in 1507 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 16 in the form of white solid (20.5 g, 68%, MS: [M+H]⁺ = 688.7).

### Synthesis Example 17: Preparation of Compound 17

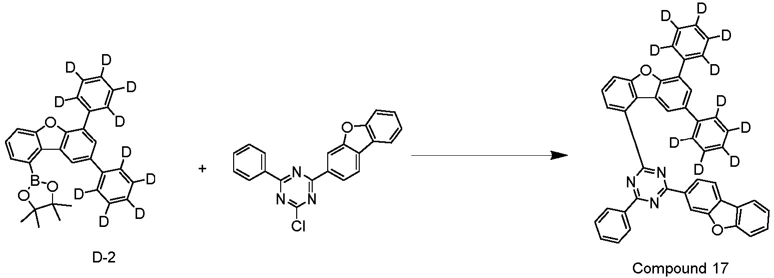

D-2 (20 g, 43.8 mmol) and 2-chloro-4-(dibenzo[b,d]furan-3-yl)-6-phenyl-1,3,5-triazine (15.7 g, 43.8 mmol) were added to 400 mL of tetrahydrofuran under a nitrogen atmosphere, and the mixture was stirred and refluxed. Then, potassium carbonate (18.2 g, 131.5 mmol) was dissolved in 18 mL of water, and then added thereto. Thereafter, it was stirred sufficiently, followed by adding tetrakis(triphenylphosphino)palladium (1.5 g, 1.3 mmol). After 2 hours of reaction, it was cooled to room temperature and the resulting solid was filtered. Then, the resulting solid was dissolved in 1427 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 17 in the form of white solid (20 g, 70%, MS: [M+H]⁺ = 652.3).

### Synthesis Example 18: Preparation of Compound 18

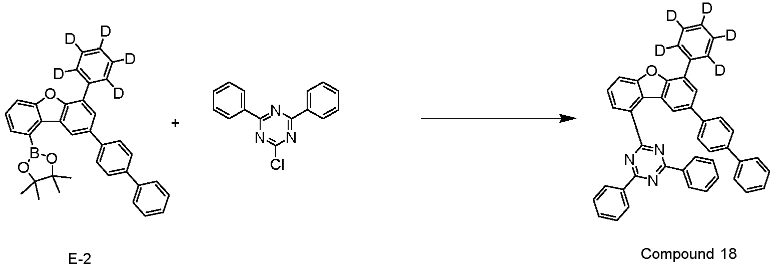

E-2 (20 g, 37.9 mmol) and 2-chloro-4,6-diphenyl-1,3,5-triazine (10.1 g, 37.9 mmol) were added to 400 mL of tetrahydrofuran under a nitrogen atmosphere, and the mixture was stirred and refluxed. Then, potassium carbonate (15.7 g, 113.8 mmol) was dissolved in 16 mL of water, and then added thereto. Thereafter, it was stirred sufficiently, followed by adding tetrakis(triphenylphosphino)palladium (1.3 g, 1.1 mmol). After 1 hour of reaction, it was cooled to room temperature and the resulting solid was filtered. Then, the resulting solid was dissolved in 1199 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 18 in the form of white solid (15.8 g, 66%, MS: [M+H]⁺ = 633.3).

### Synthesis Example 19: Preparation of Compound 19

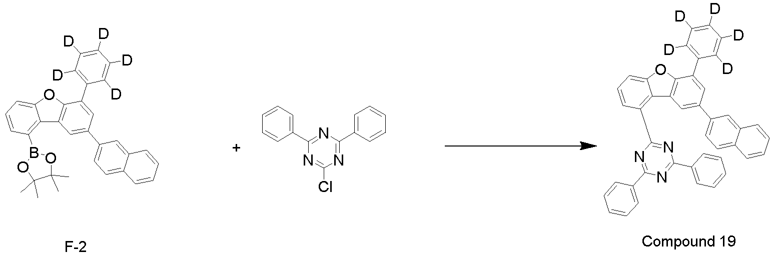

F-2 (20 g, 39.9 mmol) and 2-chloro-4,6-diphenyl-1,3,5-triazine (10.7 g, 39.9 mmol) were added to 400 mL of tetrahydrofuran under a nitrogen atmosphere, and the mixture was stirred and refluxed. Then, potassium carbonate (16.5 g, 119.7 mmol) was dissolved in 17 mL of water, and then added thereto. Thereafter, it was stirred sufficiently, followed by adding tetrakis(triphenylphosphino)palladium (1.4 g, 1.2 mmol). After 3 hours of reaction, it was cooled to room temperature and the resulting solid was filtered. Then, the resulting solid was dissolved in 1209 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 19 in the form of white solid (18.6 g, 77%, MS: [M+H]⁺ = 607.3).

### Synthesis Example 20: Preparation of Compound 20

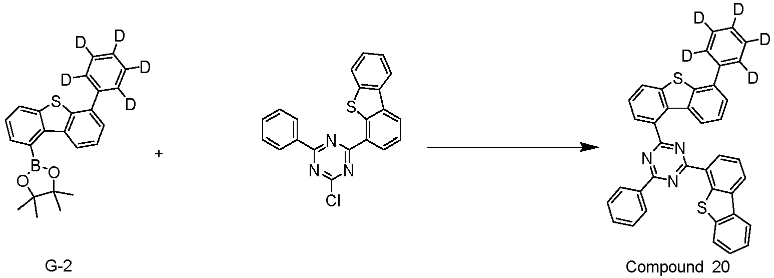

G-2 (20 g, 51.1 mmol) and 2-chloro-4-(dibenzo[b,d]thiophen-4-yl)-6-phenyl-1,3,5-triazine (19.1 g, 51.1 mmol) were added to 400 mL of tetrahydrofuran under a nitrogen atmosphere, and the mixture was stirred and refluxed. Then, potassium carbonate (21.2 g, 153.4 mmol) was dissolved in 21 mL of water, and then added thereto. Thereafter, it was stirred sufficiently, followed by adding tetrakis(triphenylphosphino)palladium (1.8 g, 1.5 mmol). After 2 hours of reaction, it was cooled to room temperature and the resulting solid was filtered. Then, the resulting solid was dissolved in 1539 mL of chloroform, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 20 in the form of white solid (23.7 g, 77%, MS: [M+H]⁺ = 603.2).

### Example 1: Preparation of organic light emitting device

A glass substrate on which ITO (Indium Tin Oxide) was coated as a thin film to a thickness of 1300 Å was put into distilled water in which a detergent was dissolved, and ultrasonically cleaned. At this time, a product manufactured by Fischer Co. was used as the detergent, and distilled water filtered twice using a filter manufactured by Millipore Co. was used as the distilled water. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

The following Compound HI-1 was thermally vacuum-deposited on the prepared ITO transparent electrode to a thickness of 50 Å to form a hole injection layer. Then, the following Compound HT-1 was thermally vacuum-deposited thereon to a thickness of 250 Å to form a hole transport layer. The following Compound HT-2 was vacuum-deposited thereon to a thickness of 50 Å to form an electron blocking layer.

The Compound 1 prepared in Synthesis Example 1, the following Compound YGH-1, and a phosphorescent dopant YGD-1 were co-deposited on the HT-2 deposited film at a weight ratio of 44:44:12 to form a light emitting layer having a thickness of 400 Å.

The following Compound ET-1 was vacuum-deposited on the light emitting layer to a thickness of 250 Å to form an electron transport layer, and the following Compound ET-2 and LiF were vacuum-deposited on the electron transport layer at a weight ratio of 98:2 to form an electron injection layer having a thickness of 100 Å. A cathode was formed by depositing aluminum on the electron injection layer to a thickness of 1000 Å.

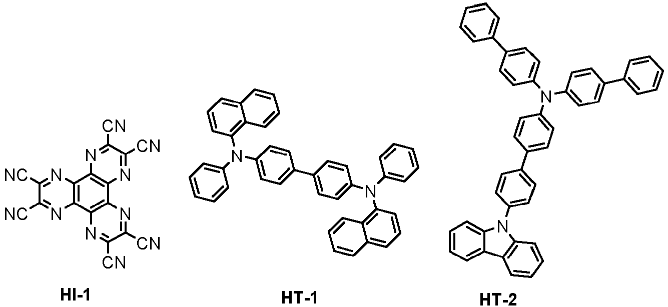

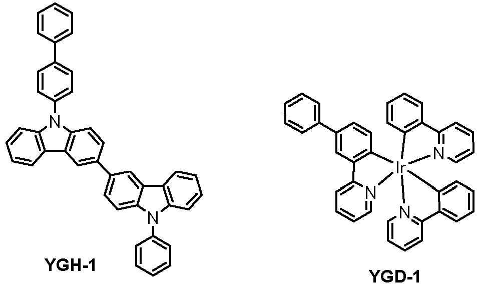

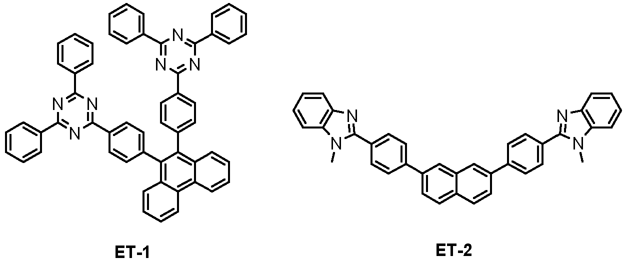

In the above process, the deposition rate of the organic material was maintained at 0.4 to 0.7 Å/sec, the deposition rate of aluminum was maintained at 2 Å/sec, and the degree of vacuum during the deposition was maintained at 1.3 x 10⁻⁵ to 6.7 x 10⁻⁶ Pa (1 x 10⁻⁷ to 5 x 10⁻⁸ torr).

### Examples 2 to 20

Organic light emitting devices were manufactured in the same manner as in Example 1, except that the compound shown in Table 1 was used instead of Compound 1 of Synthesis Example 1 as one of the host materials of the light emitting layer in Example 1.

At this time, the structures of the compounds used in Examples 1 to 20 are as follows.

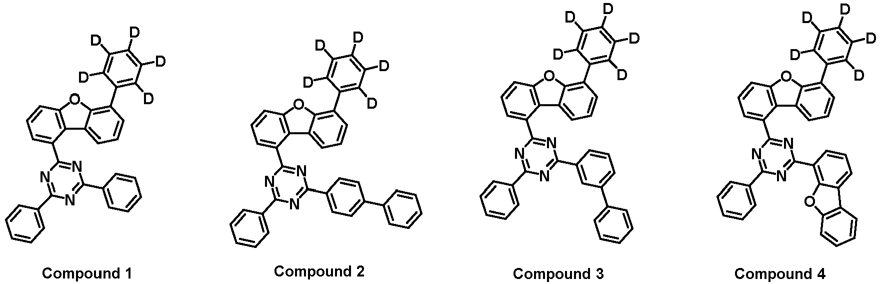

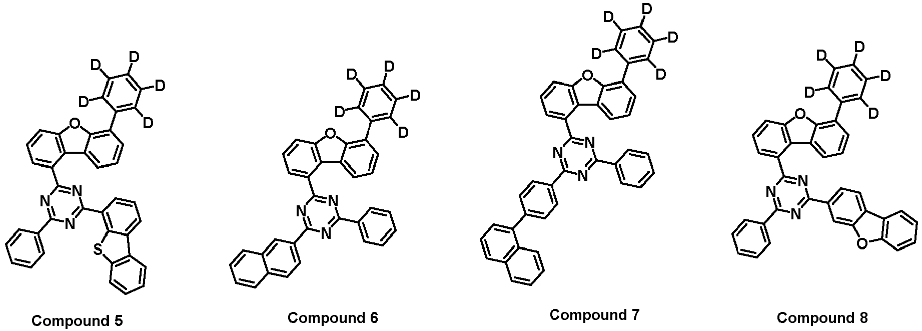

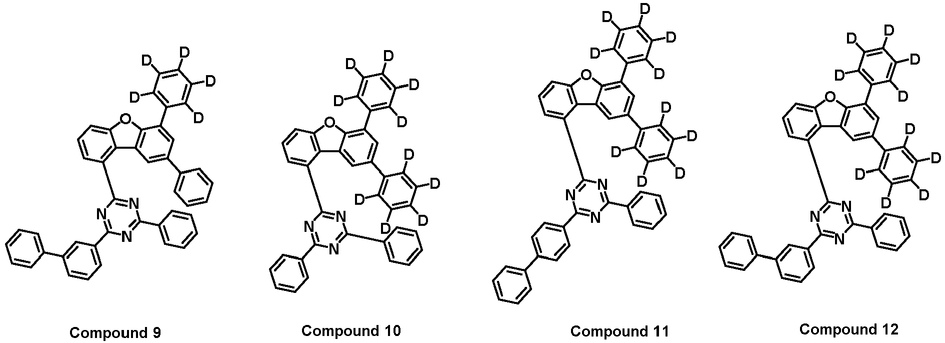

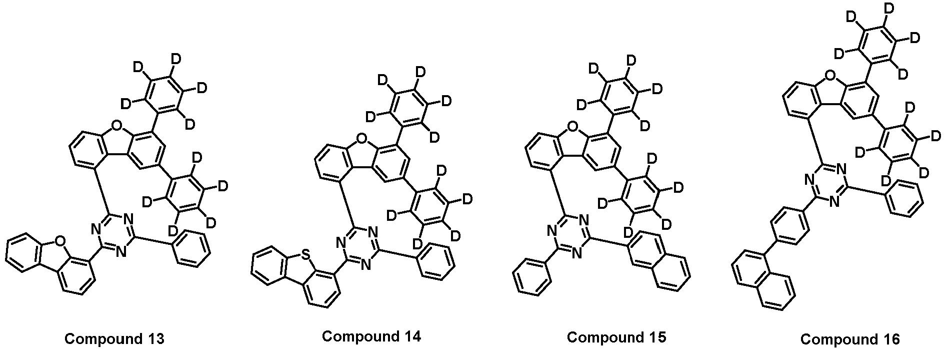

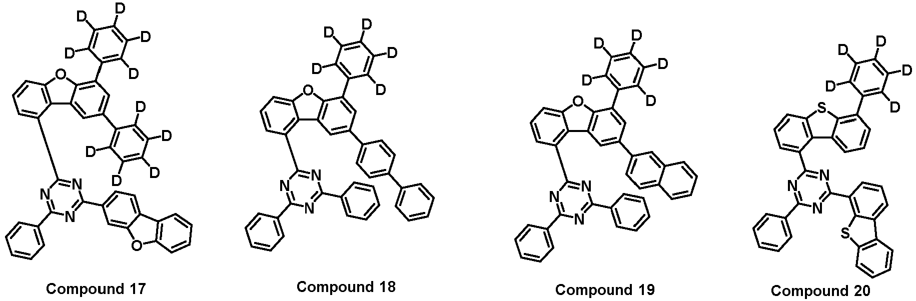

### Comparative Examples 1 to 5

Organic light emitting devices were manufactured in the same manner as in Example 1, except that the compound shown in Table 1 was used instead of Compound 1 of Synthesis Example 1 as one of the host materials of the light emitting layer in Example 1. At this time, the Compounds CE1 to CE5 shown in Table 1 below are as follows.

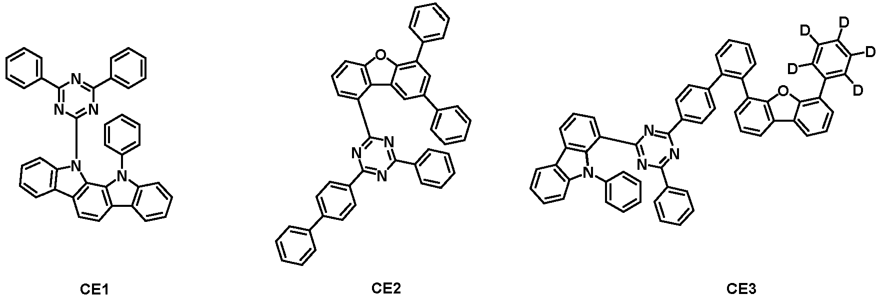

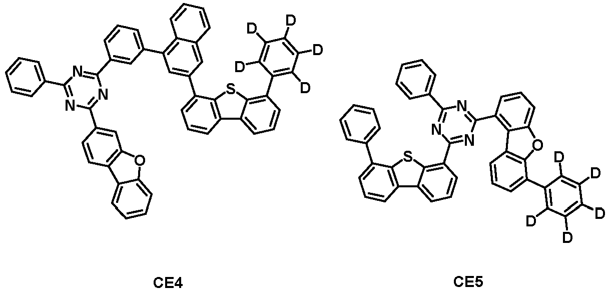

### Experimental Example 1: Evaluation of device characteristics

For the organic light emitting devices prepared in Examples and Comparative Examples, the voltage and efficiency were measured by applying a current density of 10 mA/cm², and the lifespan was measured by applying a current density of 50 mA/cm². Then, the results are shown in Table 1 below. Herein, LT95 means the time taken until the initial luminance decreases to 95%.

**[Table 1]**

| | Compound | Voltage (V) (@ 10mA/cm²) | Efficiency (Cd/A) (@ 10mA/cm²) | Chromaticity coordinates (x,y) | Lifespan (h) (LT₉₅ at 50mA/cm²) |
|---|---|---|---|---|---|
| Example 1 | Compound 1 | 4.1 | 75 | 0.45, 0.53 | 200 |
| Example 2 | Compound 2 | 4.2 | 76 | 0.44, 0.54 | 210 |
| Example 3 | Compound 3 | 4.3 | 74 | 0.45, 0.54 | 250 |
| Example 4 | Compound 4 | 4.2 | 77 | 0.44, 0.53 | 220 |
| Example 5 | Compound 5 | 4.1 | 78 | 0.45, 0.53 | 230 |
| Example 6 | Compound 6 | 4.3 | 77 | 0.44, 0.54 | 240 |
| Example 7 | Compound 7 | 4.2 | 75 | 0.45, 0.53 | 250 |
| Example 8 | Compound 8 | 4.2 | 77 | 0.44, 0.54 | 250 |
| Example 9 | Compound 9 | 4.2 | 80 | 0.45, 0.54 | 190 |
| Example 10 | Compound 10 | 4.1 | 81 | 0.44, 0.53 | 200 |
| Example 11 | Compound 11 | 4.2 | 82 | 0.45, 0.54 | 230 |
| Example 12 | Compound 12 | 4.3 | 81 | 0.44, 0.53 | 240 |
| Example 13 | Compound 13 | 4.2 | 82 | 0.45, 0.53 | 230 |
| Example 14 | Compound 14 | 4.1 | 81 | 0.44, 0.54 | 270 |
| Example 15 | Compound 15 | 4.4 | 80 | 0.45, 0.53 | 220 |
| Example 16 | Compound 16 | 4.3 | 79 | 0.44, 0.54 | 230 |
| Example 17 | Compound 17 | 4.2 | 81 | 0.45, 0.54 | 290 |
| Example 18 | Compound 18 | 4.2 | 82 | 0.44, 0.53 | 260 |
| Example 19 | Compound 19 | 4.3 | 80 | 0.44, 0.54 | 240 |
| Example 20 | Compound 20 | 4.2 | 80 | 0.45, 0.54 | 270 |
| Comparative Example 1 | CE1 | 4.1 | 75 | 0.45, 0.53 | 100 |
| Comparative Example 2 | CE2 | 4.2 | 82 | 0.44, 0.53 | 130 |
| Comparative Example 3 | CE3 | 4.6 | 64 | 0.45, 0.54 | 25 |
| Comparative Example 4 | CE4 | 4.6 | 75 | 0.44, 0.53 | 150 |
| Comparative Example 5 | CE5 | 4.9 | 72 | 0.44, 0.53 | 140 |

As shown in Table 1, it was confirmed that the organic light emitting devices of Examples using the compound represented by the Chemical Formula 1 as the host material of the light emitting layer had significantly improved lifespan without a decrease in efficiency compared to the organic light emitting devices of Comparative Examples using a compound having a different structure. Therefore, it could be confirmed that the compound of the present disclosure can improve the characteristics of the organic light emitting device compared to the compound of Comparative Examples, considering that the luminous efficiency and lifespan of the organic light emitting device generally have a trade-off relationship.

**[DESCRIPTION OF SYMBOLS]**

| | | | |
|---|---|---|---|
| 1: | Substrate | 2: | Anode |
| 3: | Light emitting layer | 4: | Cathode |
| 5: | Hole injection layer | 6: | Hole transport layer |
| 7: | Electron blocking layer | 8: | Electron transport layer |
| 9: | Electron injection layer | | |

## Claims

1. A compound represented by the following Chemical Formula 1: 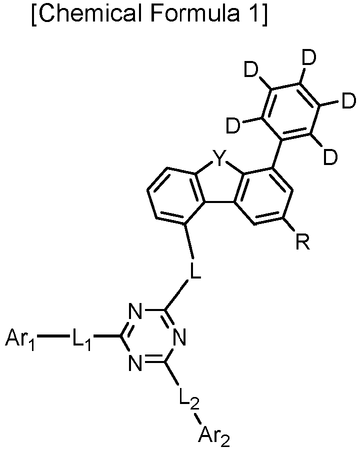 in the Chemical Formula 1,
Y is O or S,
D is deuterium,
L, L₁ and L₂ are each independently a single bond, or C₆₋₂₀ arylene unsubstituted or substituted with deuterium,
Ar₁ and Ar₂ are each independently C₆₋₂₀ aryl unsubstituted or substituted with at least one substituent selected from the group consisting of deuterium, methyl and phenyl; unsubstituted dibenzofuranyl; unsubstituted dibenzothiophenyl; or C₂₋₂₀ heteroaryl containing N unsubstituted or substituted with at least one substituent selected from the group consisting of deuterium, methyl and phenyl, and
R is hydrogen; or C₆₋₆₀ aryl unsubstituted or substituted with deuterium.

2. The compound of Claim 1,
wherein L is a single bond.

3. The compound of Claim 1,
wherein L₁ and L₂ are each independently a single bond, or phenylene.

4. The compound of Claim 1,
wherein Ar₁ and Ar₂ are each independently phenyl, biphenylyl, terphenylyl, naphthyl, phenanthryl, fluorenyl, dibenzofuranyl, dibenzothiophenyl, or carbazolyl, and
the phenyl, biphenylyl, terphenylyl, naphthyl, phenanthryl, fluorenyl, and carbazolyl are each independently unsubstituted or substituted with at least one substituent selected from the group consisting of deuterium, methyl and phenyl.

5. The compound of Claim 4,
wherein Ar₁ and Ar₂ are each independently any one selected from the group consisting of the following:

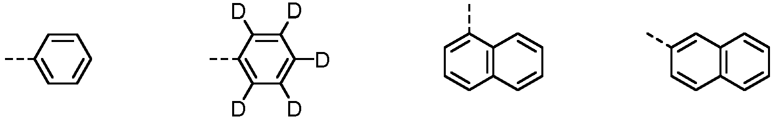

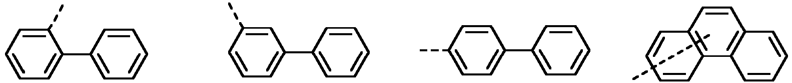

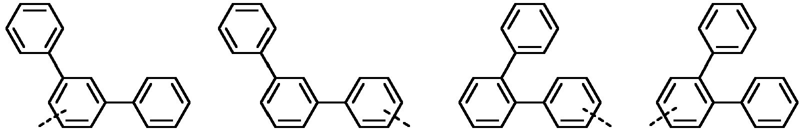

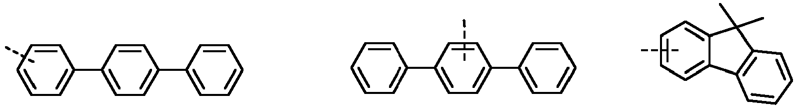

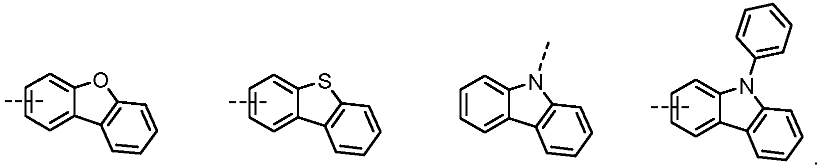

6. The compound of Claim 1,
wherein Ar₁ and Ar₂ are each independently C₆₋₁₂ aryl unsubstituted or substituted with deuterium.

7. The compound of Claim 1,
wherein R is hydrogen; phenyl unsubstituted or substituted with deuterium; biphenylyl unsubstituted or substituted with deuterium; or naphthyl unsubstituted or substituted with deuterium.

8. The compound of Claim 7,
wherein R is hydrogen, 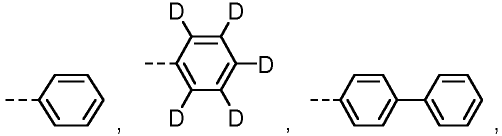 or

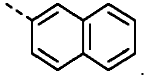

9. The compound of Claim 1,
wherein the compound is represented by any one of the following Chemical Formulae 1-1 to 1-5: 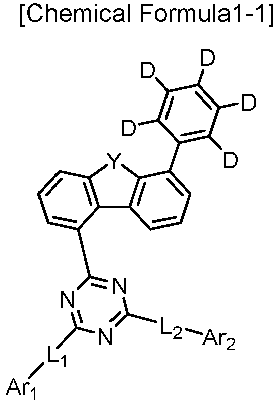 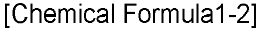 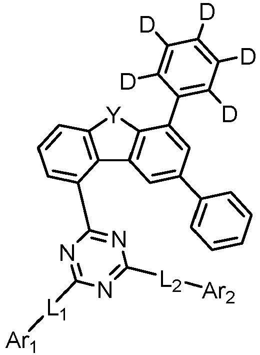 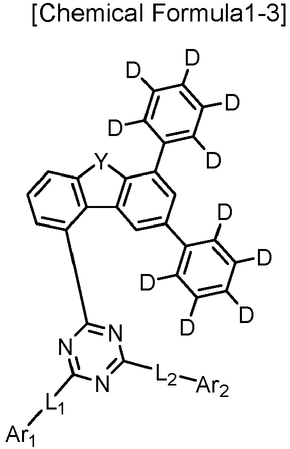 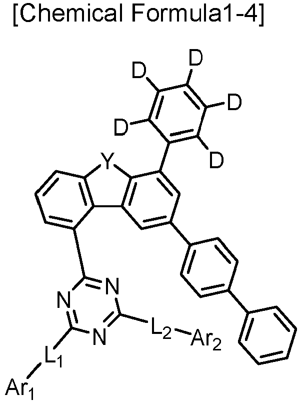 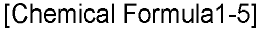 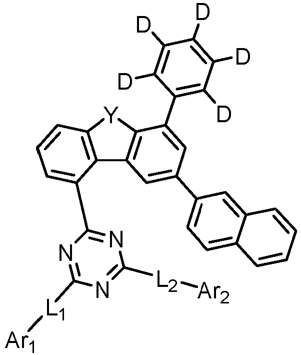
in the Chemical Formulae 1-1 to 1-5,
Y, L₁, L₂, Ar₁ and Ar₂ are as defined in Claim 1.

10. The compound of Claim 1,
wherein the compound is any one selected from the group consisting of the following compounds:

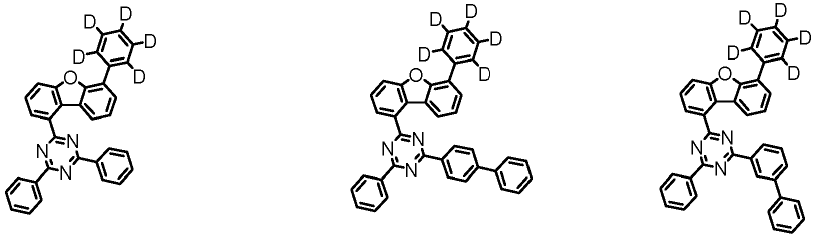

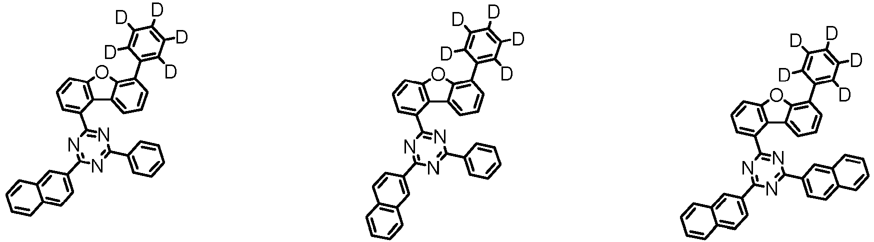

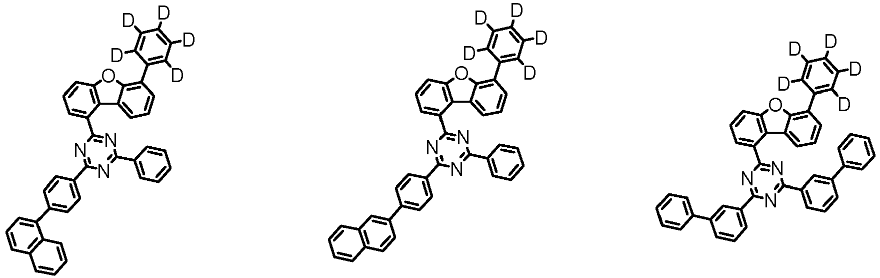

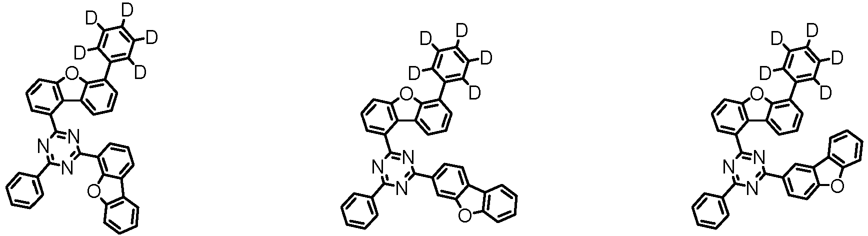

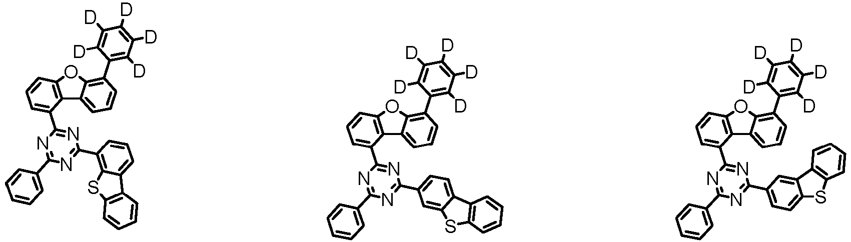

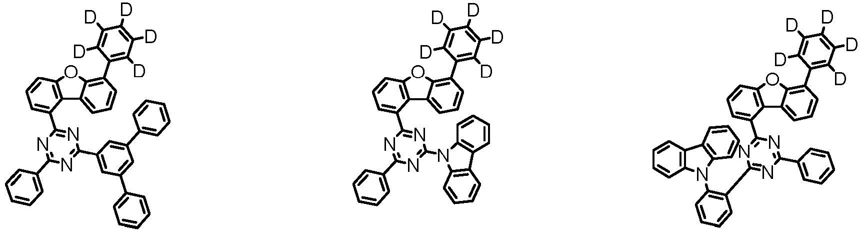

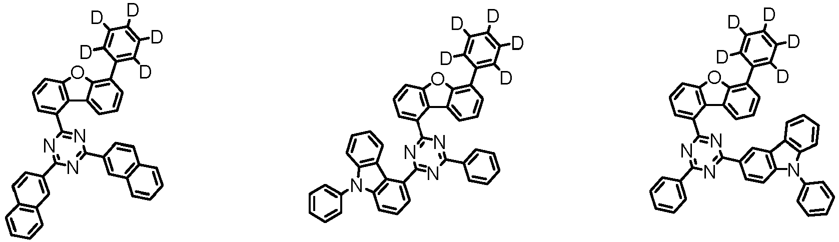

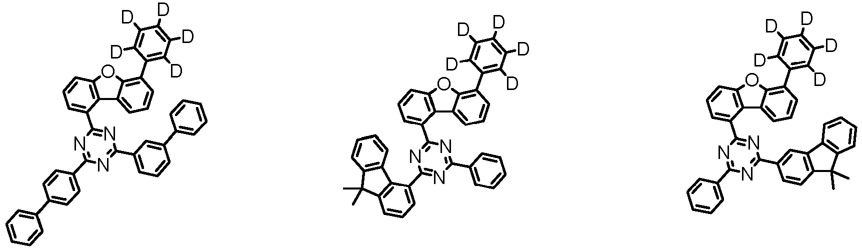

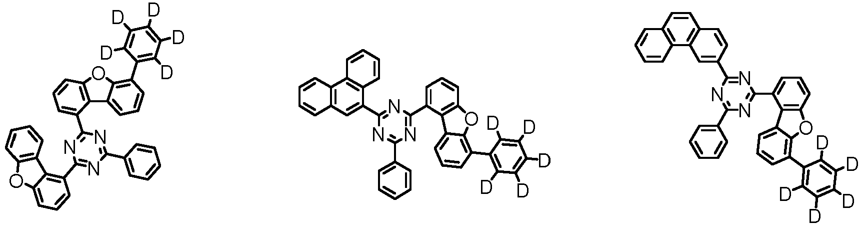

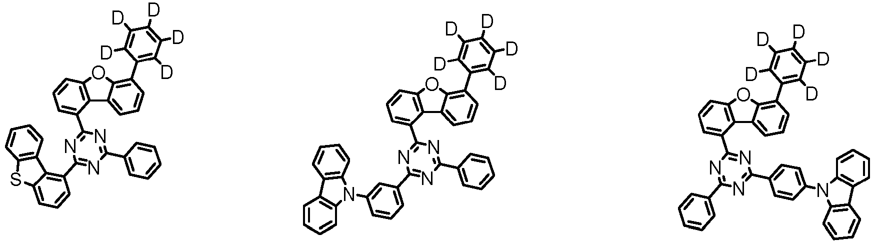

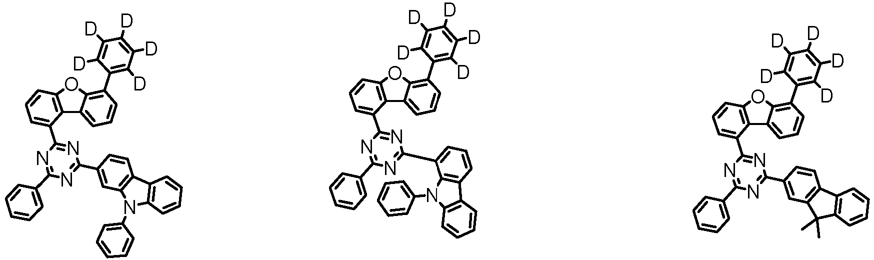

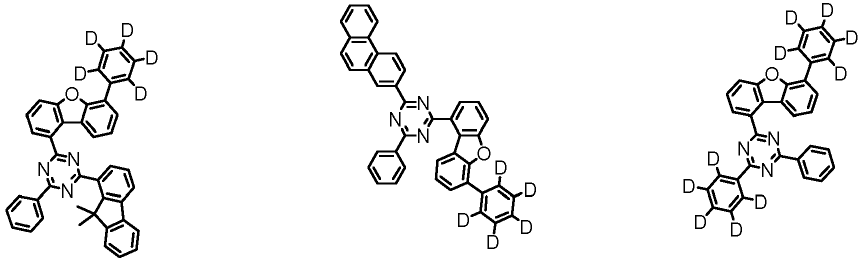

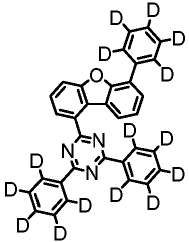

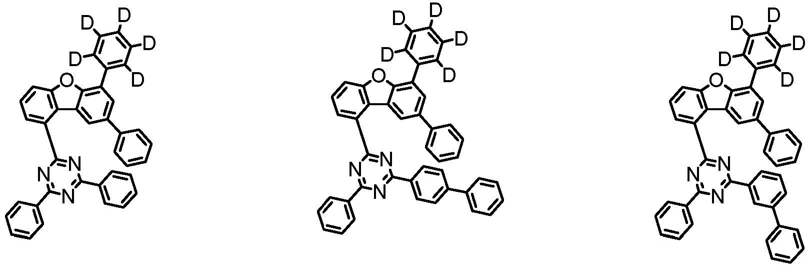

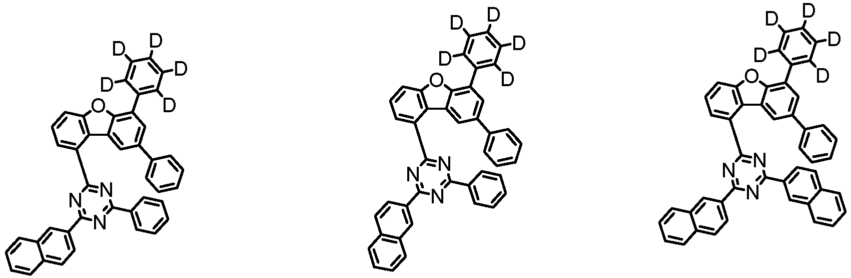

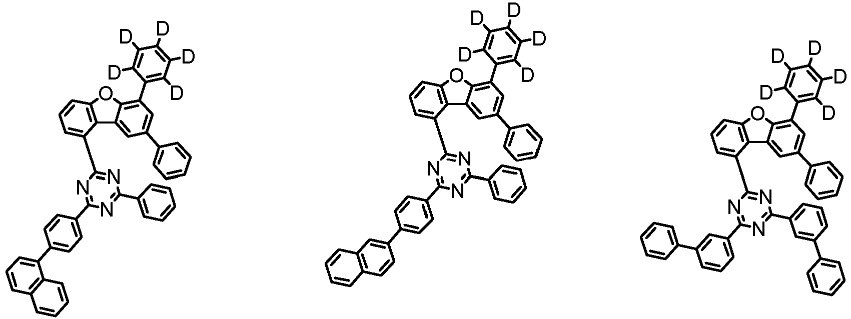

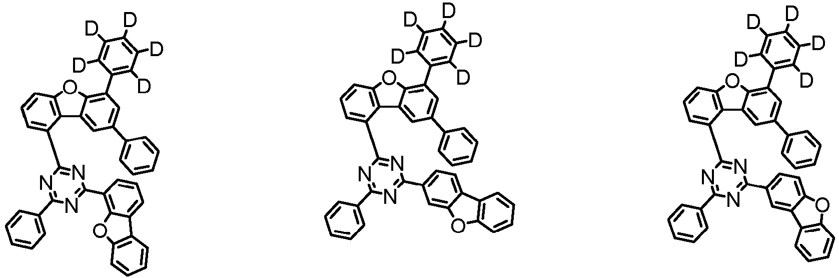

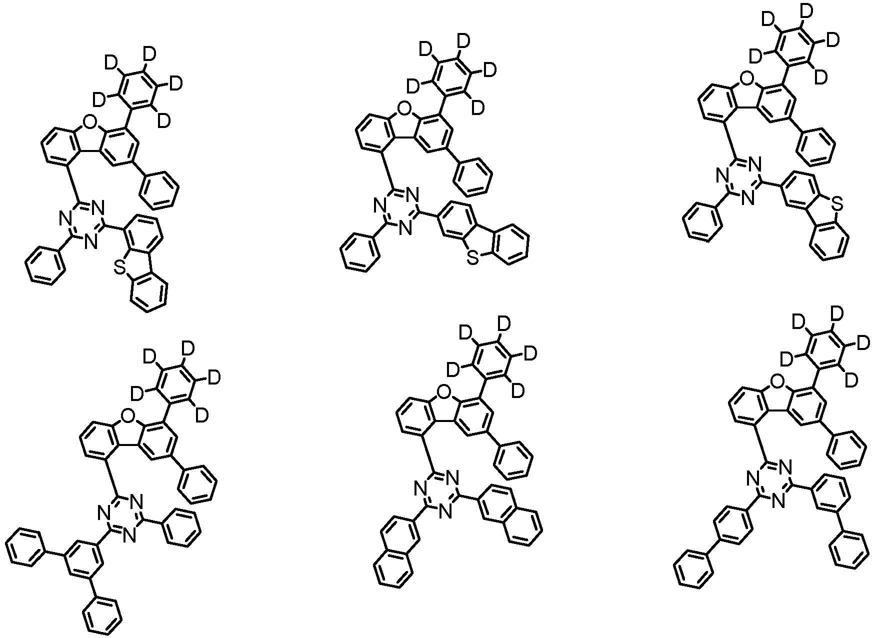

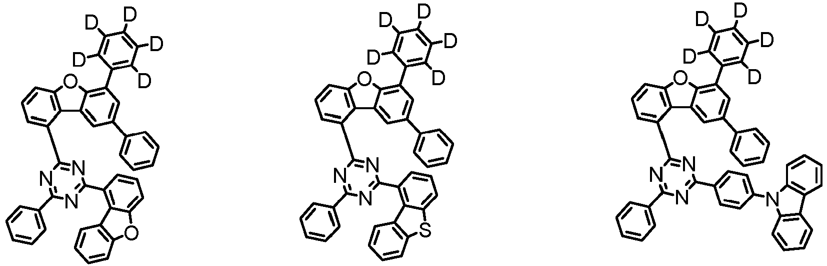

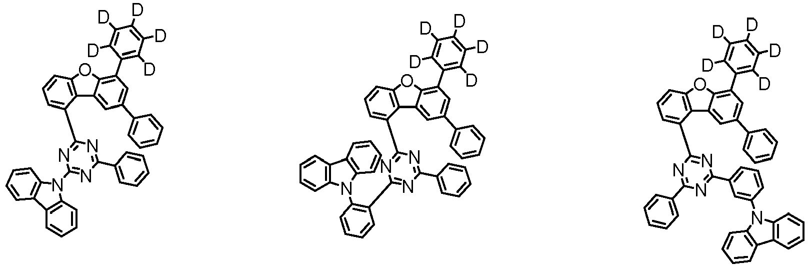

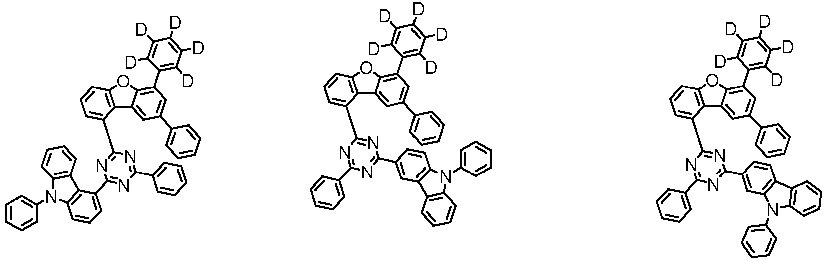

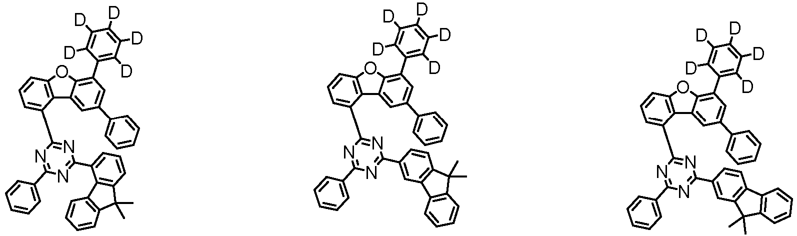

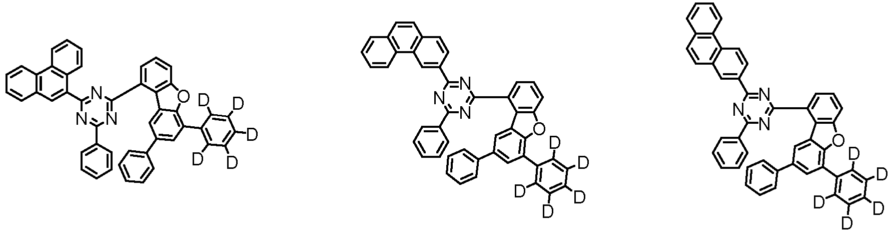

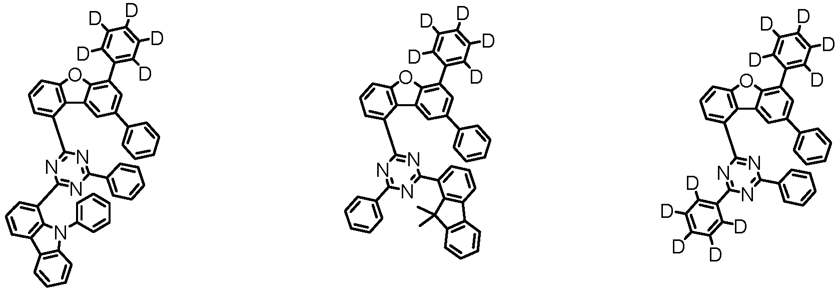

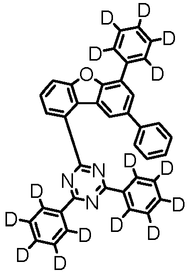

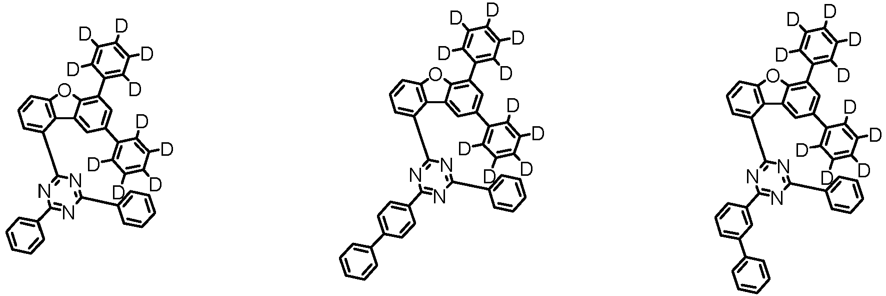

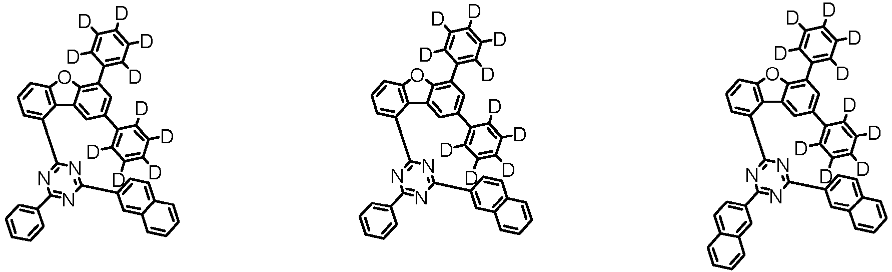

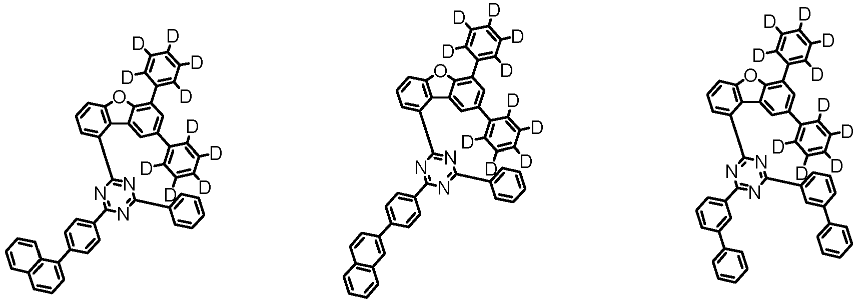

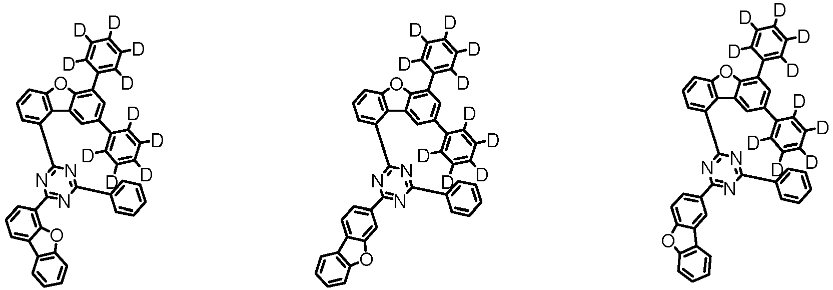

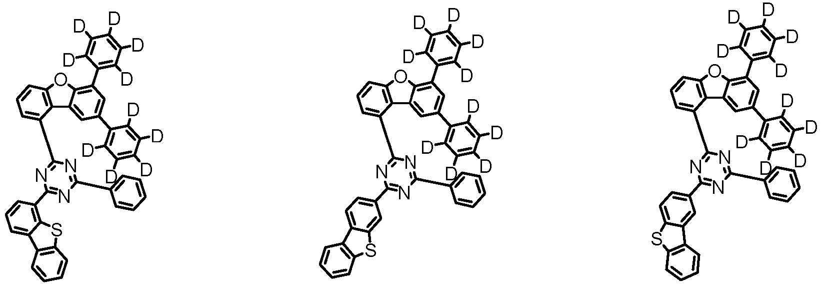

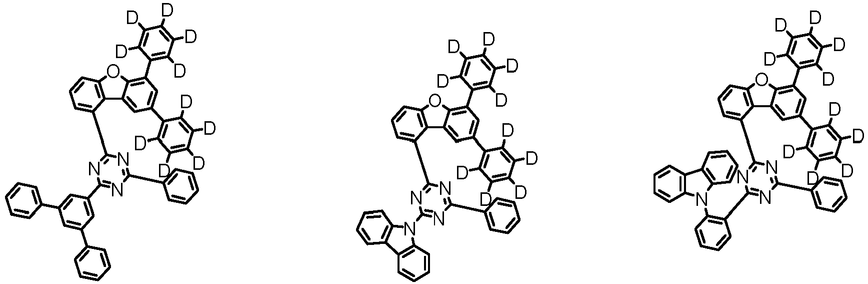

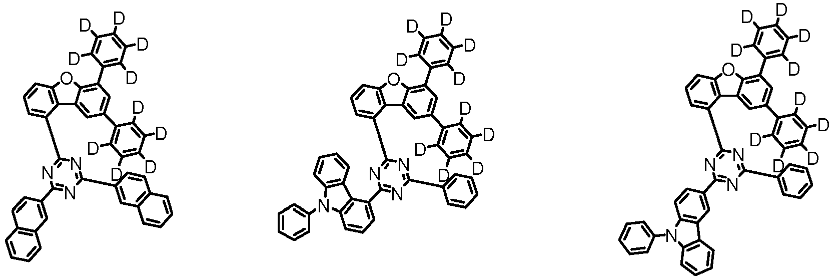

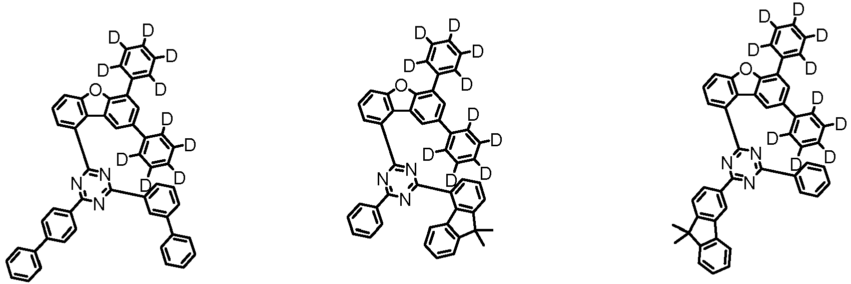

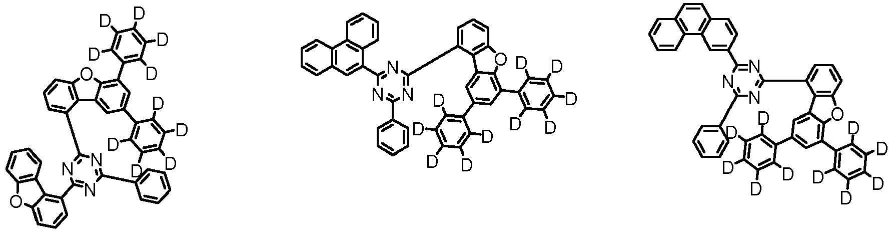

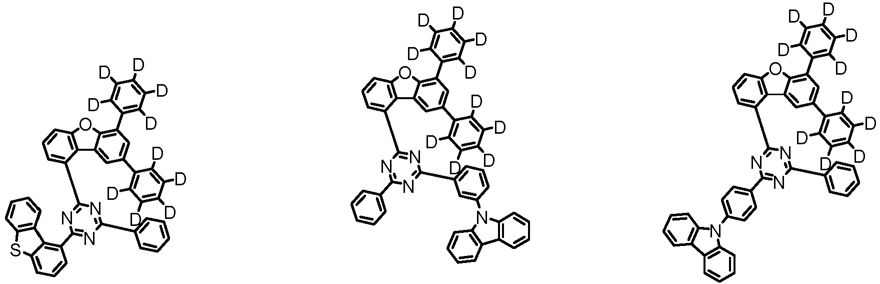

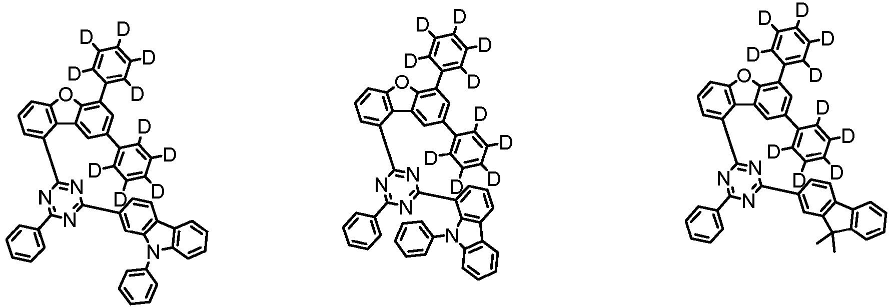

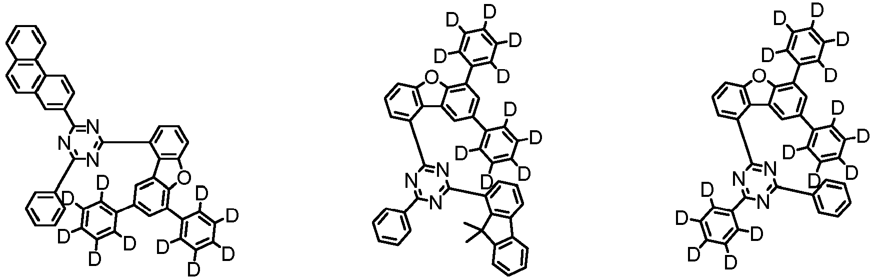

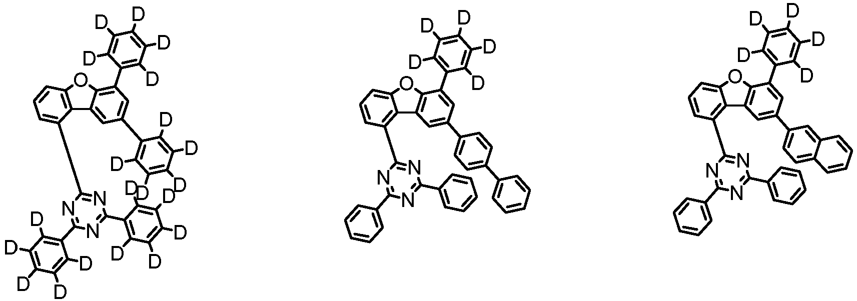

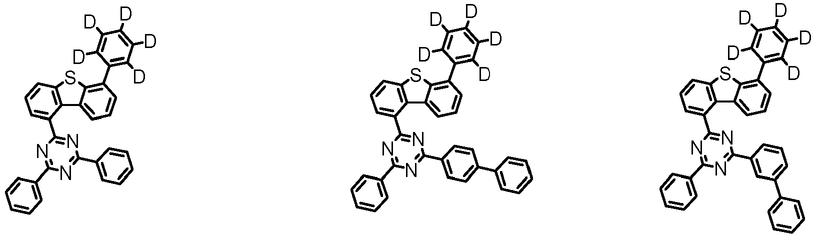

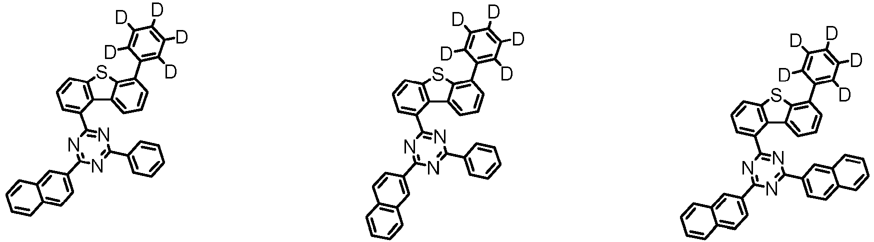

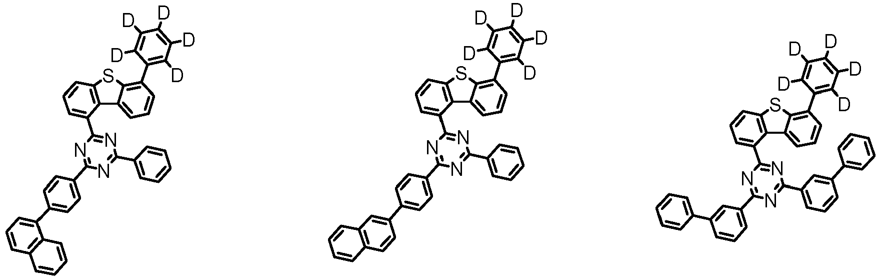

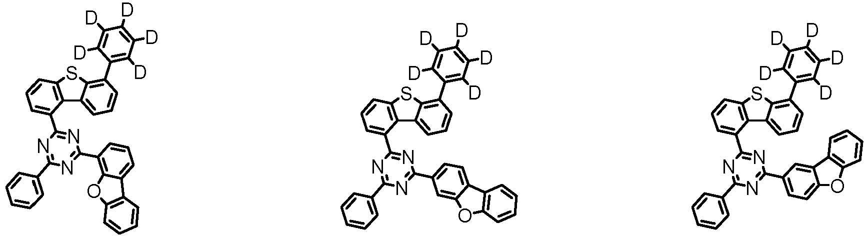

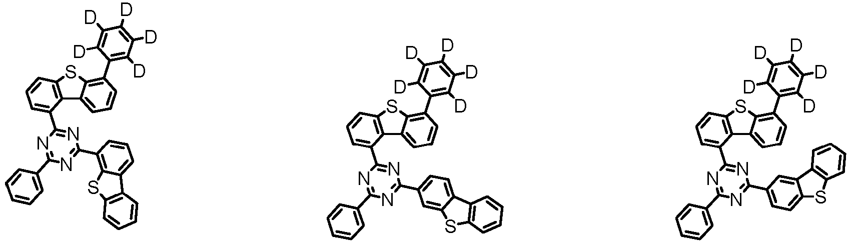

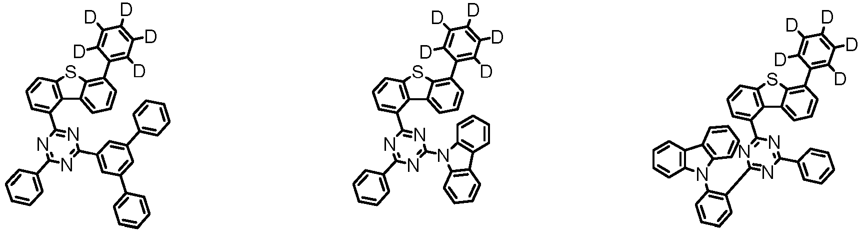

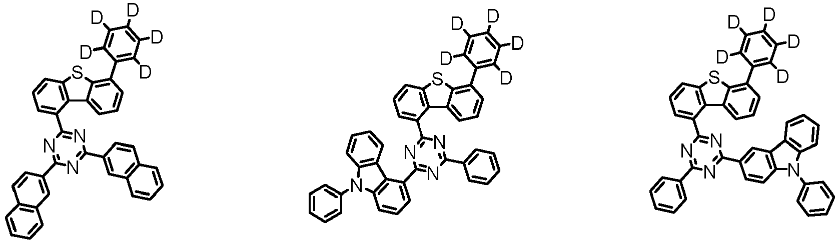

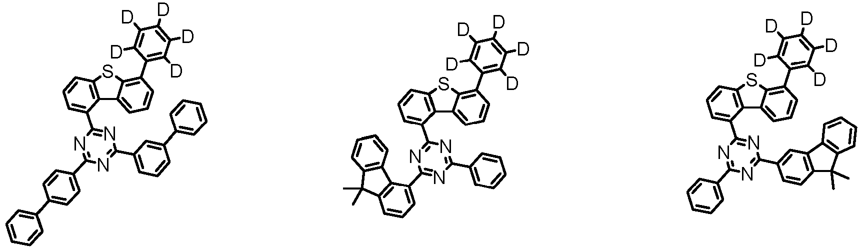

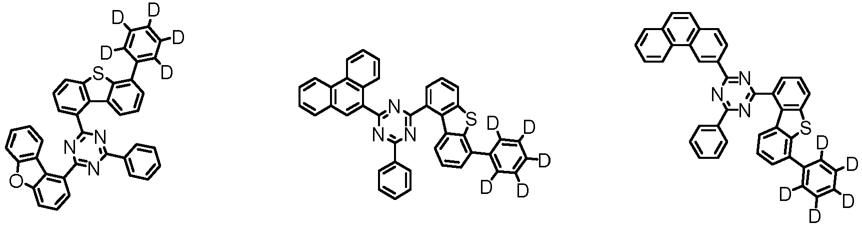

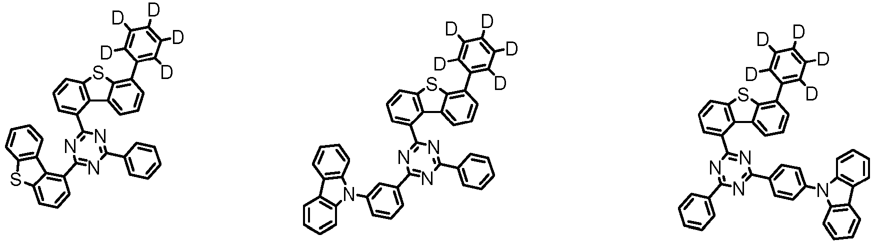

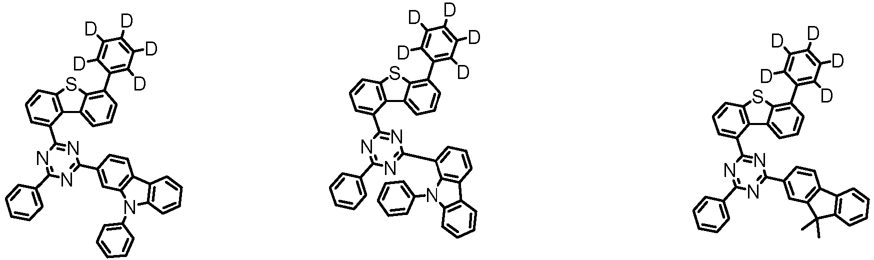

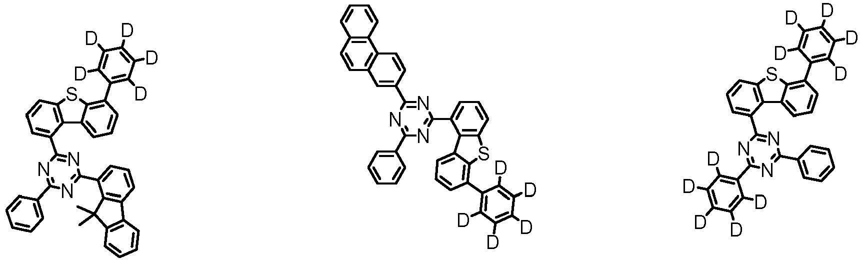

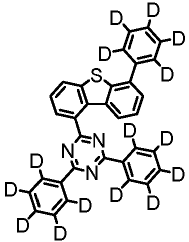

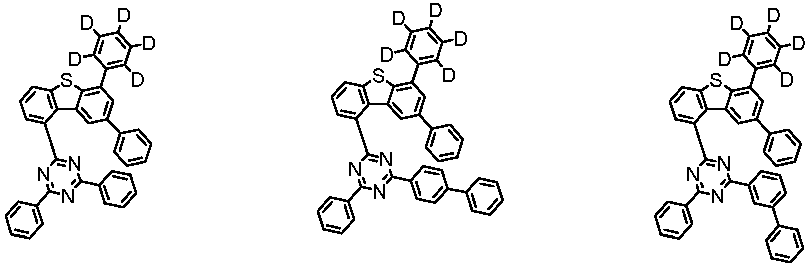

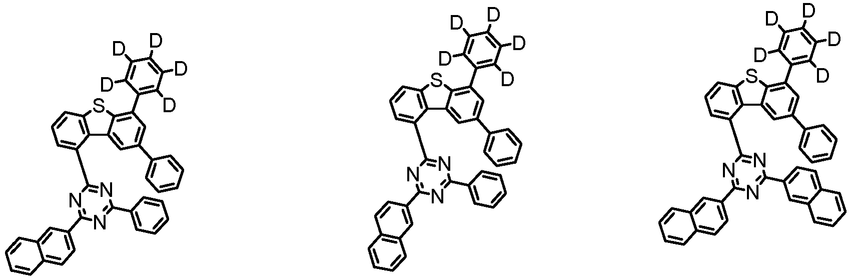

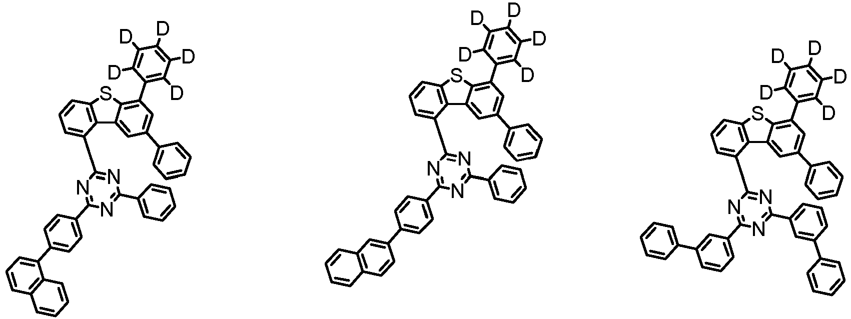

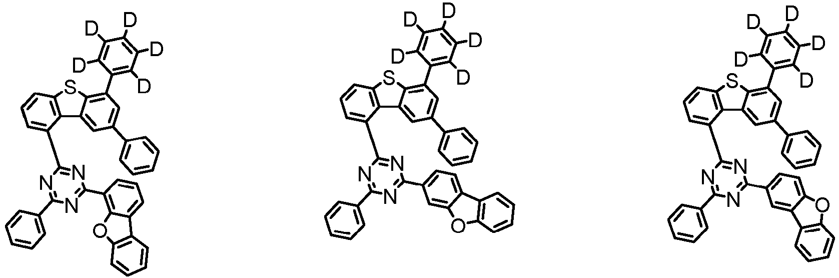

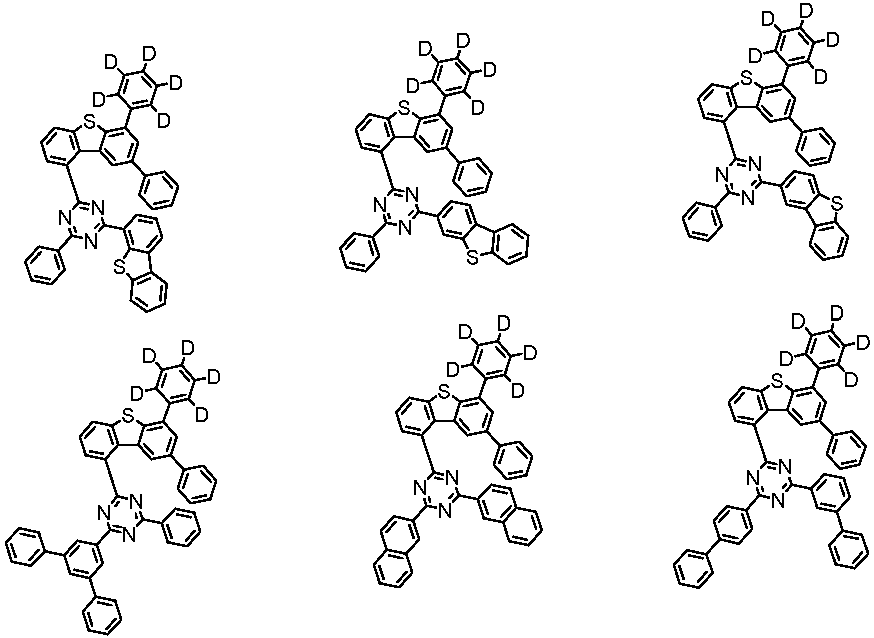

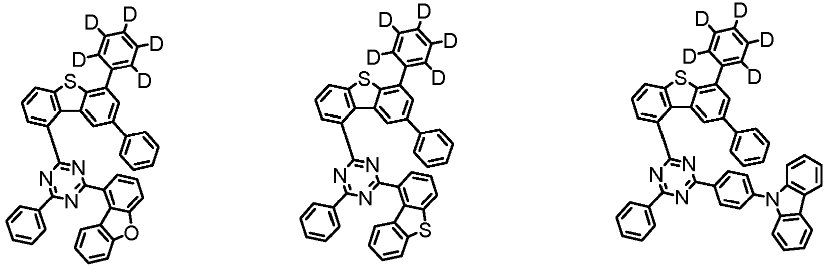

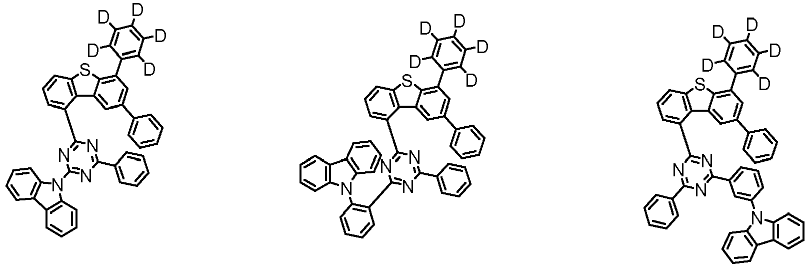

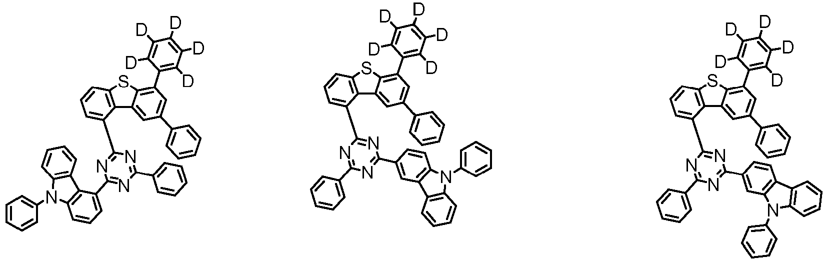

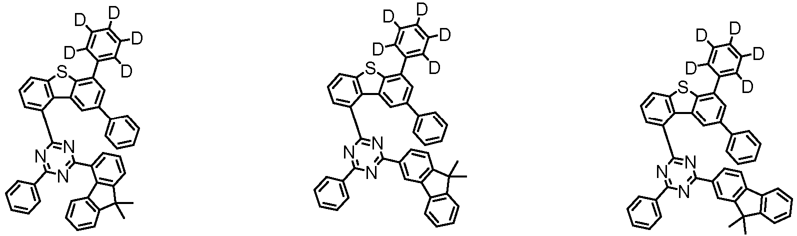

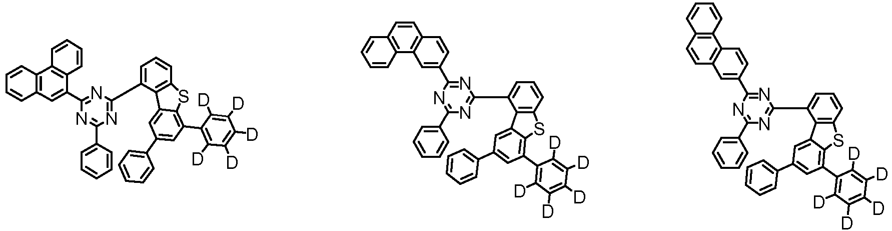

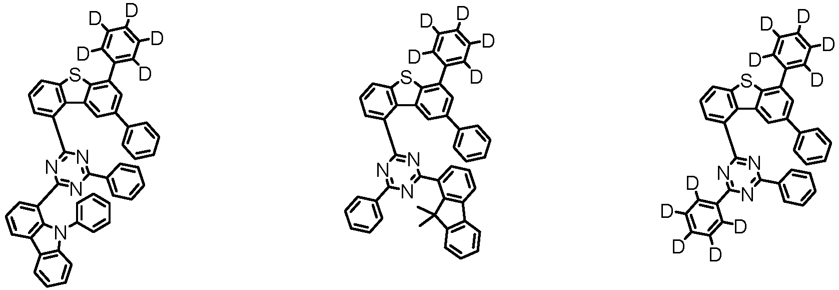

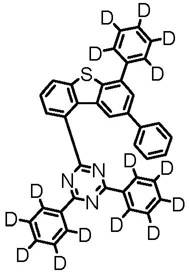

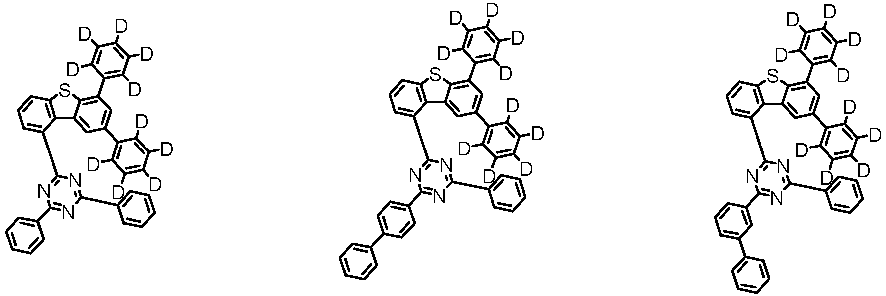

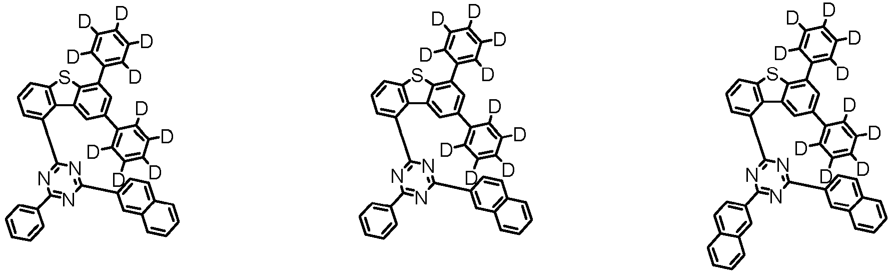

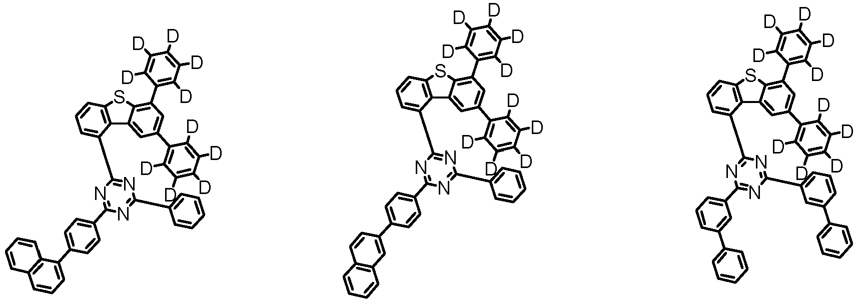

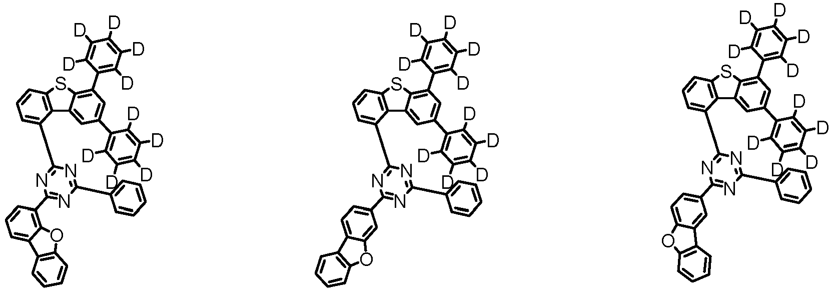

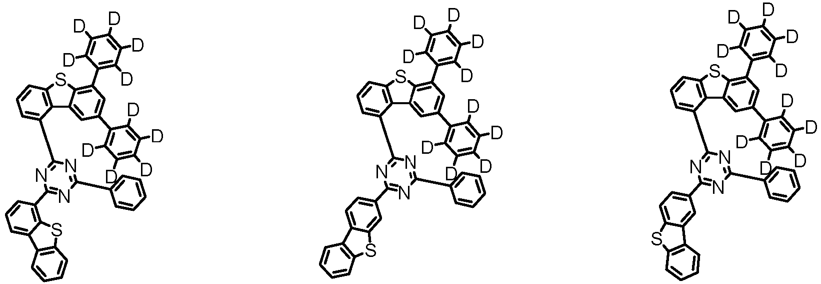

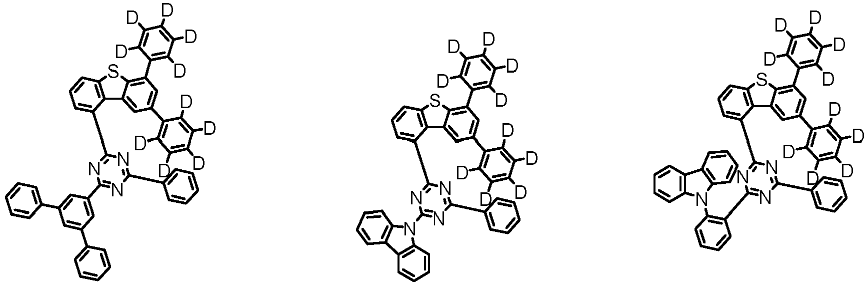

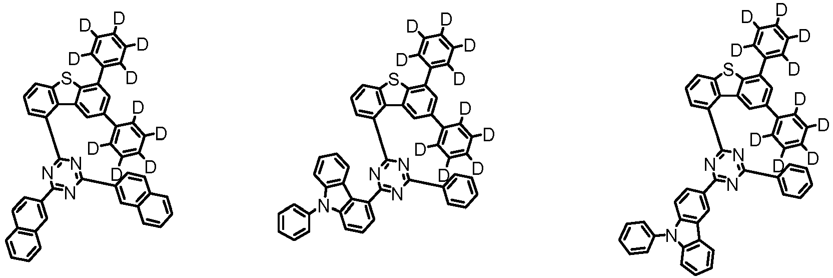

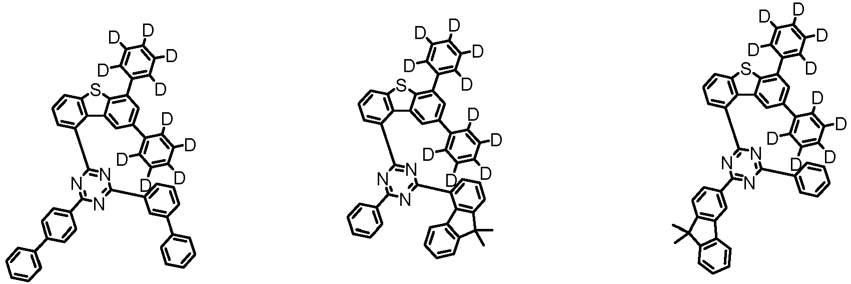

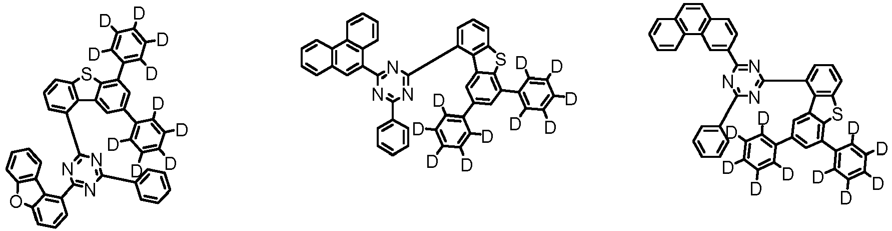

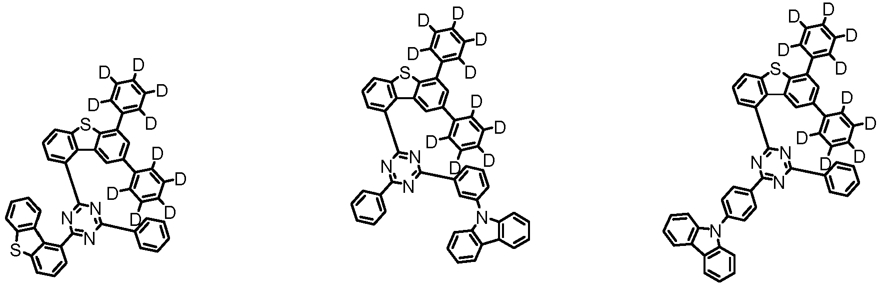

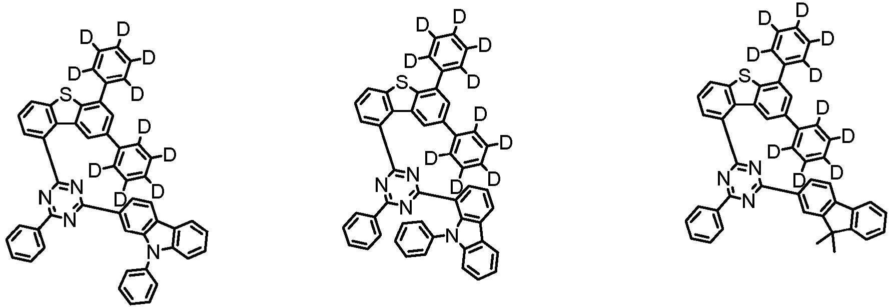

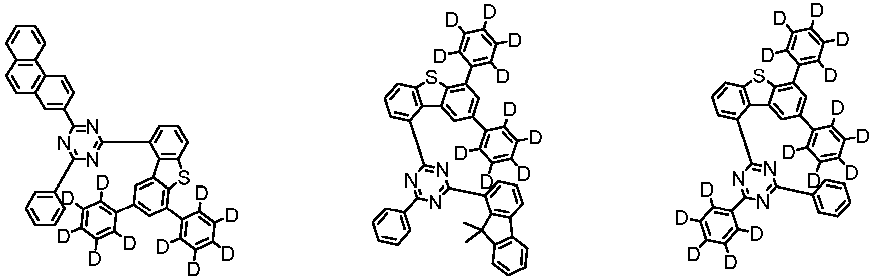

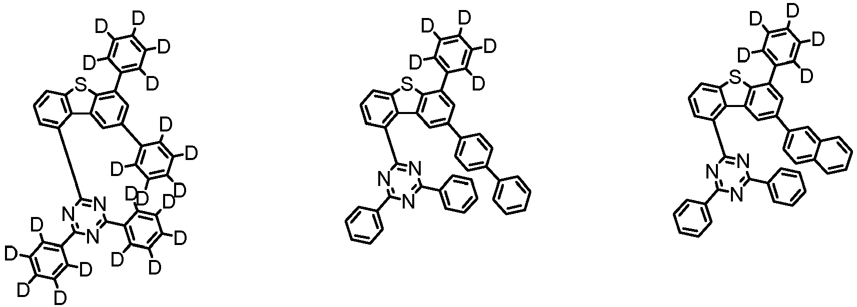

11. An organic light emitting device comprising: a first electrode; a second electrode that is provided opposite to the first electrode; and one or more organic material layers that are provided between the first electrode and the second electrode, wherein at least one layer of the organic material layers comprises the compound according to any one of Claims 1 to 10.

12. The organic light emitting device of Claim 11,
wherein the organic material layer comprising the compound is a light emitting layer.

13. The organic light emitting device of Claim 12,
wherein the light emitting layer further comprises a compound represented by the following Chemical Formula 2: 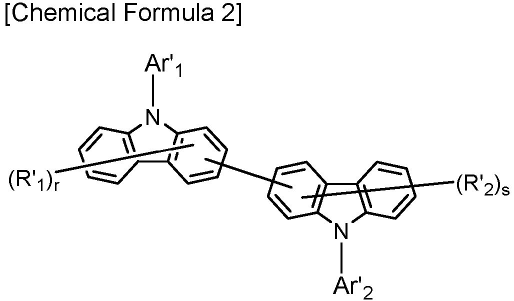
in the Chemical Formula 2,
Ar'₁ and Ar'₂ are each independently substituted or unsubstituted C₆₋₆₀ aryl; or substituted or unsubstituted C₂₋₆₀ heteroaryl containing at least one heteroatom selected from the group consisting of N, O and S, and
R'₁ and R'₂ are each independently hydrogen; deuterium; C₁₋₆₀ alkyl; C₆₋₆₀ aryl; or C₂₋₆₀ heteroaryl containing at least one heteroatom selected from the group consisting of N, O and S; and
r and s are each independently an integer of 0 to 7.

14. The organic light emitting device of Claim 13,
wherein the compound represented by the Chemical Formula 2 is any one selected from the group consisting of the following compounds:

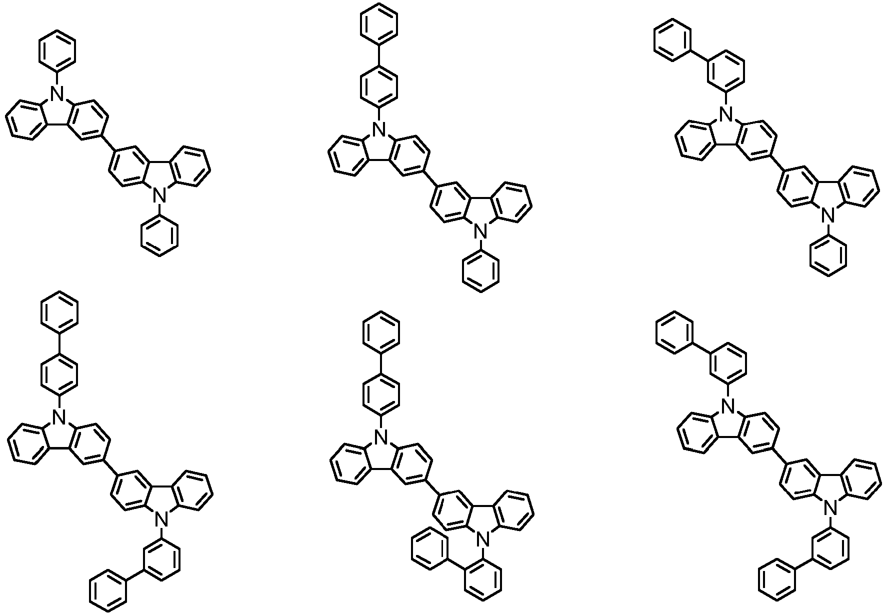

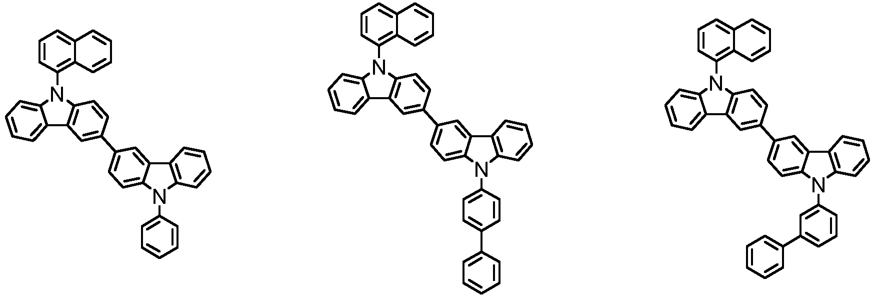

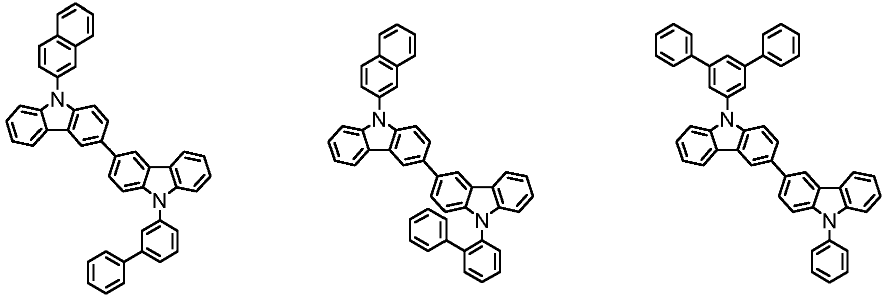

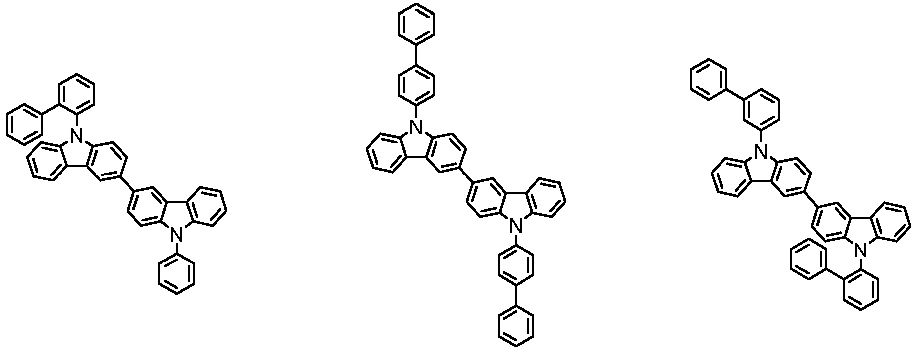

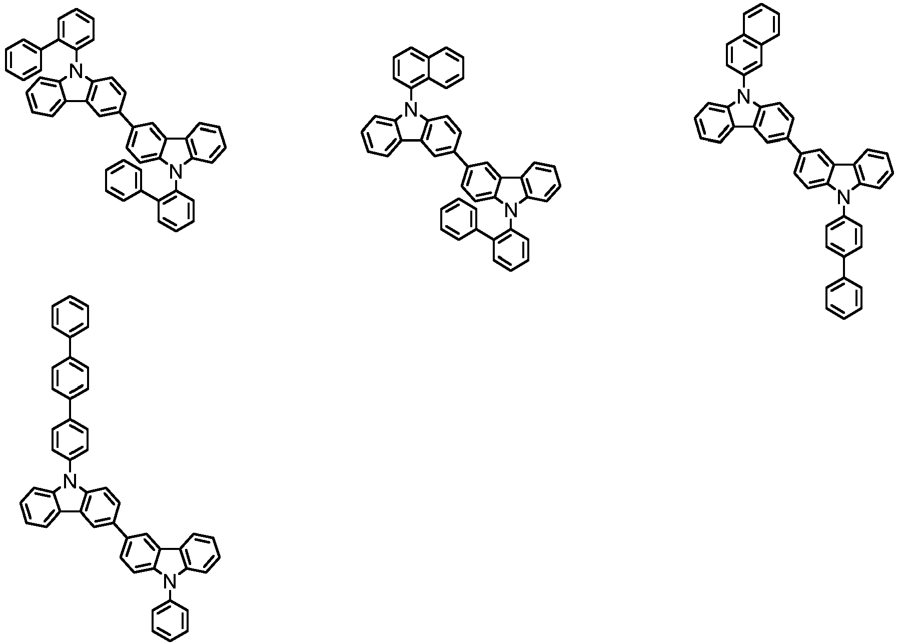

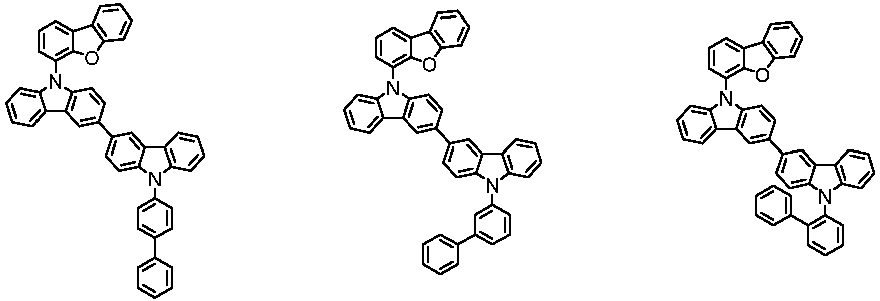

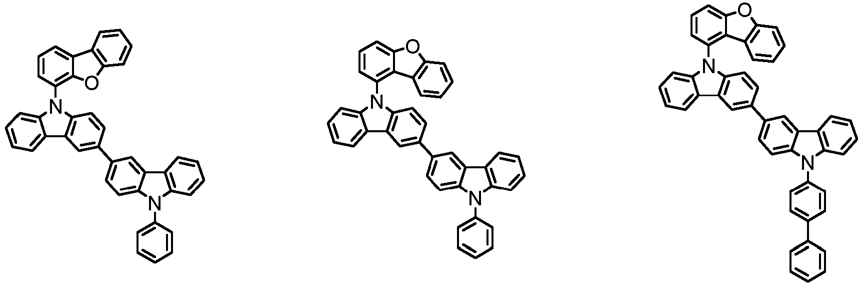

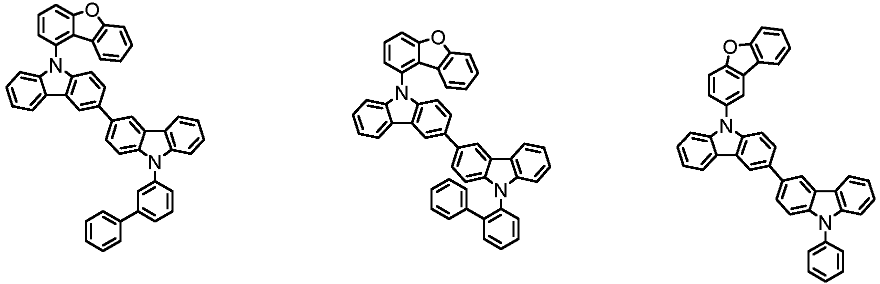

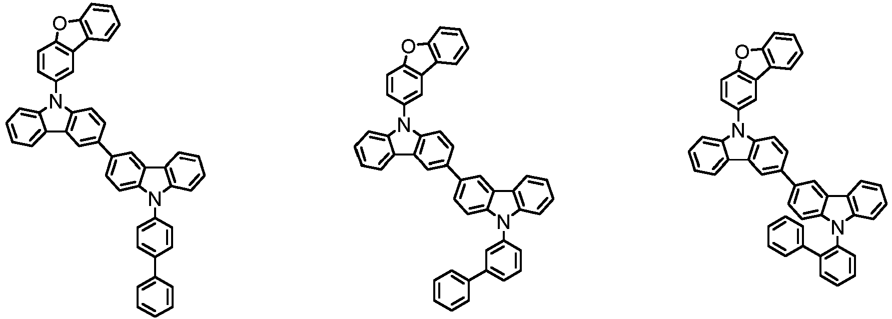

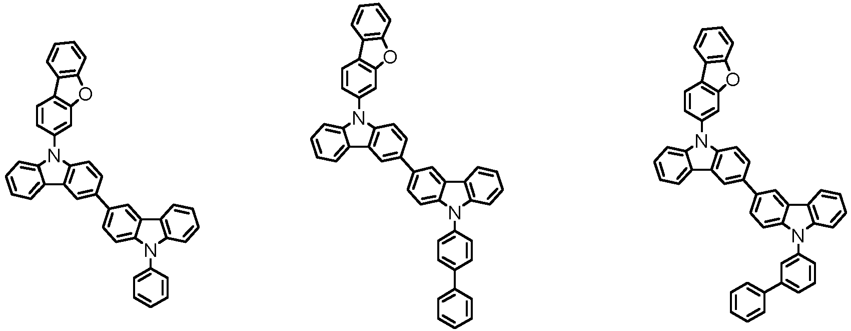

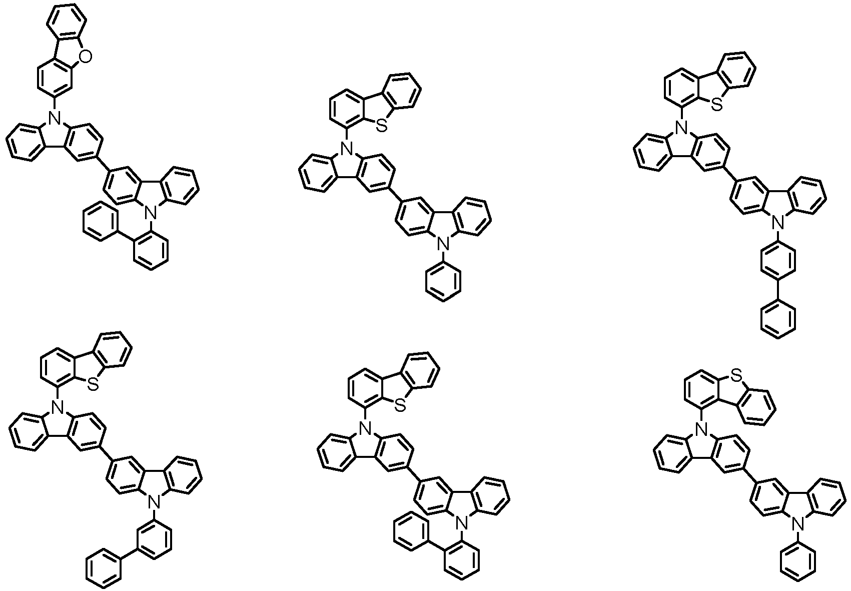

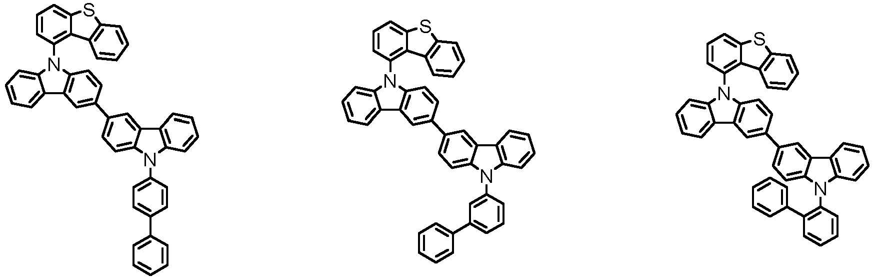

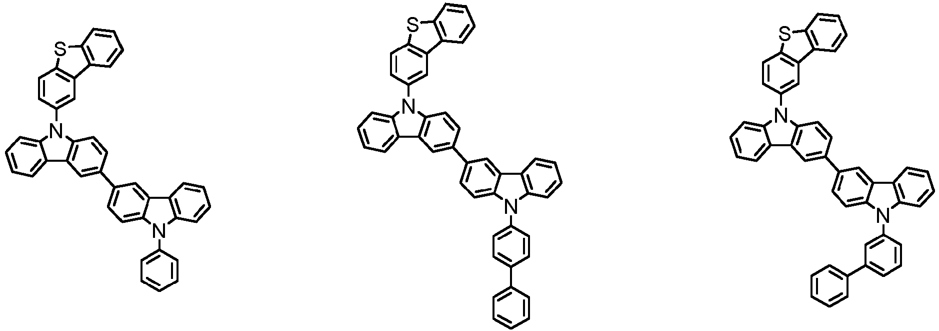

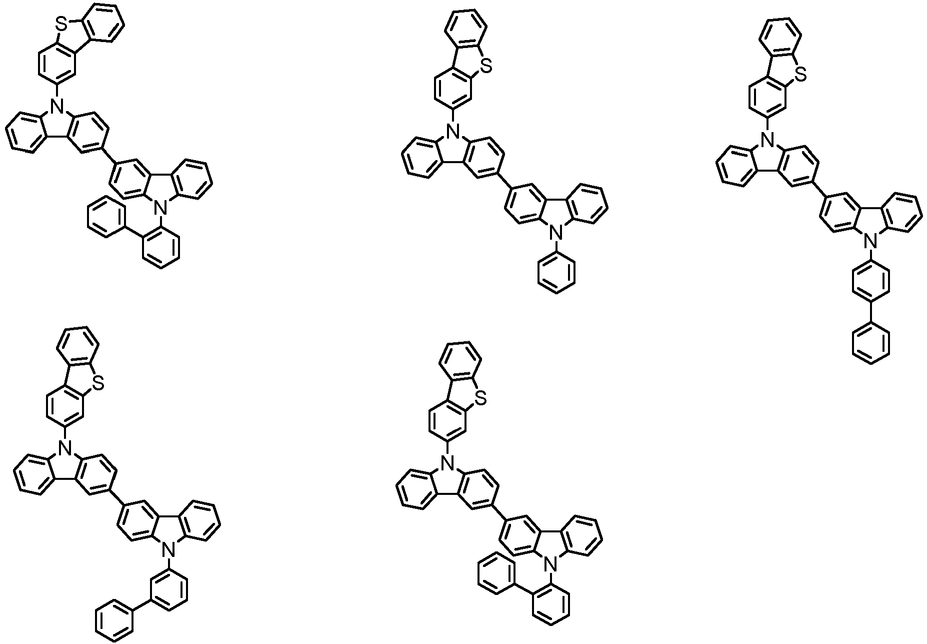

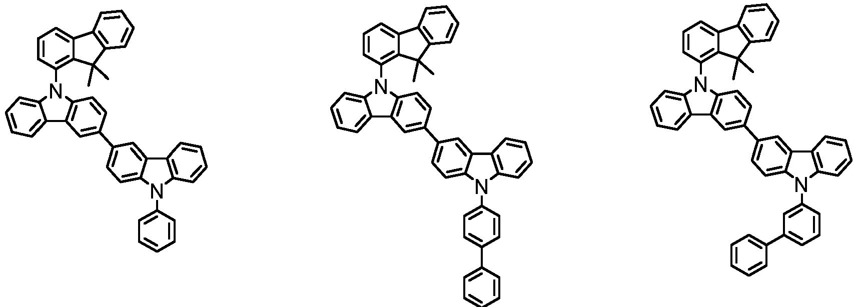

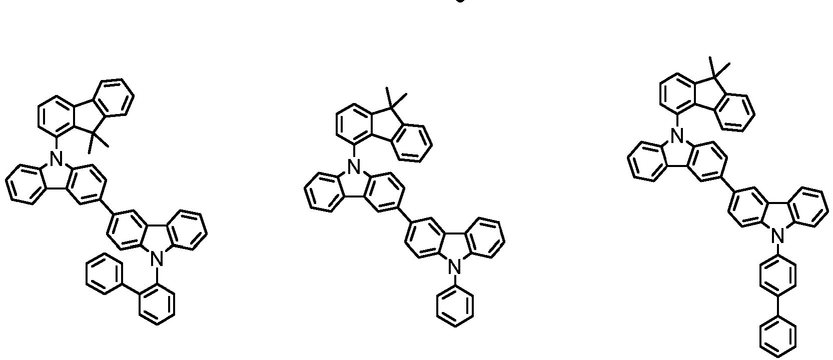

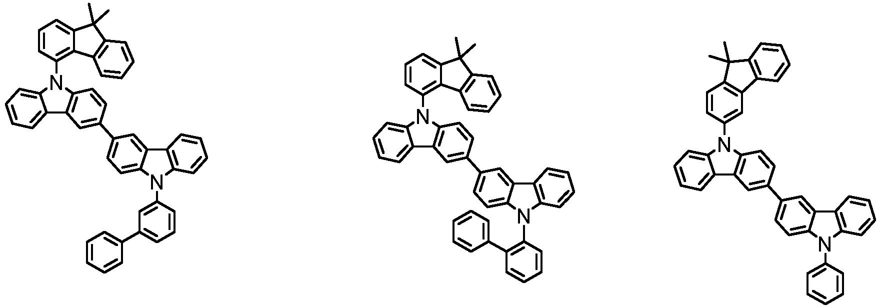

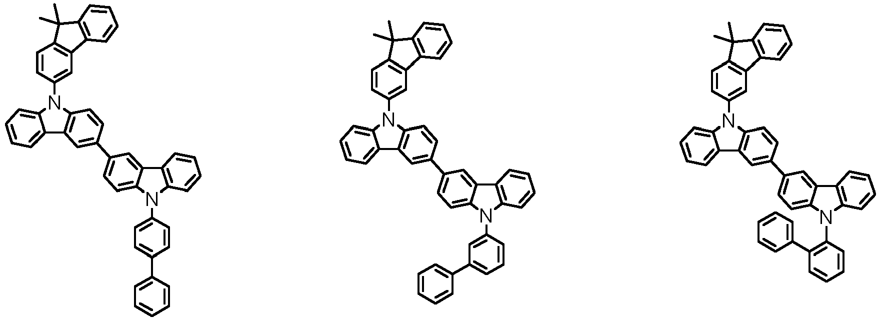

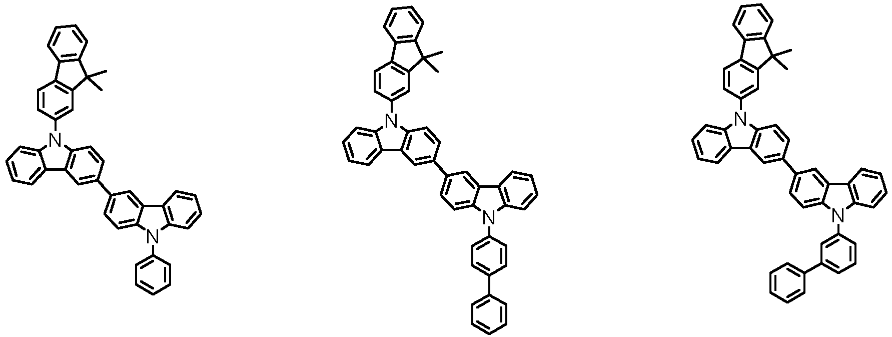

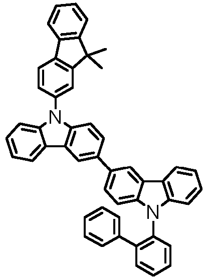

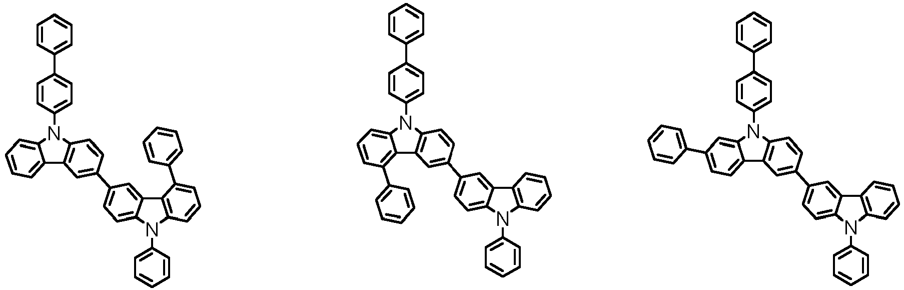

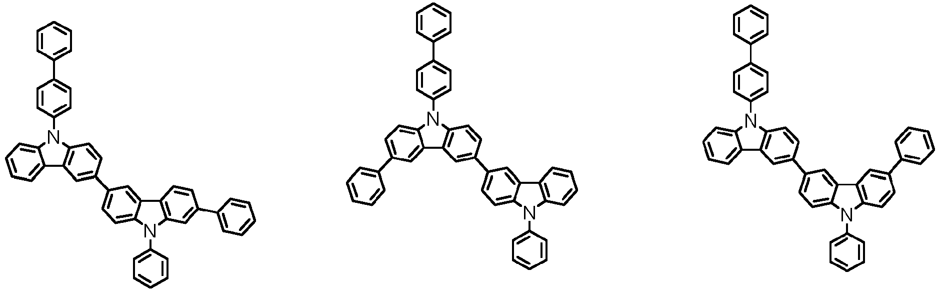

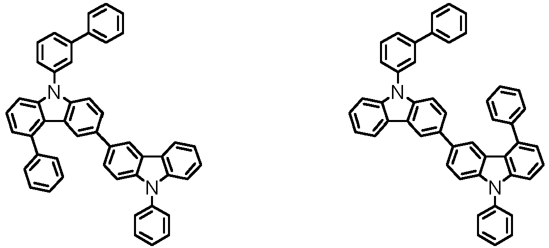

## Patentansprüche

1. Verbindung, dargestellt durch die folgende chemische Formel 1: 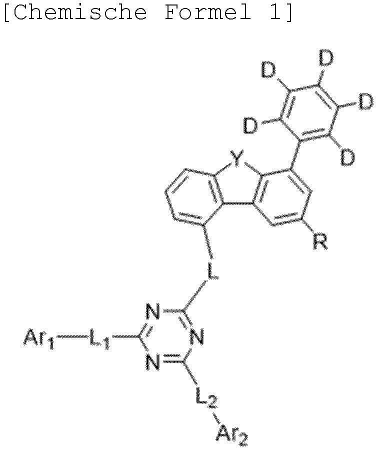 worin in der chemischen Formel 1,
Y O oder S ist,
D Deuterium ist,
L, L₁ und L₂ jeweils unabhängig eine Einfachbindung oder C₆₋₂₀-Arylen, das unsubstituiert oder mit Deuterium substituiert ist, sind,
Ar₁ und Ar₂ jeweils unabhängig C₆₋₂₀-Aryl, das unsubstituiert oder mit mindestens einem Substituenten substituiert ist, der ausgewählt ist aus der Gruppe bestehend aus Deuterium, Methyl und Phenyl; unsubstituiertes Dibenzofuranyl; unsubstituiertes Dibenzothiophenyl; oder C₂₋₂₀-Heteroaryl, das N enthält und unsubstituiert oder substituiert ist mit mindestens einem Substituenten, der ausgewählt ist aus der Gruppe bestehend aus Deuterium, Methyl und Phenyl, sind, und
R Wasserstoff; oder C₆₋₆₀-Aryl ist, das unsubstituiert oder substituiert mit Deuterium ist.

2. Verbindung gemäß Anspruch 1,
worin L eine Einfachbindung ist.

3. Verbindung gemäß Anspruch 1,
worin L₁ und L₂ jeweils unabhängig eine Einfachbindung oder Phenylen sind.

4. Verbindung gemäß Anspruch 1,
worin Ar₁ und Ar₂ jeweils unabhängig Phenyl, Biphenylyl, Terphenylyl, Naphthyl, Phenanthryl, Fluorenyl, Dibenzofuranyl, Dibenzothiophenyl oder Carbazolyl sind und
das Phenyl, Biphenylyl, Terphenylyl, Naphthyl, Phenanthryl, Fluorenyl und Carbazolyl jeweils unabhängig unsubstituiert oder mit mindestens einem Substituenten substituiert sind, der ausgewählt ist aus der Gruppe bestehend aus Deuterium, Methyl und Phenyl.

5. Verbindung gemäß Anspruch 4,
worin Ar₁ und Ar₂ jeweils unabhängig ausgewählt sind aus der Gruppe bestehend aus den folgenden:

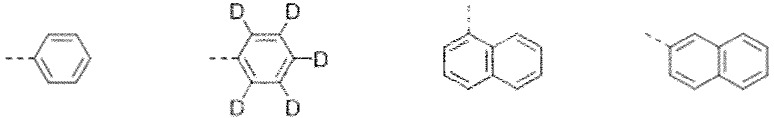

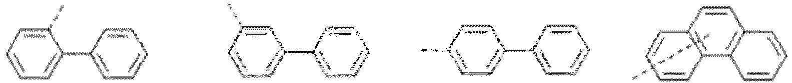

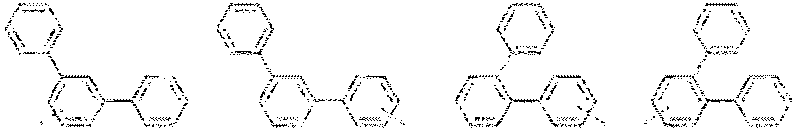

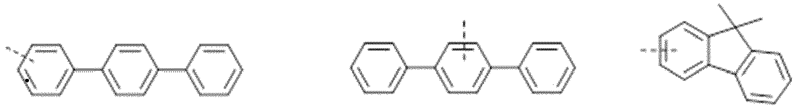

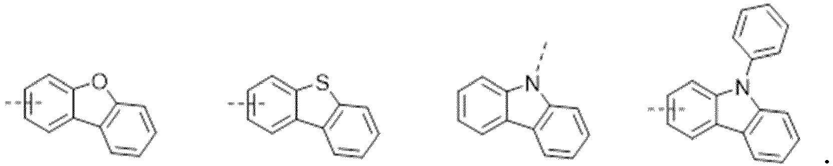

6. Verbindung gemäß Anspruch 1,
worin Ar₁ und Ar₂ jeweils unabhängig C₆₋₁₂-Aryl sind, das unsubstituiert oder mit Deuterium substituiert ist.

7. Verbindung gemäß Anspruch 1,
worin R Wasserstoff; Phenyl, das unsubstituiert oder mit Deuterium substituiert ist; Biphenylyl, das unsubstituiert oder mit Deuterium substituiert ist; oder Naphthyl, das unsubstituiert oder mit Deuterium substituiert ist, ist.

8. Verbindung gemäß Anspruch 7,
worin R Wasserstoff, 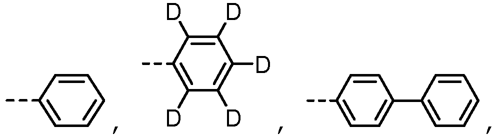 oder 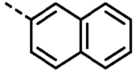 ist.

9. Verbindung gemäß Anspruch 1,
wobei die Verbindung dargestellt ist durch eine der folgenden chemischen Formeln 1-1 bis 1-5: 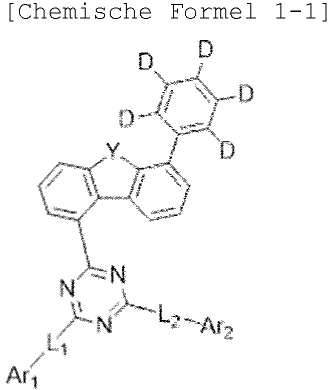 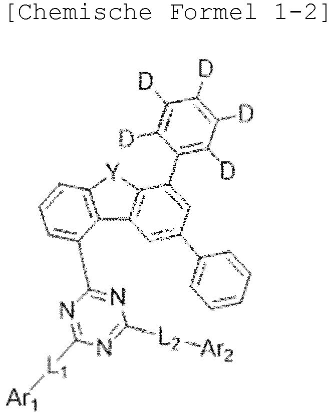 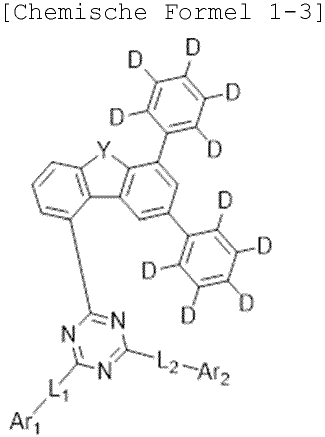 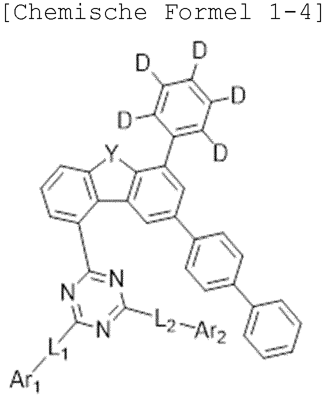 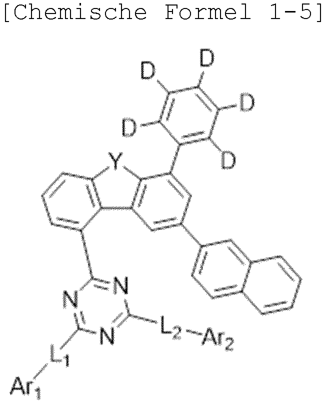
worin in den chemischen Formeln 1-1 bis 1-5
Y, L₁, L₂, Ar₁ und Ar₂ wie in Anspruch 1 definiert sind.

10. Verbindung gemäß Anspruch 1,
wobei die Verbindung eine beliebige ist, die ausgewählt ist aus der Gruppe bestehend aus den folgenden Verbindungen:

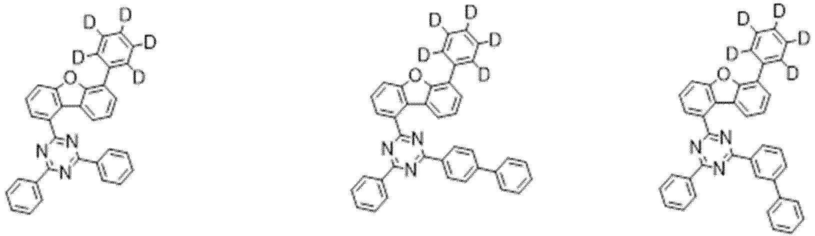

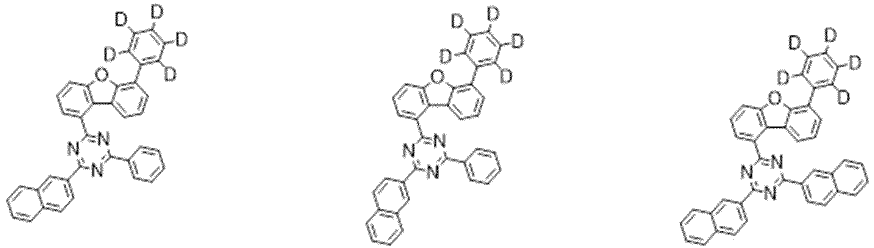

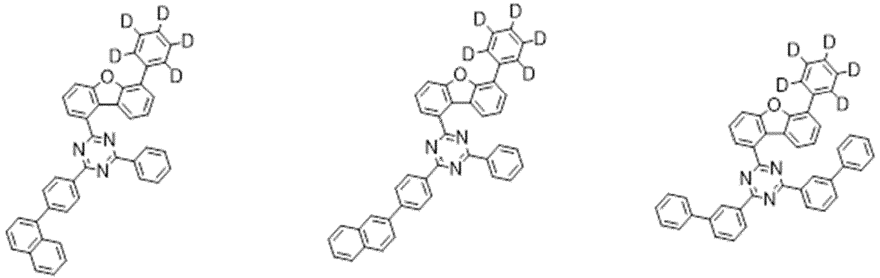

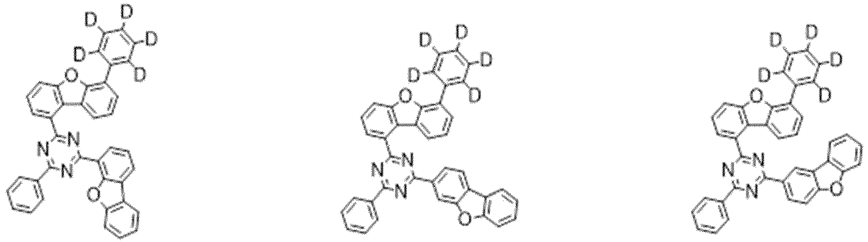

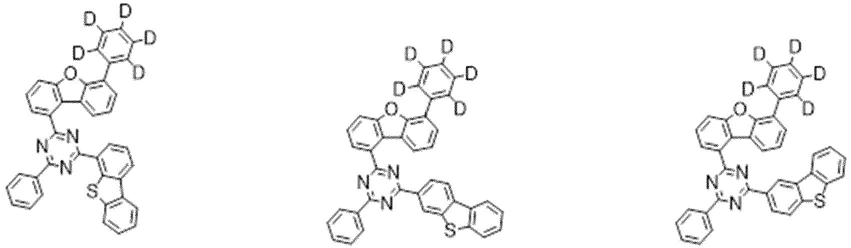

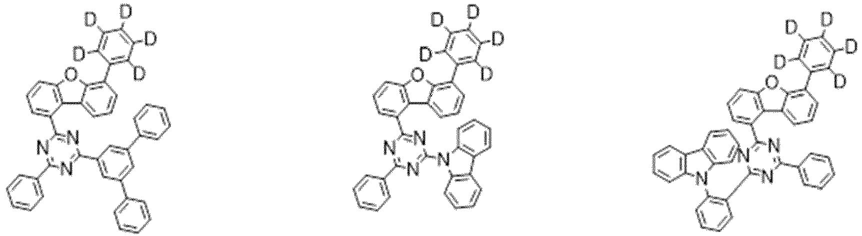

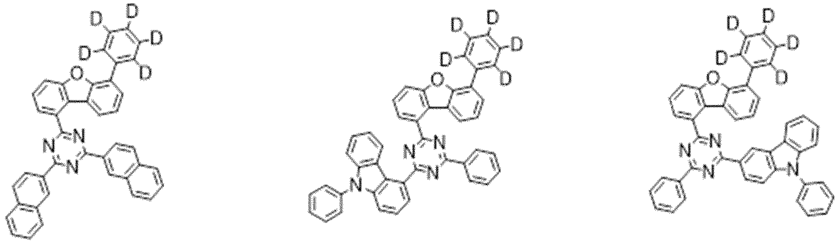

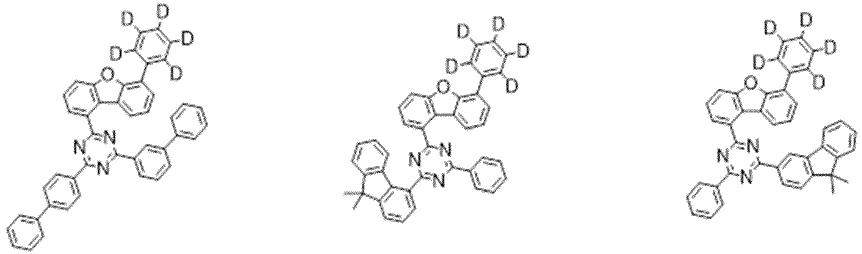

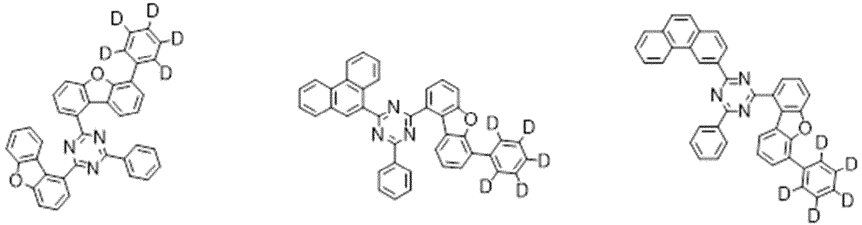

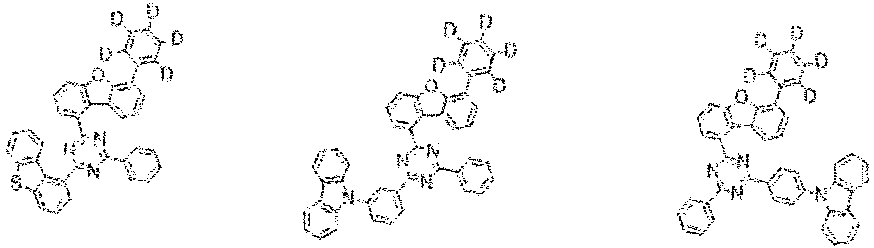

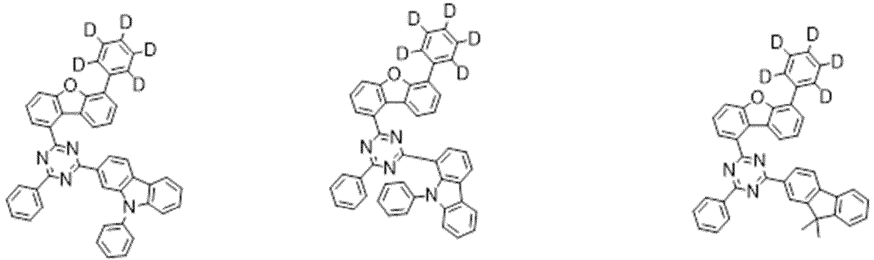

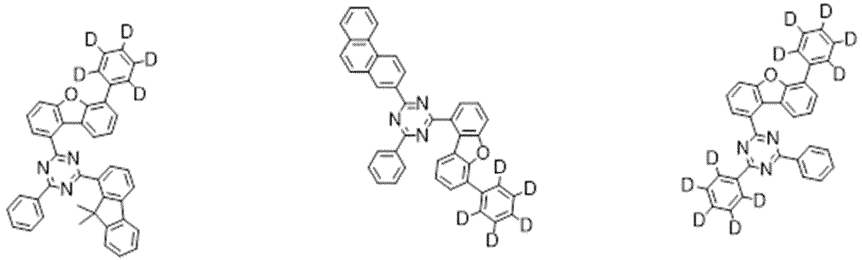

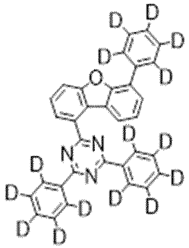

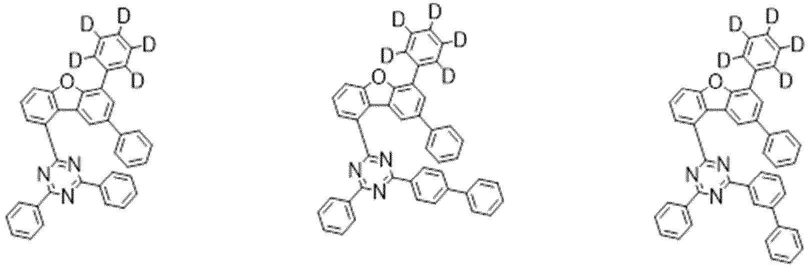

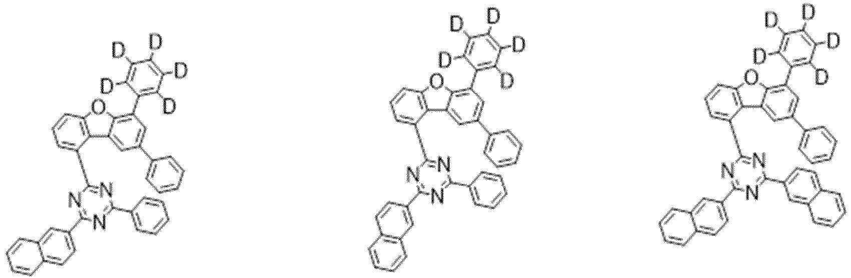

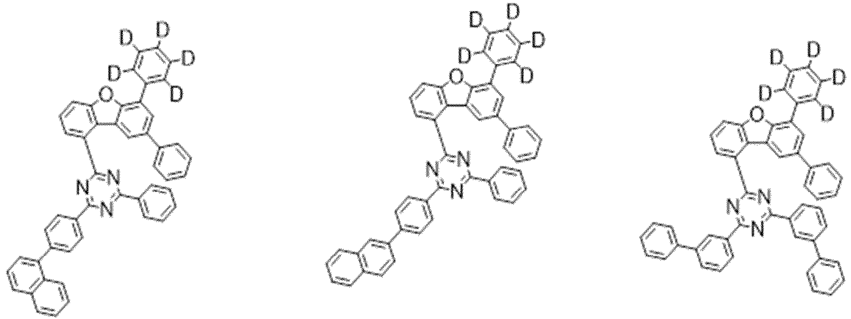

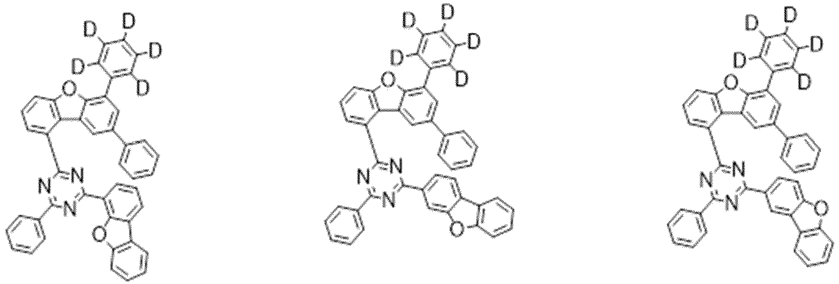

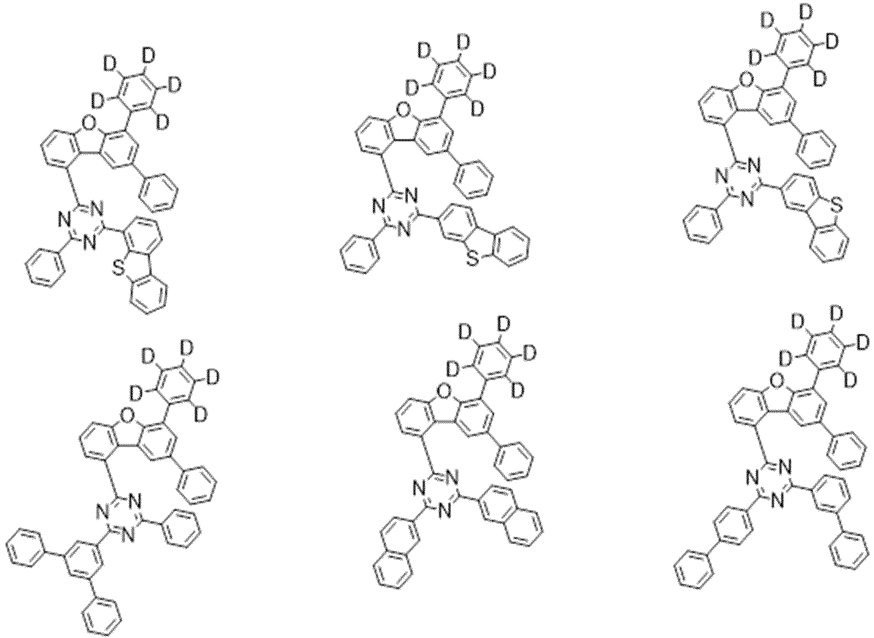

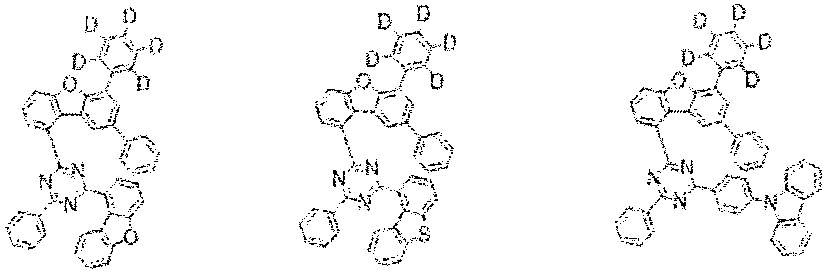

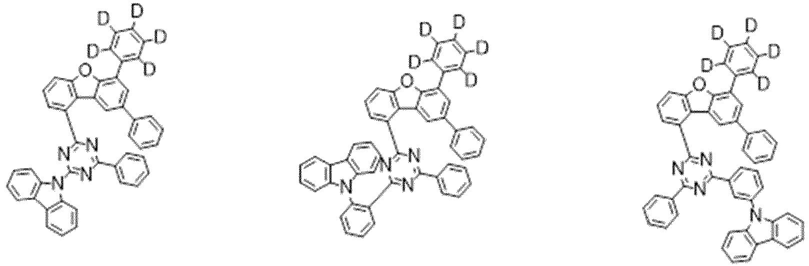

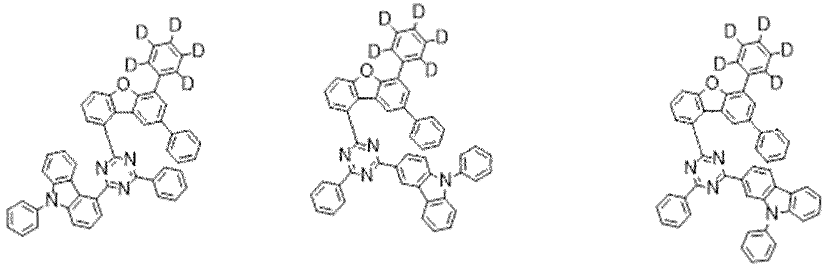

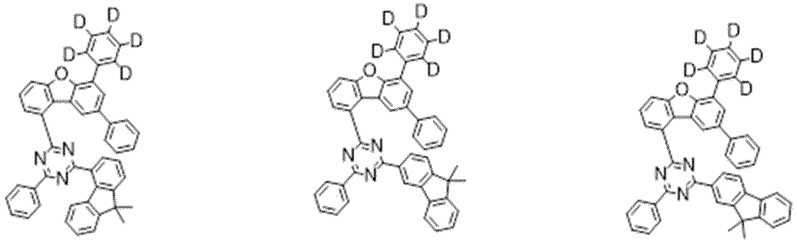

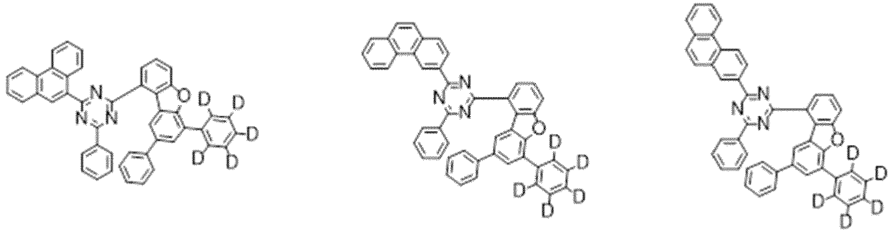

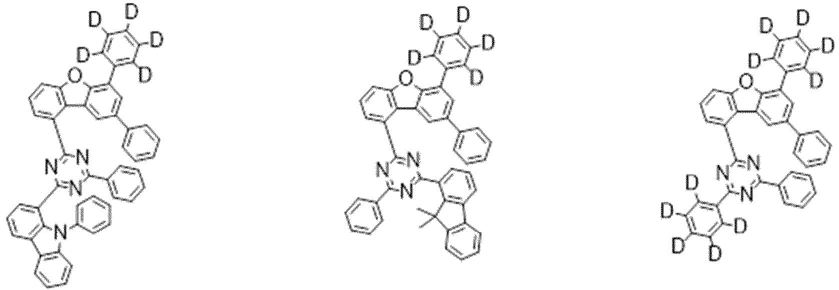

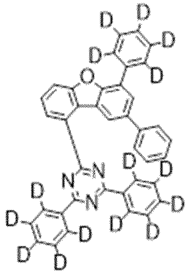

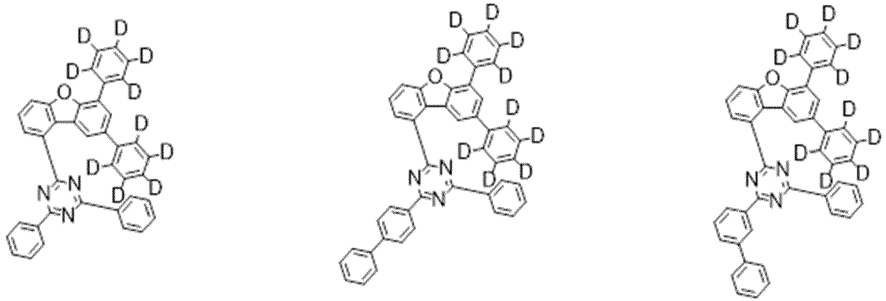

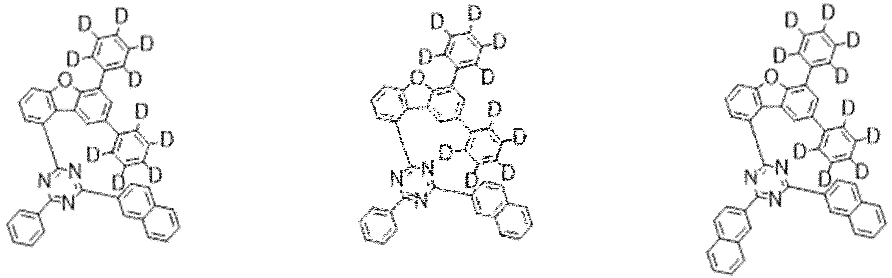

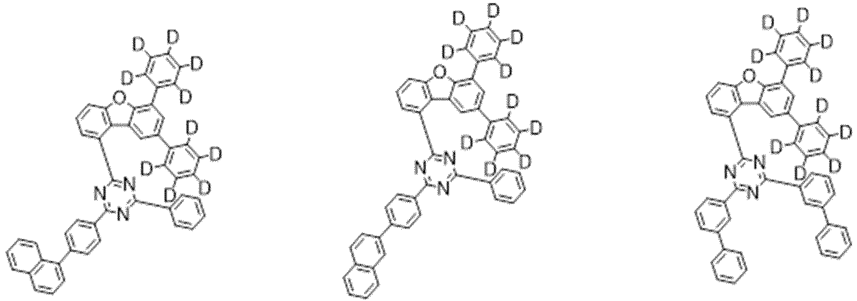

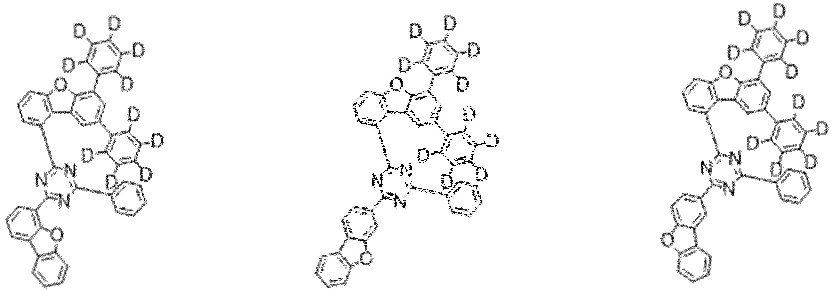

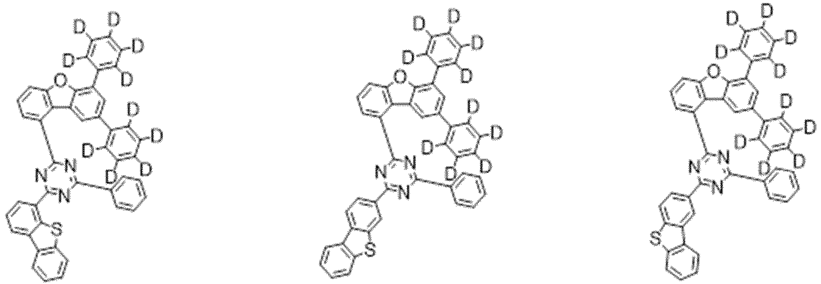

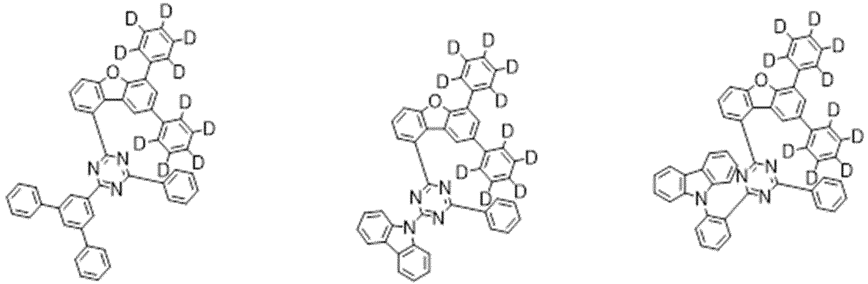

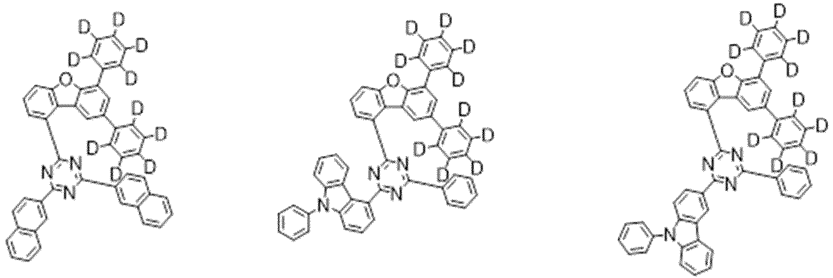

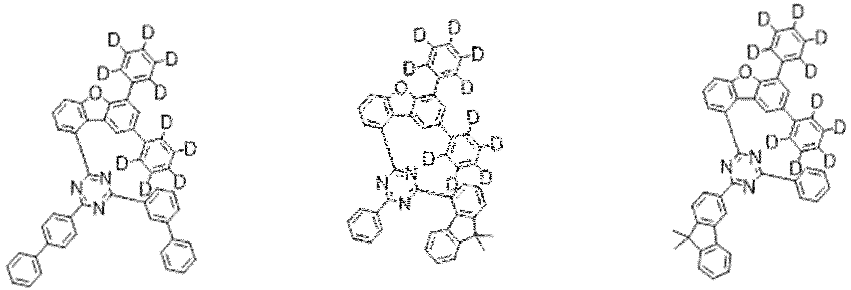

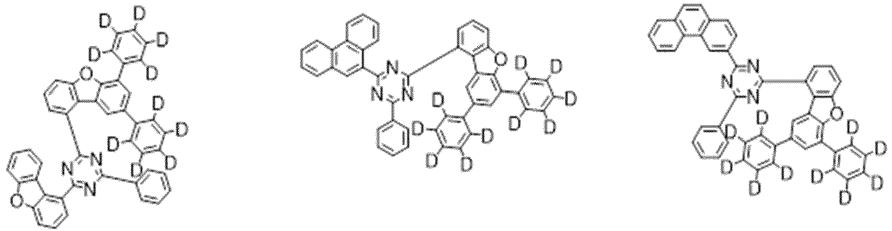

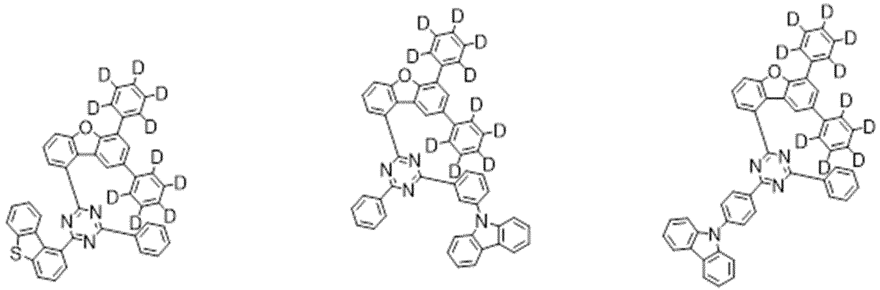

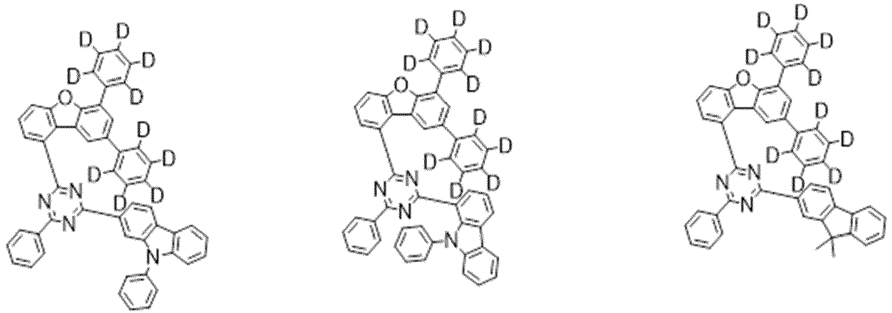

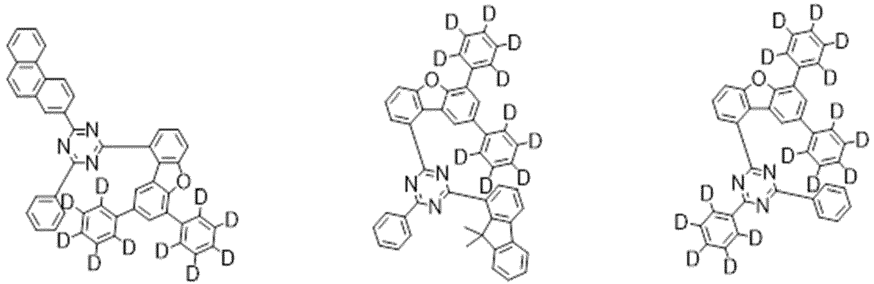

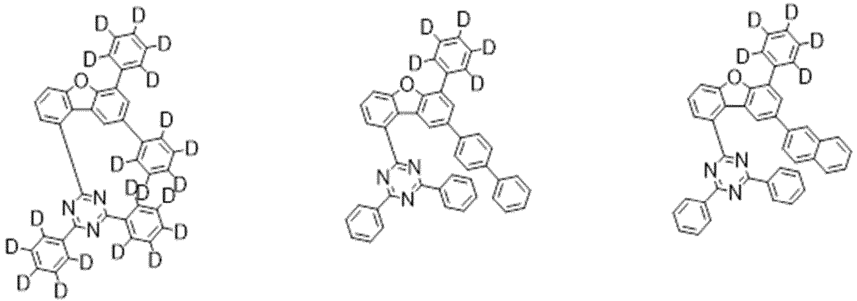

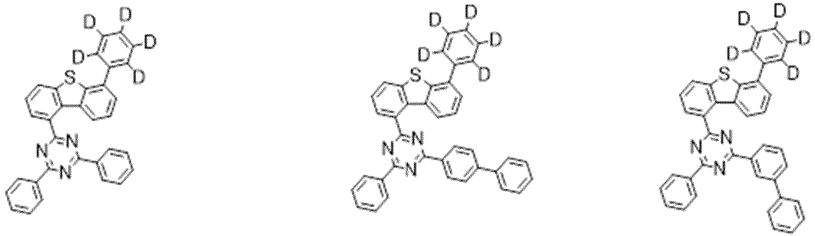

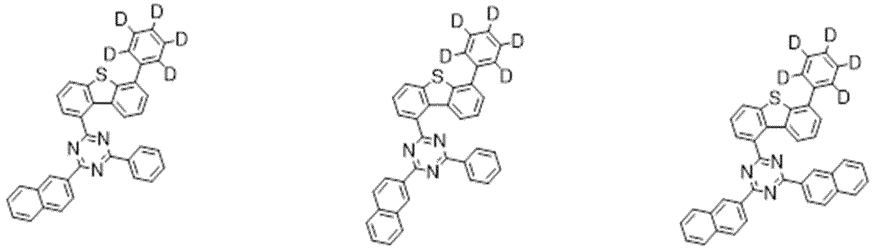

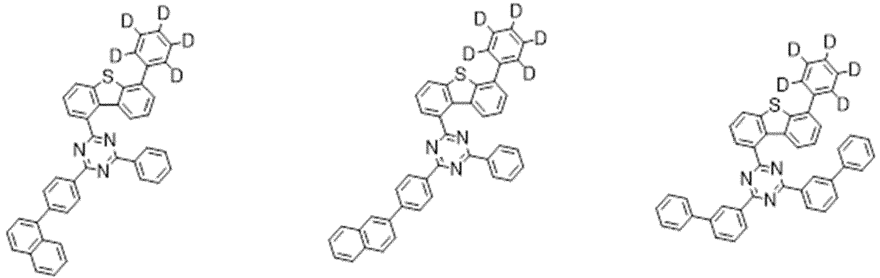

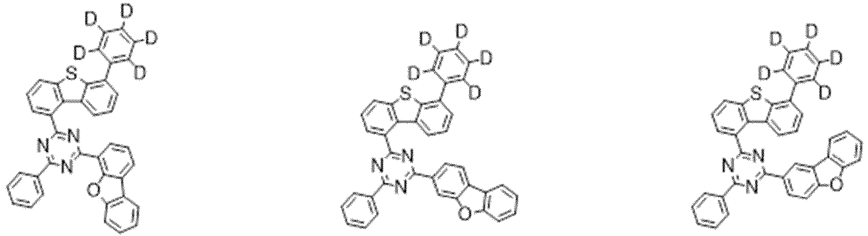

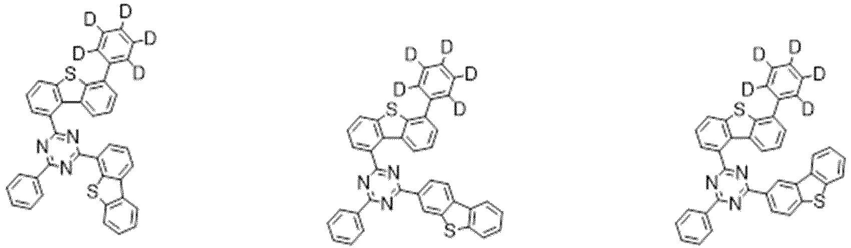

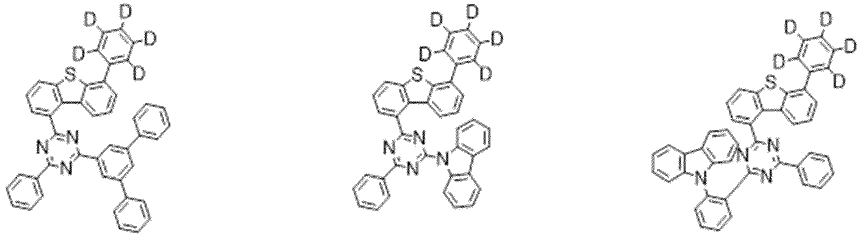

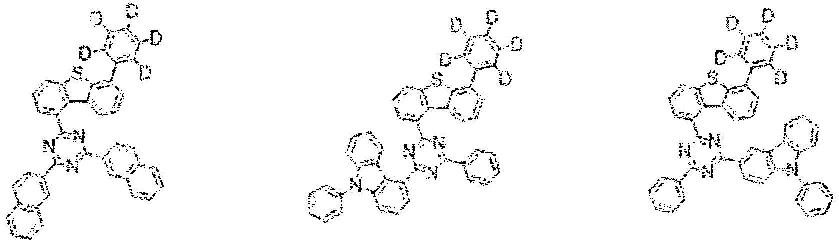

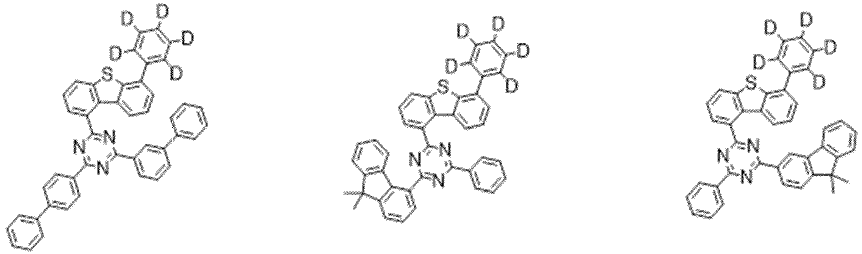

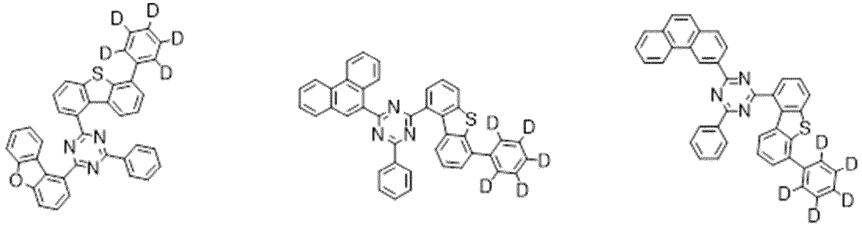

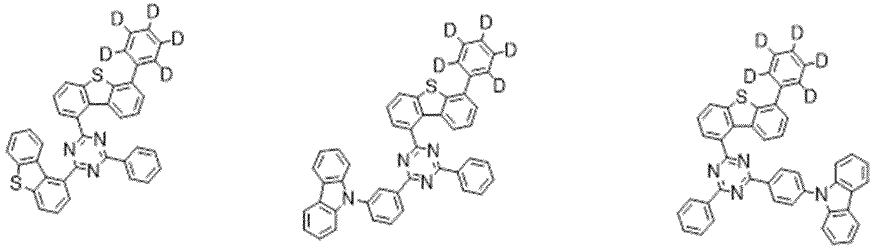

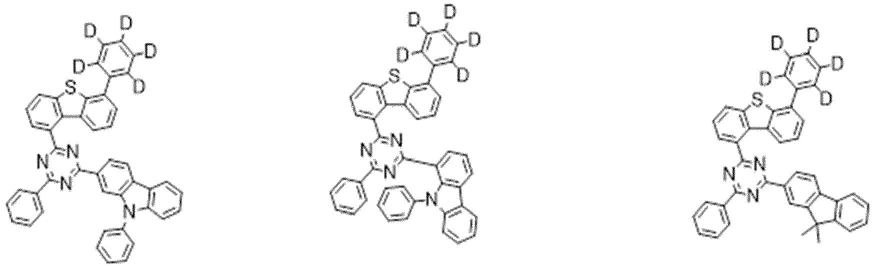

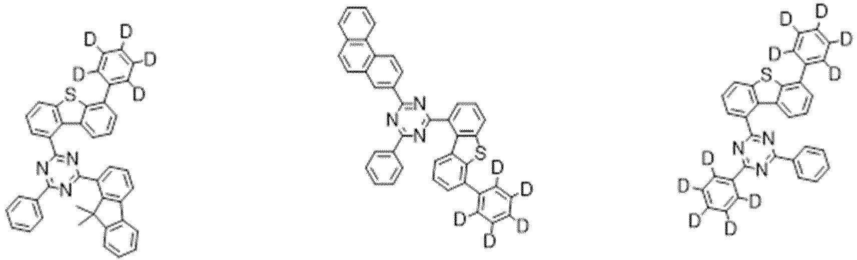

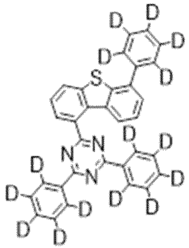

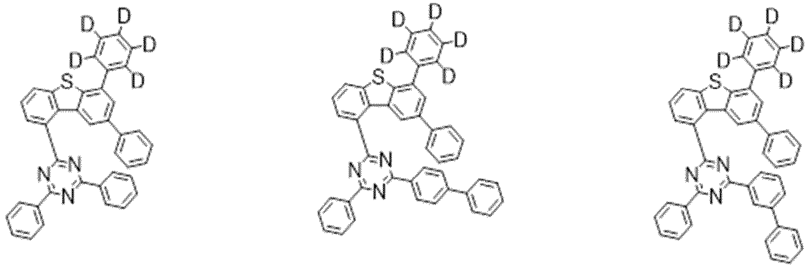

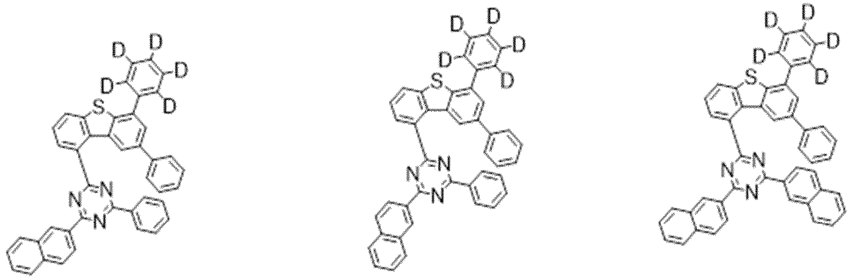

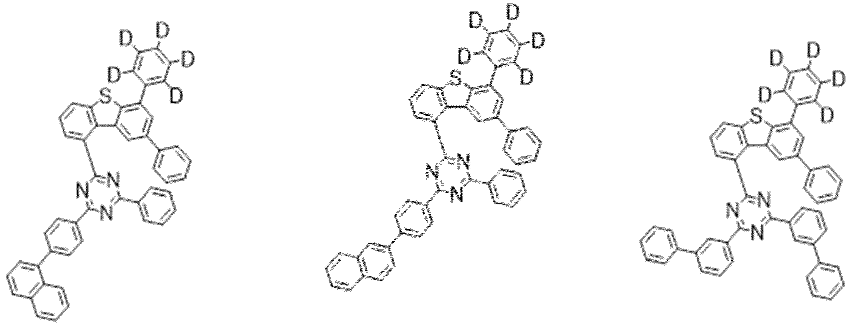

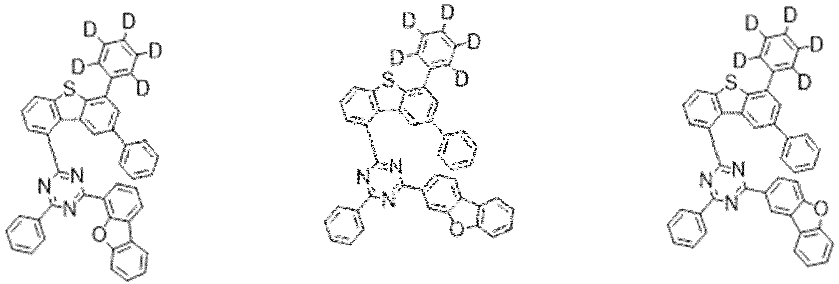

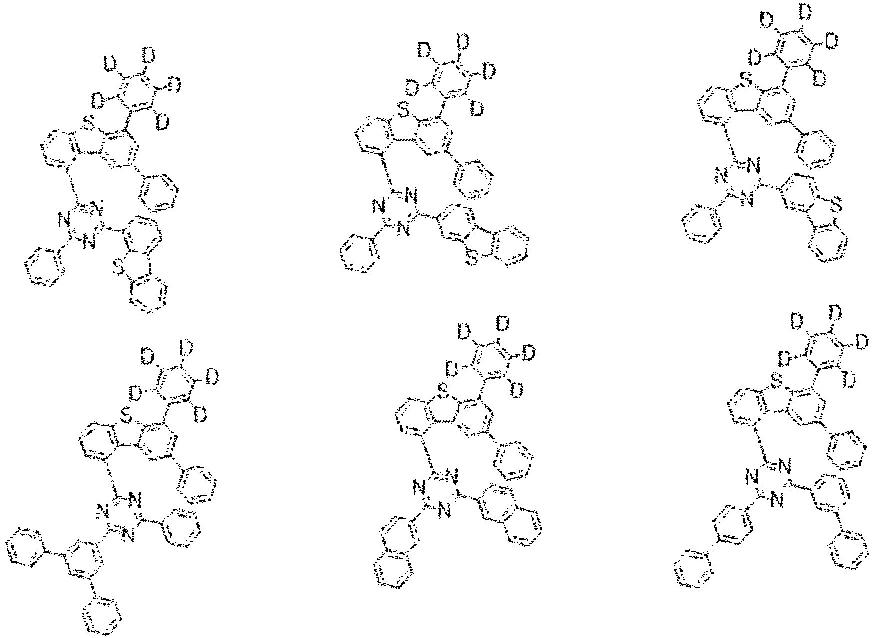

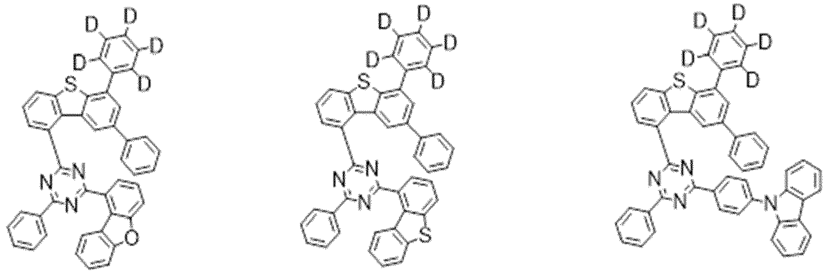

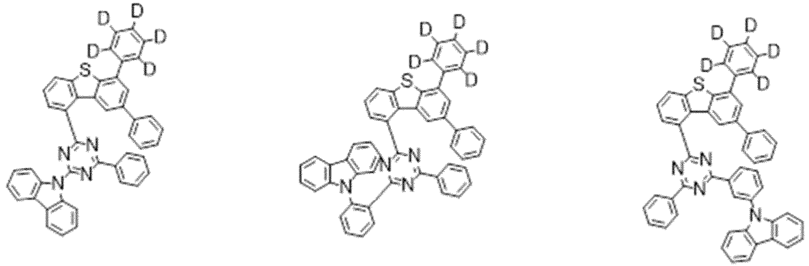

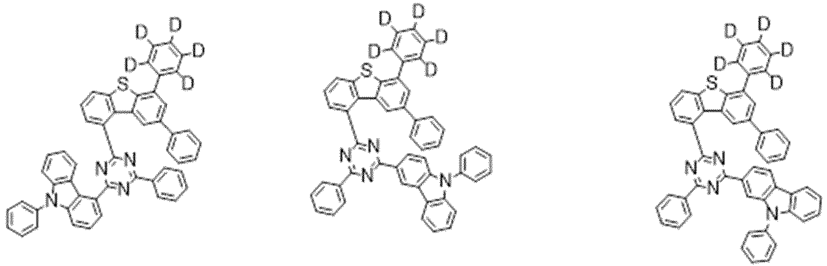

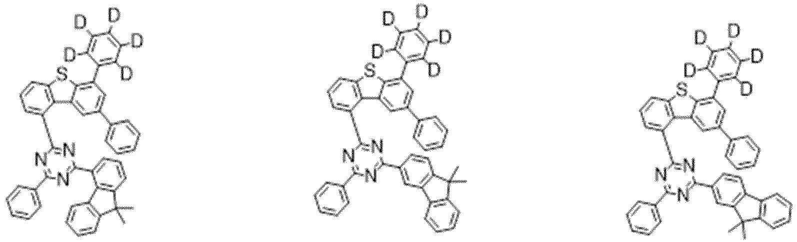

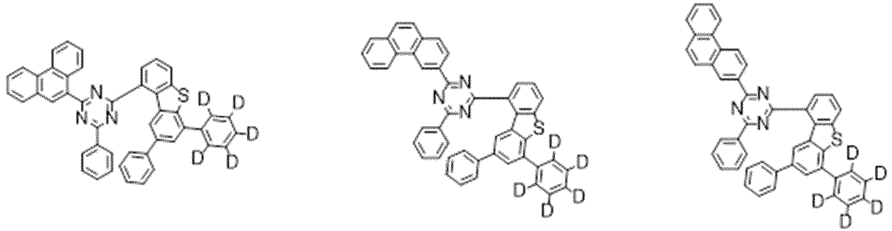

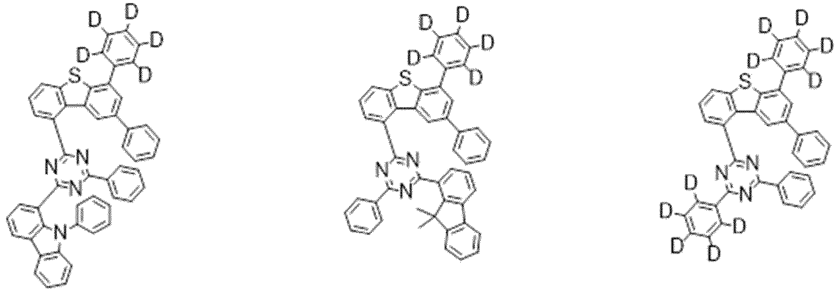

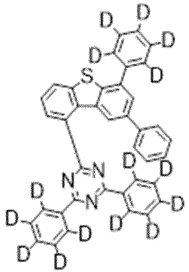

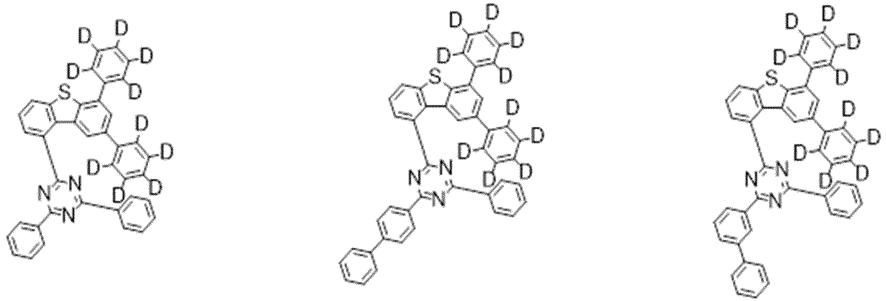

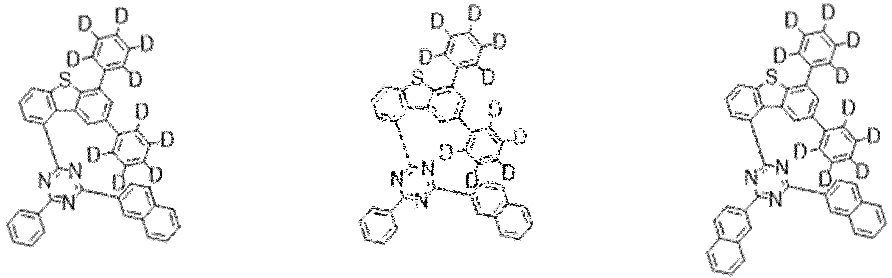

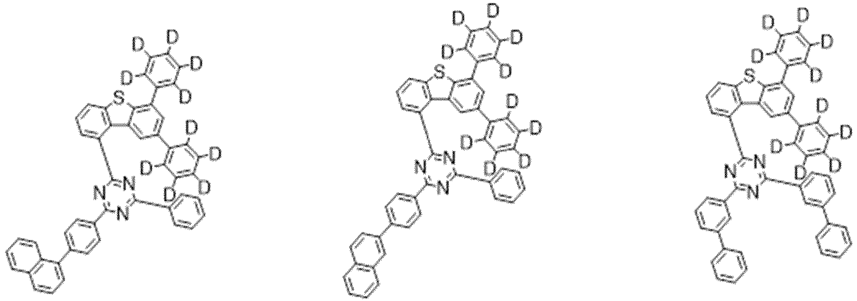

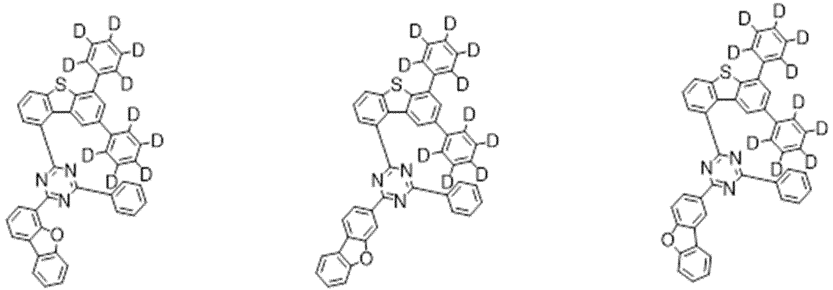

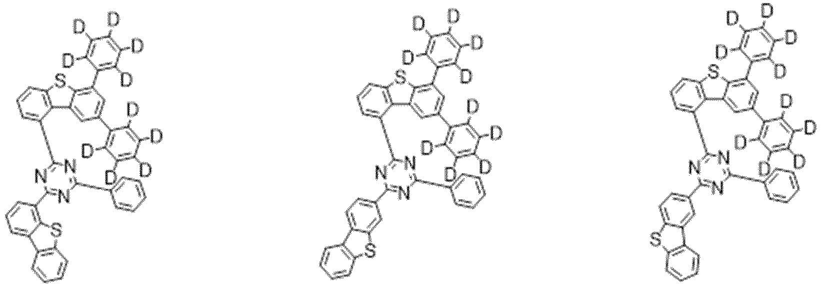

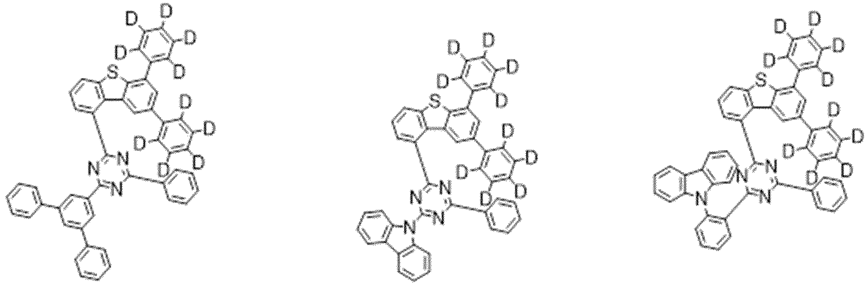

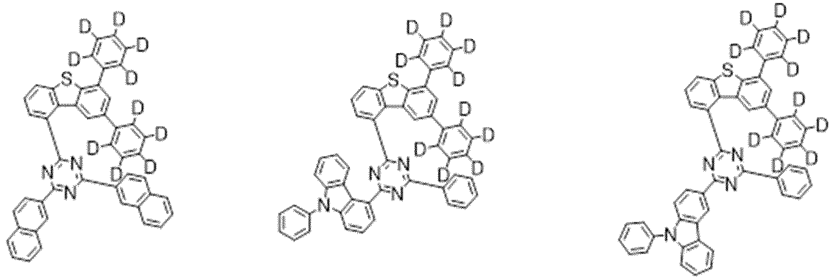

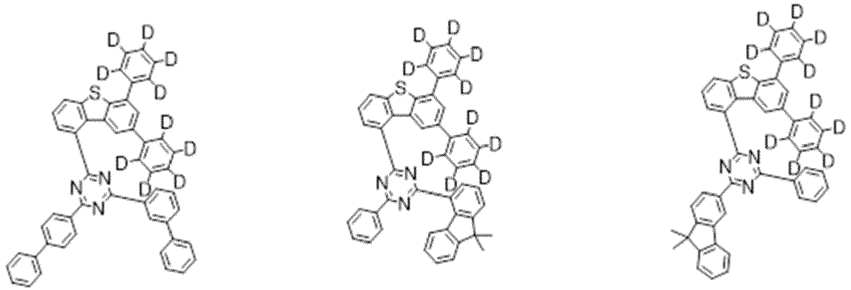

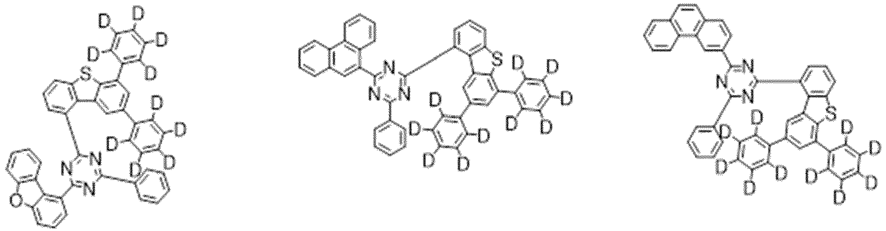

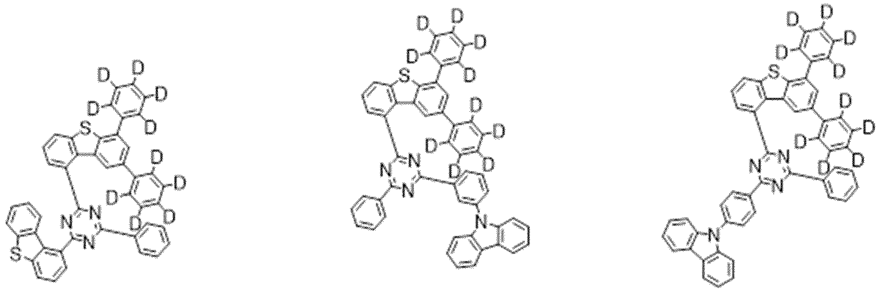

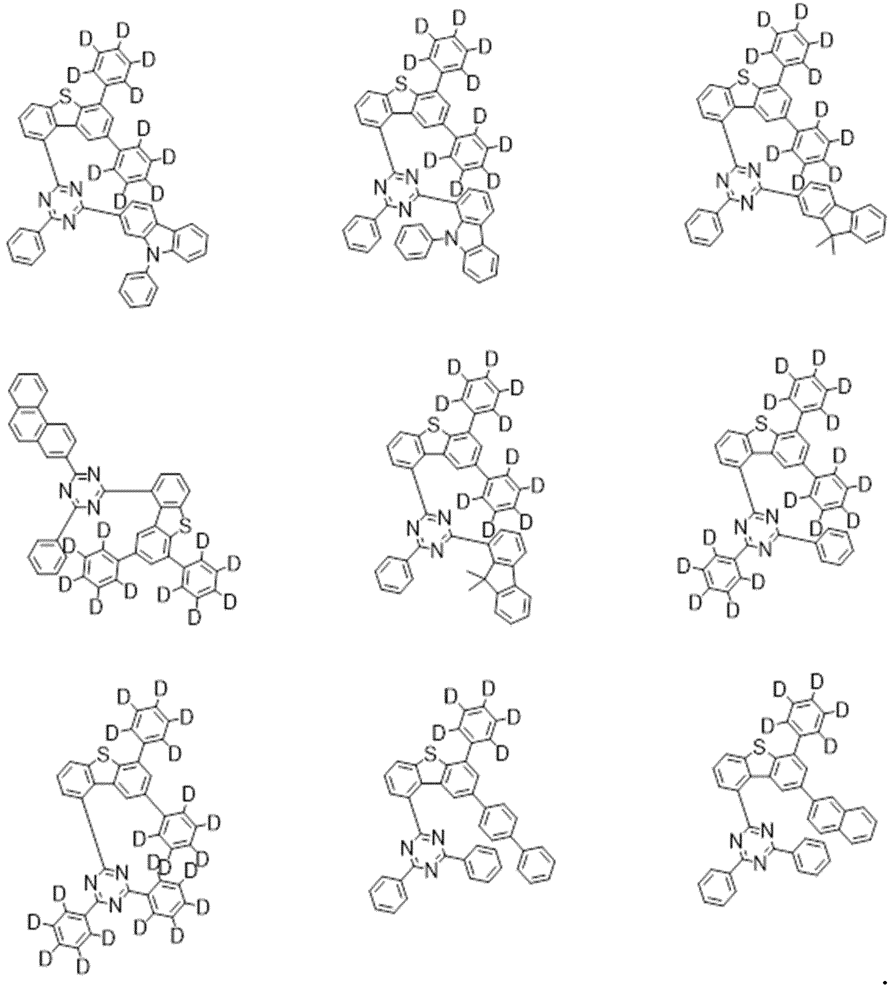

11. Organische lichtemittierende Vorrichtung, umfassend:
eine erste Elektrode; eine zweite Elektrode, die gegenüber der ersten Elektrode angeordnet ist; und eine oder mehrere Schichten aus organischem Material, die zwischen der ersten Elektrode und der zweiten Elektrode angeordnet sind, wobei mindestens eine Schicht von den Schichten aus organischem Material die Verbindung gemäß mindestens einem der Ansprüche 1 bis 10 umfasst.

12. Organische lichtemittierende Vorrichtung gemäß Anspruch 11,
wobei die Schicht aus organischem Material, die die Verbindung umfasst, eine lichtemittierende Schicht ist.

13. Organische lichtemittierende Vorrichtung gemäß Anspruch 12,
wobei die lichtemittierende Schicht ferner eine Verbindung umfasst, dargestellt durch die folgende chemische Formel 2: 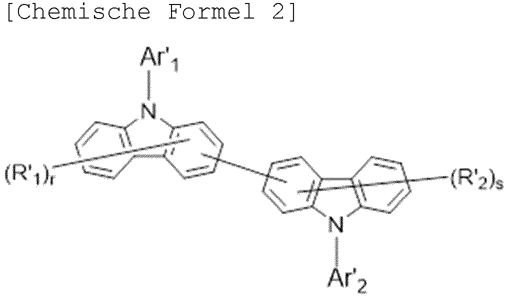
worin in der chemischen Formel 2,
Ar'₁ und Ar'₂ jeweils unabhängig substituiertes oder unsubstituiertes C₆₋₆₀-Aryl oder substituiertes oder unsubstituiertes C₂₋₆₀-Heteroaryl, das mindestens ein Heteroatom enthält, das ausgewählt ist aus der Gruppe bestehend aus N, O und S, sind, und
R'₁ und R'₂ jeweils unabhängig Wasserstoff, Deuterium, C₁₋₆₀-Alkyl, C₆₋₆₀-Aryl oder C₂₋₆₀-Heteroaryl, das mindestens ein Heteroatom enthält, das ausgewählt ist aus der Gruppe bestehend aus N, O und S, sind; und
r und s jeweils unabhängig eine ganze Zahl von 0 bis 7 sind.

14. Organische lichtemittierende Vorrichtung gemäß Anspruch 13,
wobei die durch die chemische Formel 2 dargestellte Verbindung eine beliebige ist, die ausgewählt ist aus der Gruppe bestehend aus den folgenden Verbindungen:

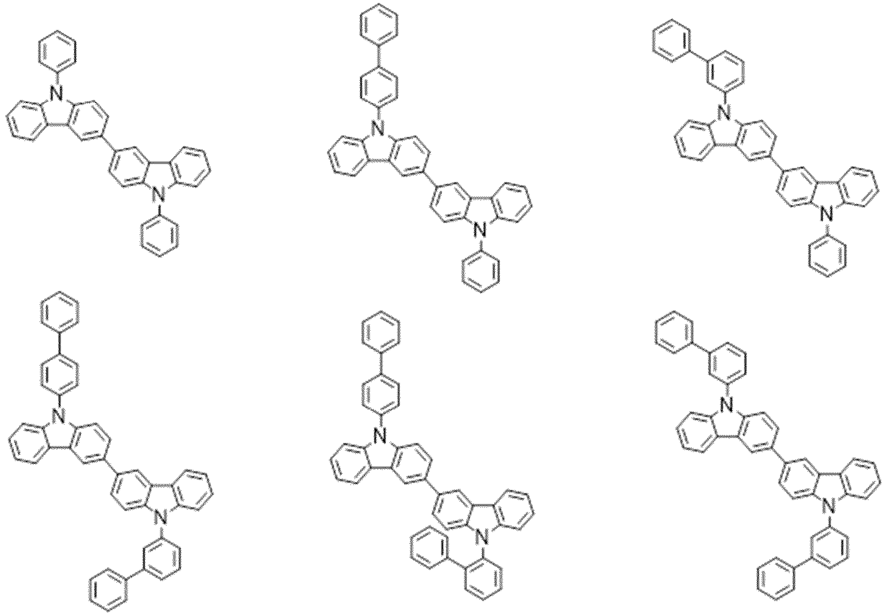

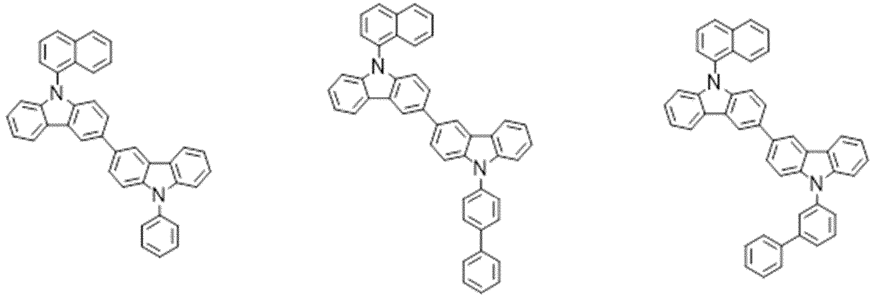

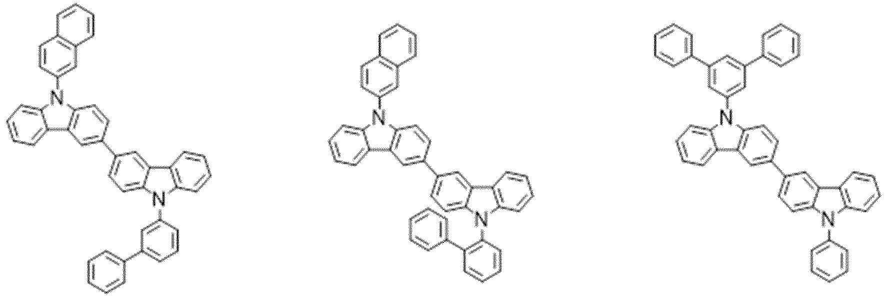

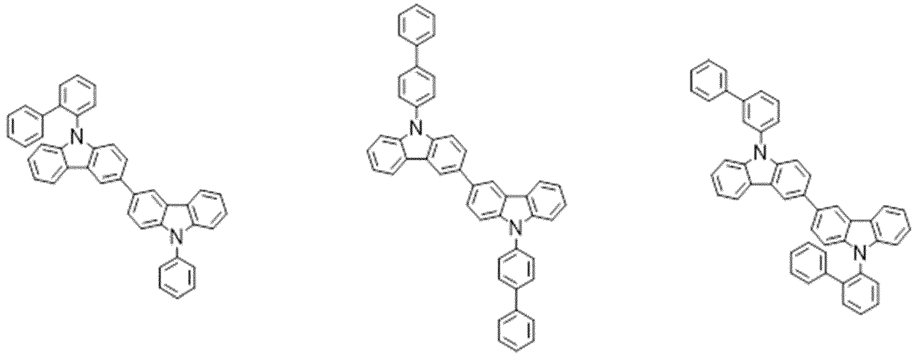

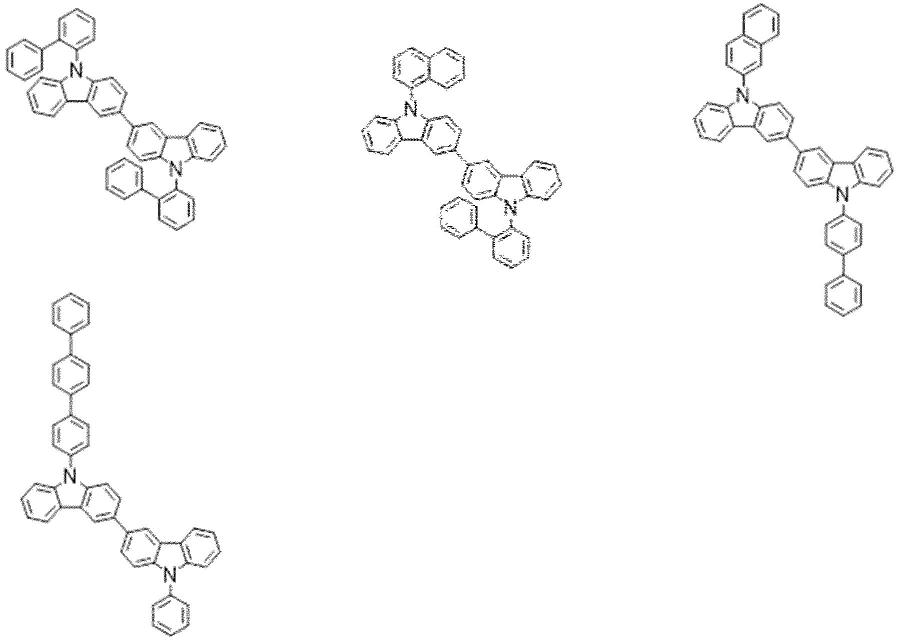

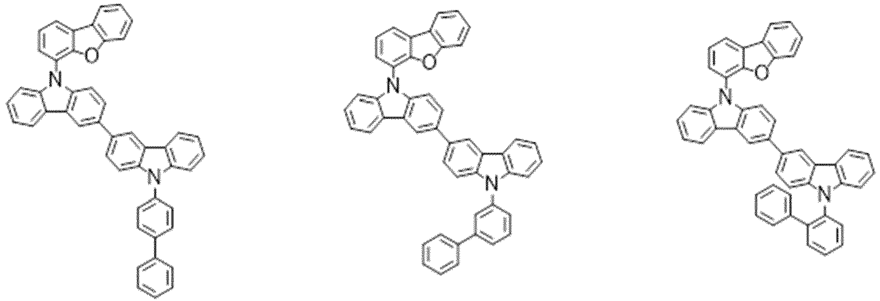

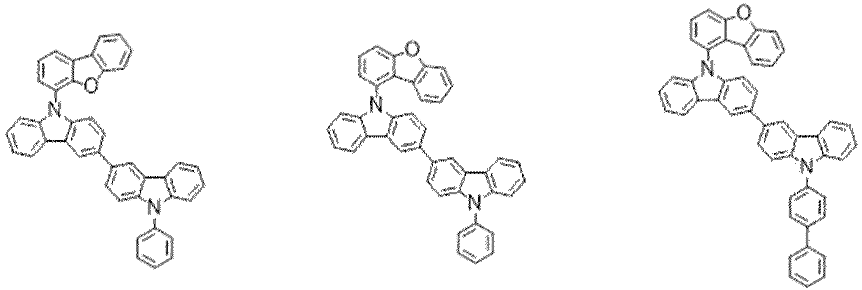

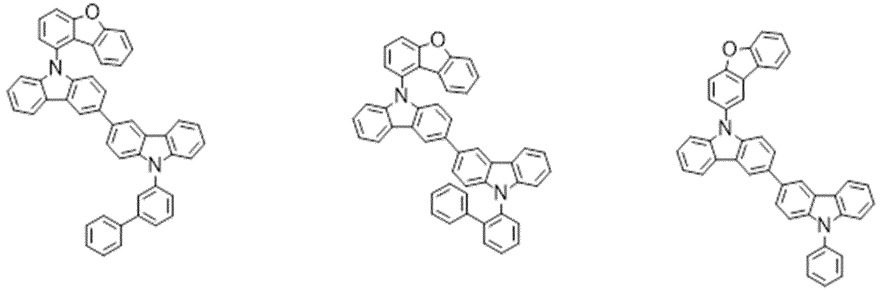

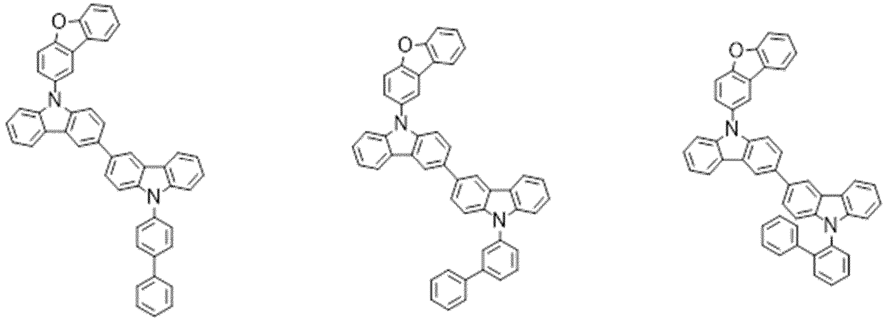

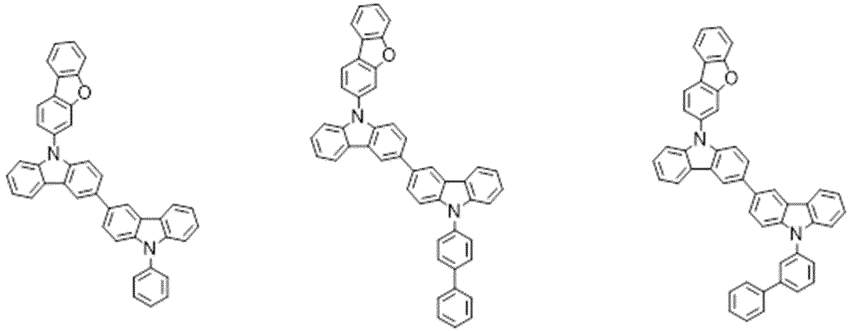

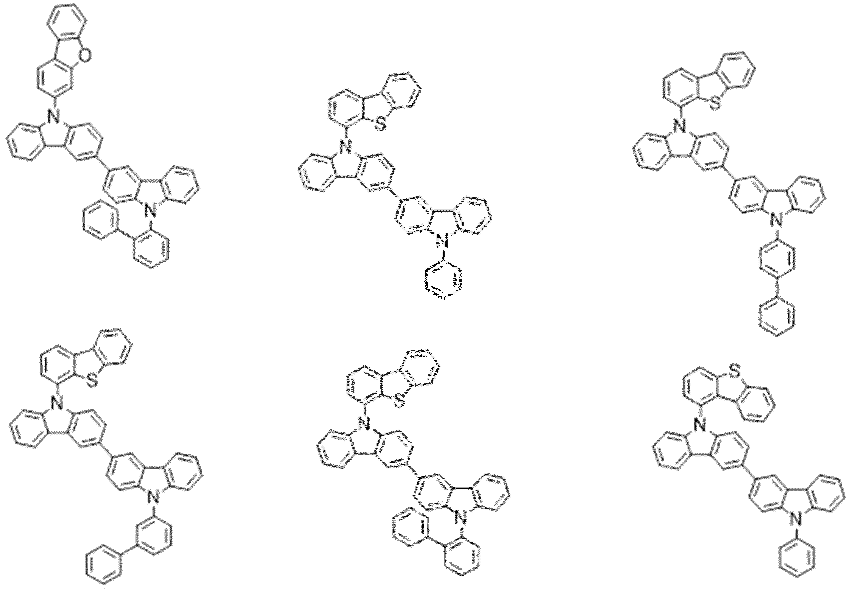

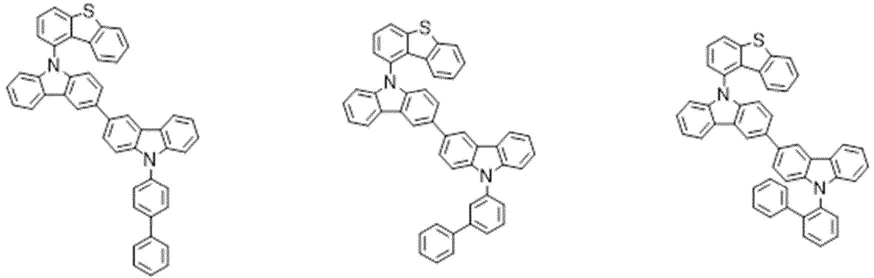

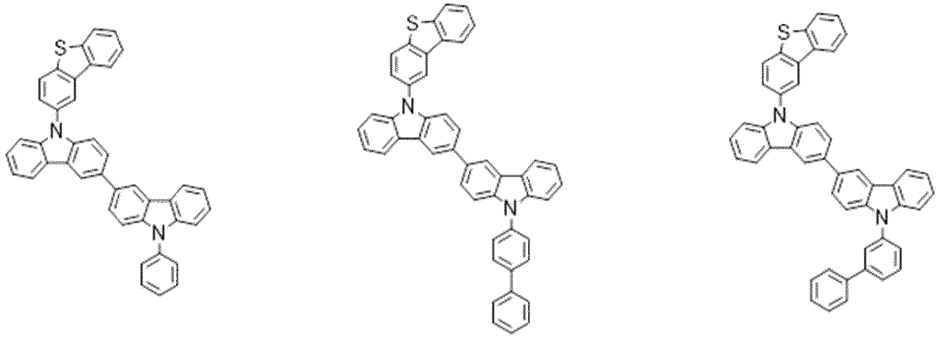

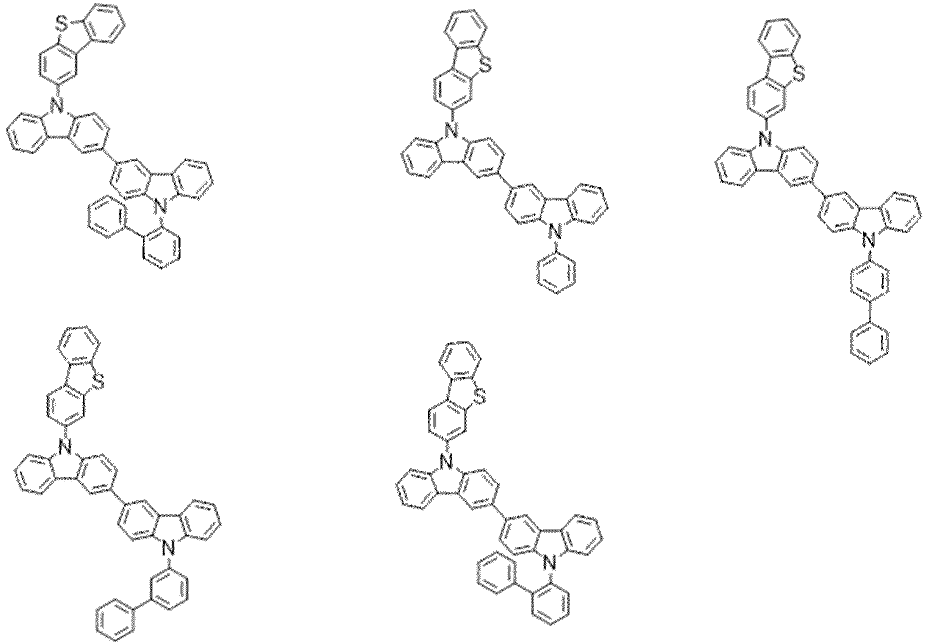

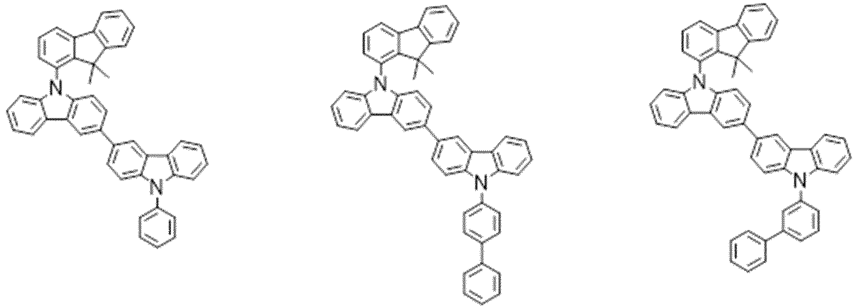

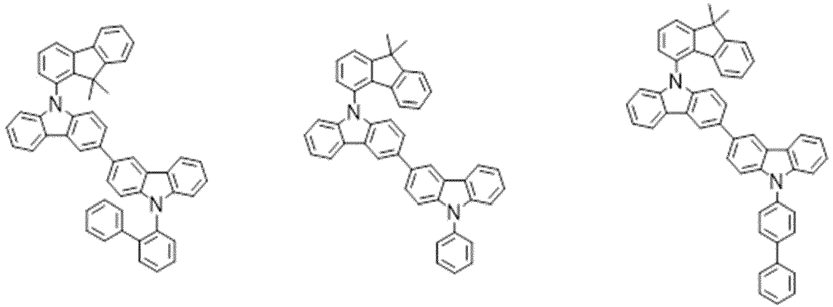

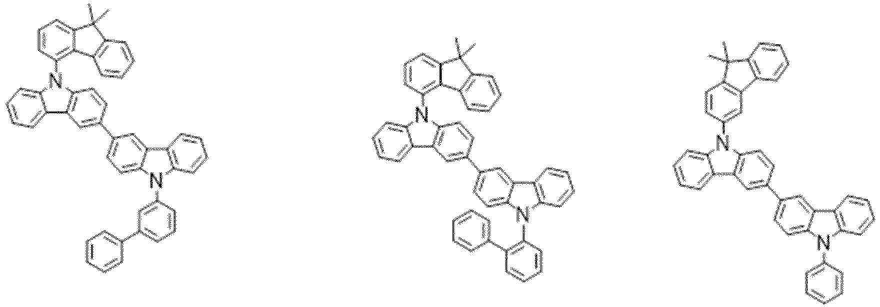

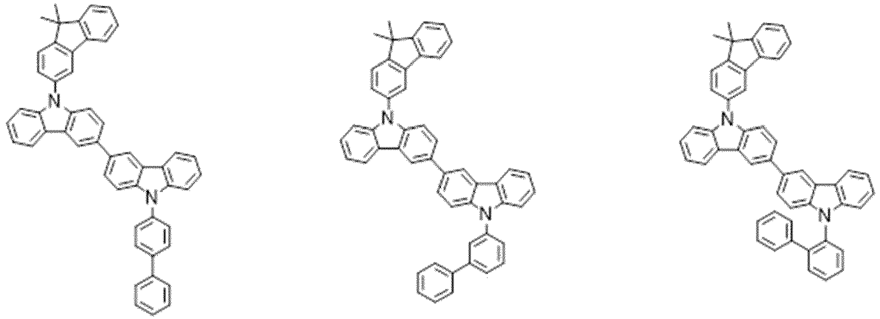

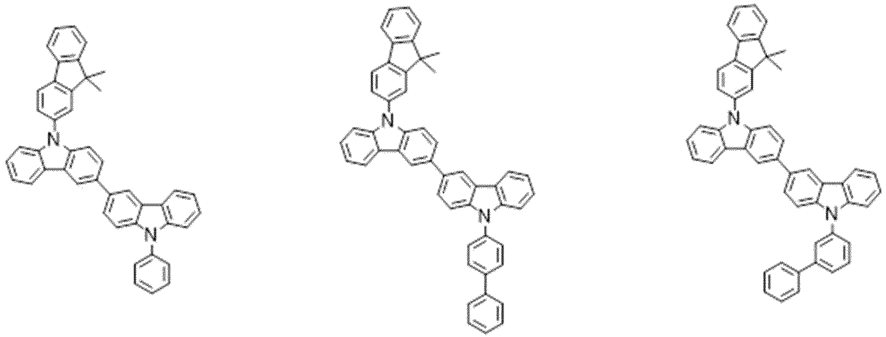

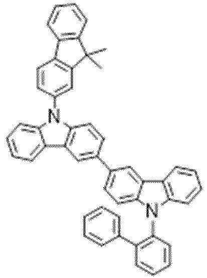

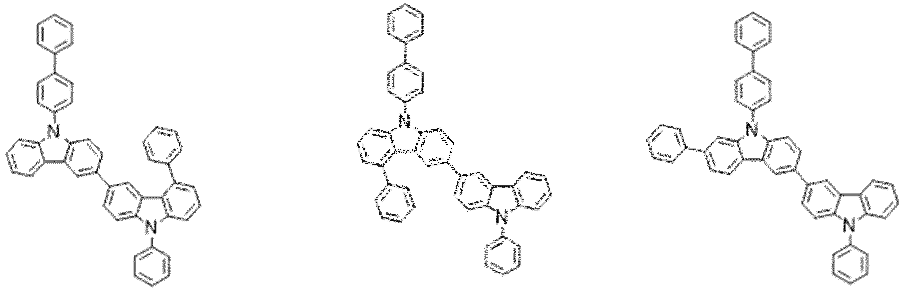

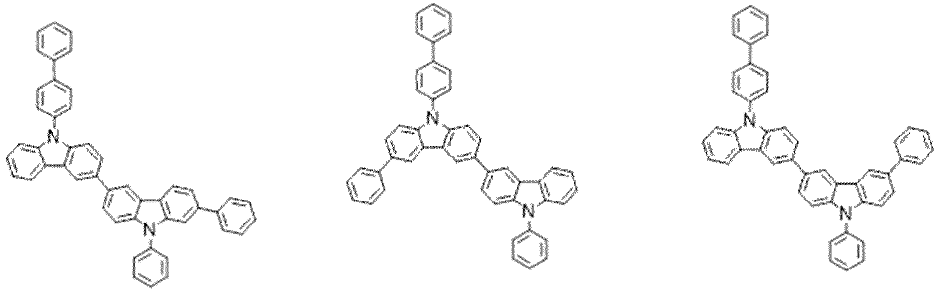

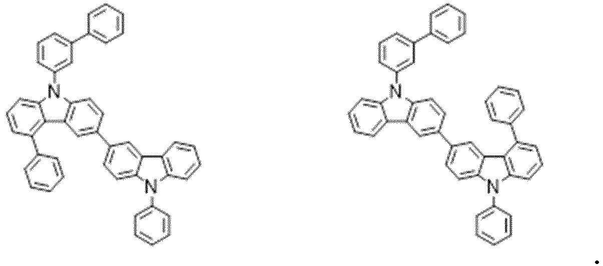

## Revendications

1. Composé représenté par la formule chimique 1 suivante : 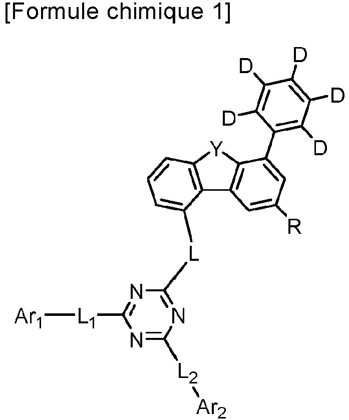 dans la formule chimique 1,
Y est O ou S,
D est un deutérium,
L, L₁ et L₂ sont chacun indépendamment une liaison simple, ou un arylène en C₆₋₂₀ non substitué ou substitué avec du deutérium,
Ar₁ et Ar₂ sont chacun indépendamment un aryle en C₆₋₂₀ non substitué ou substitué avec au moins un substituant sélectionné dans le groupe consistant en un deutérium, un méthyle et un phényle ; un dibenzofuranyle non substitué ; un dibenzothiophényle non substitué ; ou un hétéroaryle en C₂₋₂₀ contenant N non substitué ou substitué avec au moins un substituant sélectionné dans le groupe consistant en un deutérium, un méthyle et un phényle, et
R est un hydrogène ; ou un aryle en C₆₋₆₀ non substitué ou substitué avec du deutérium.

2. Composé selon la revendication 1,
dans lequel L est une liaison simple.

3. Composé selon la revendication 1,
dans lequel L₁ et L₂ sont chacun indépendamment une liaison simple, ou un phénylène.

4. Composé selon la revendication 1,
dans lequel Ar₁ et Ar₂ sont chacun indépendamment un phényle, un biphénylyle, un terphénylyle, un naphtyle, un phénanthryle, un fluorényle, un dibenzofuranyle, un dibenzothiophényle, ou un carbazolyle, et
les phényle, biphénylyle, terphénylyle, naphtyle, phénanthryle, fluorényle et carbazolyle sont chacun indépendamment non substitués ou substitués avec au moins un substituant sélectionné dans le groupe consistant en un deutérium, un méthyle et un phényle.

5. Composé selon la revendication 4,
dans lequel Ar₁ et Ar₂ sont chacun indépendamment l'un quelconque sélectionné dans le groupe consistant en les suivants :

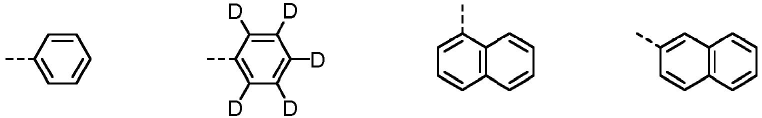

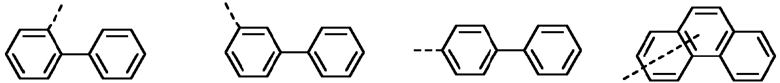

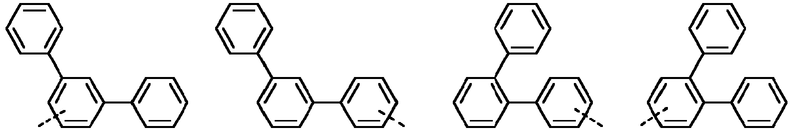

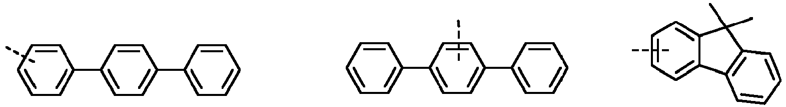

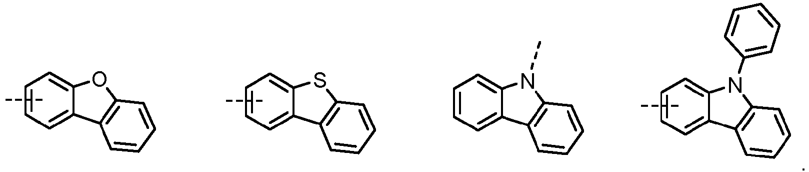

6. Composé selon la revendication 1,
dans lequel Ar₁ et Ar₂ sont chacun indépendamment un aryle en C₆₋₁₂ non substitué ou substitué avec du deutérium.

7. Composé selon la revendication 1,
dans laquelle R est un hydrogène ; un phényle non substitué ou substitué avec du deutérium ; un biphénylyle non substitué ou substitué avec du deutérium ; ou un naphtyle non substitué ou substitué avec du deutérium.

8. Composé selon la revendication 7,
dans lequel R est un hydrogène, 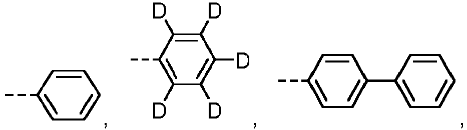 ou

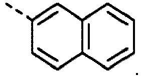

9. Composé selon la revendication 1,
dans lequel le composé est représenté par l'une quelconque des formules chimiques 1-1 à 1-5 suivantes : 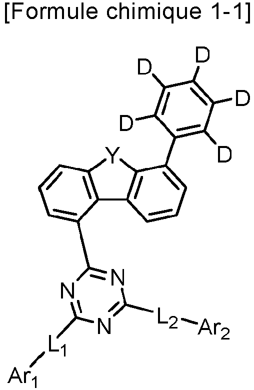 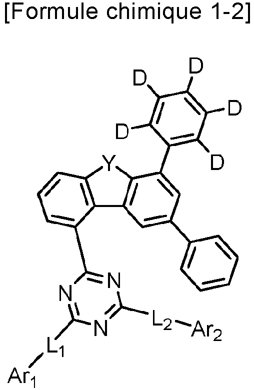 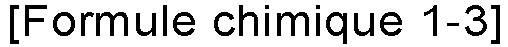 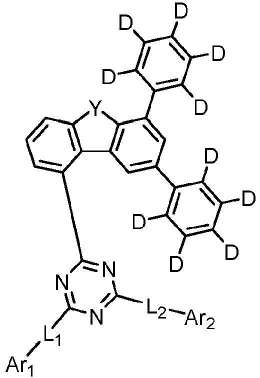 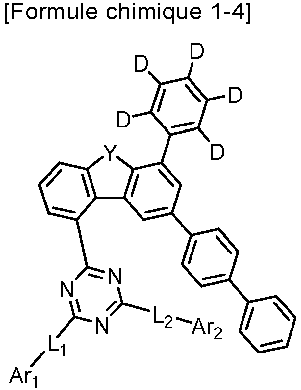 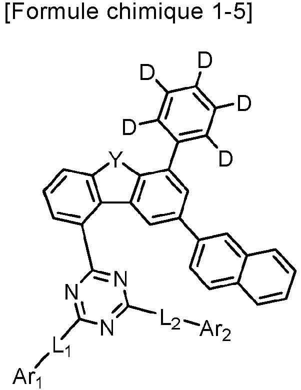
dans les formules chimiques 1-1 à 1-5,
Y, L₁, L₂, Ar₁ et Ar₂ sont tels que définis dans la revendication 1.

10. Composé selon la revendication 1,
dans lequel le composé est l'un quelconque sélectionné dans le groupe consistant en les composés suivants :

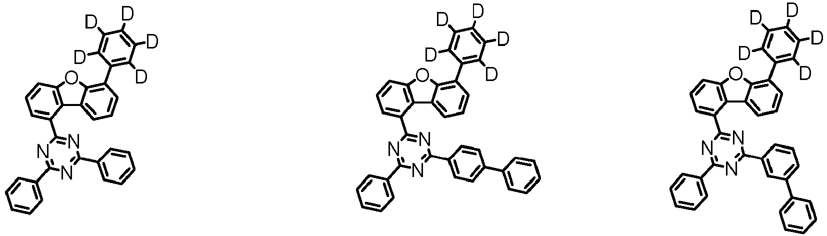

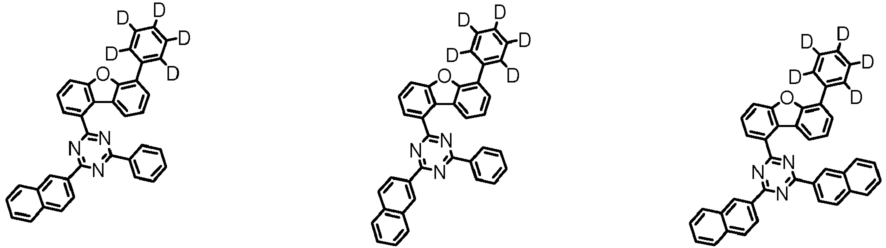

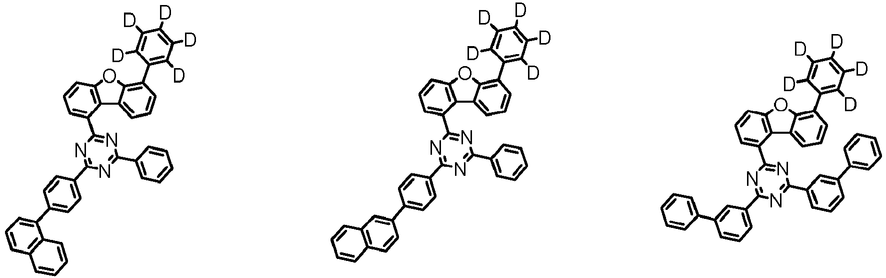

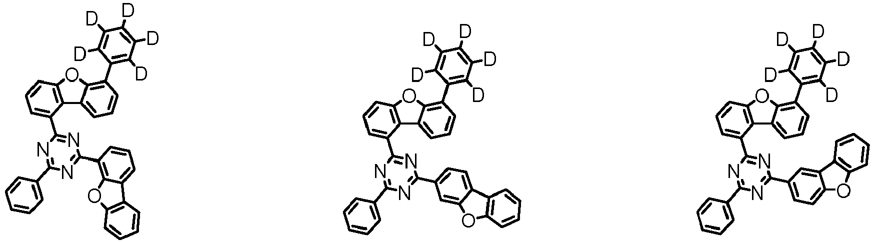

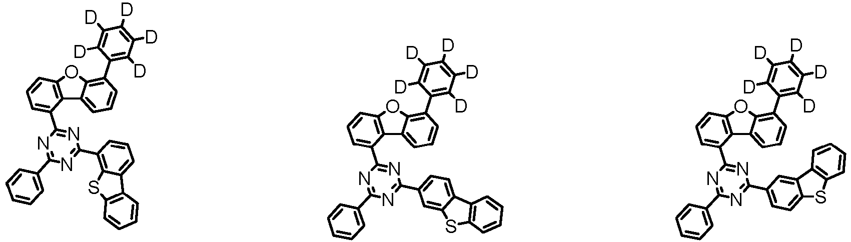

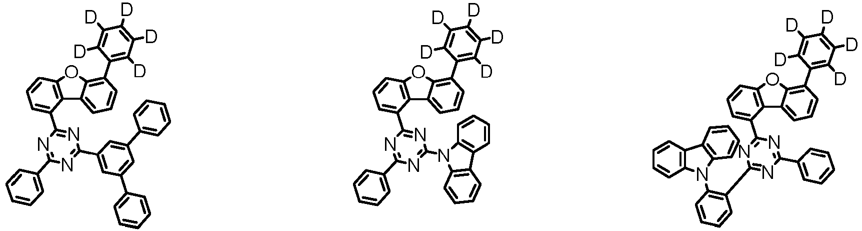

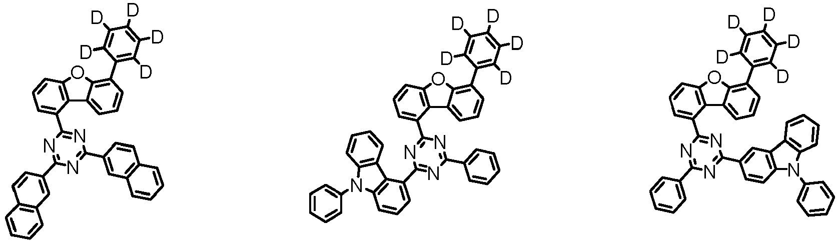

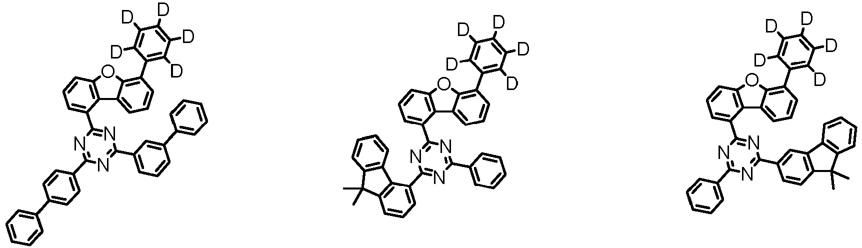

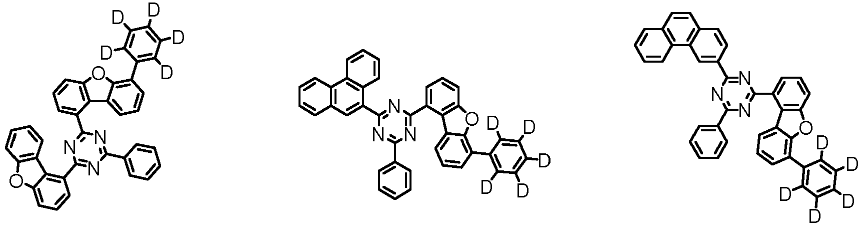

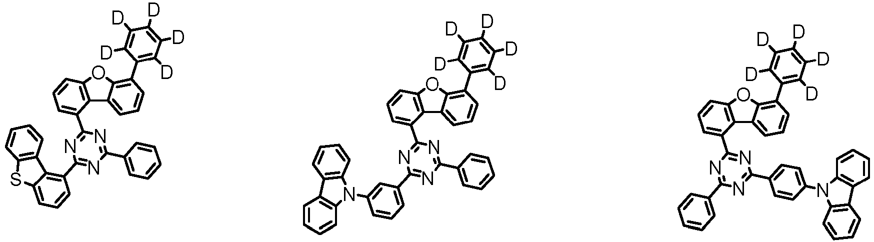

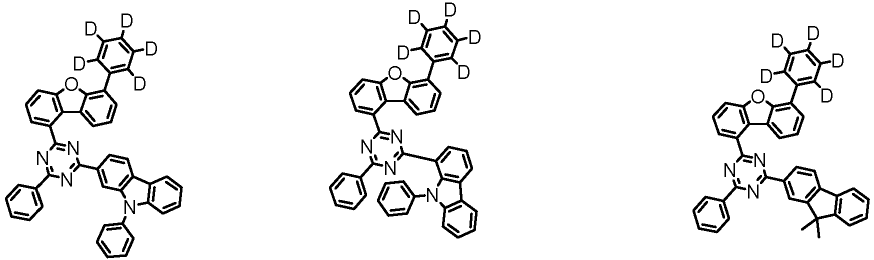

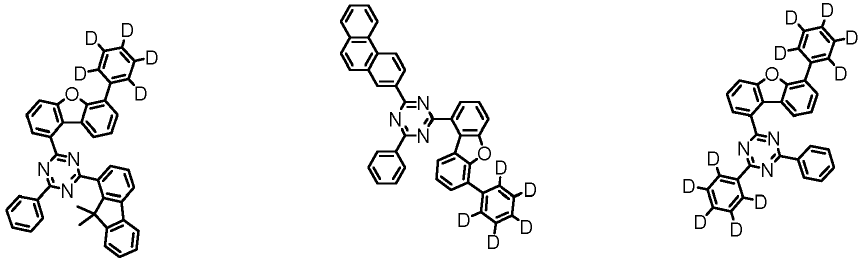

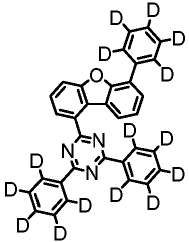

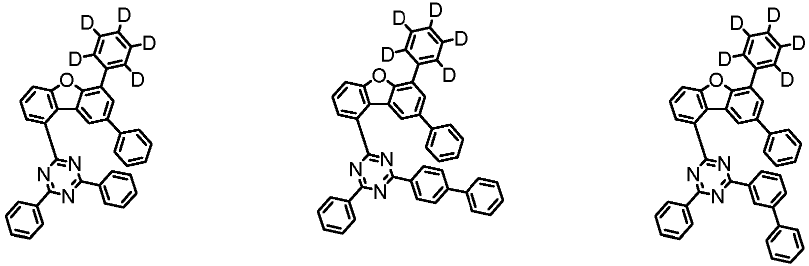

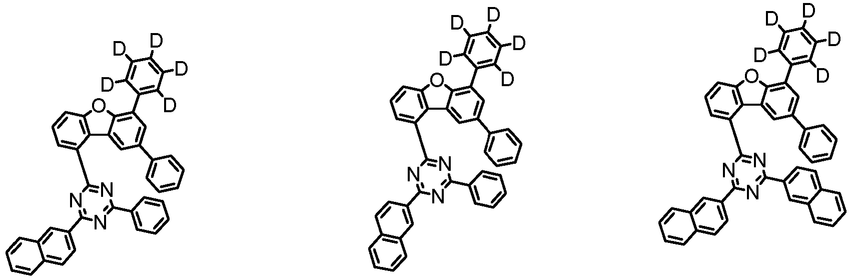

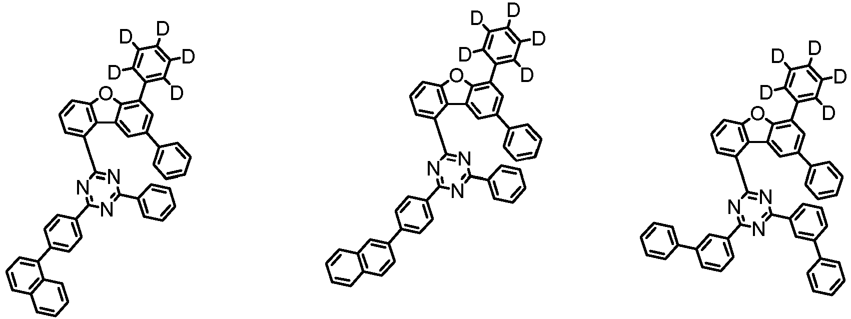

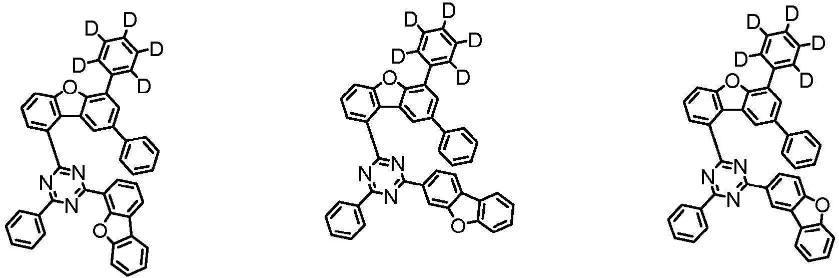

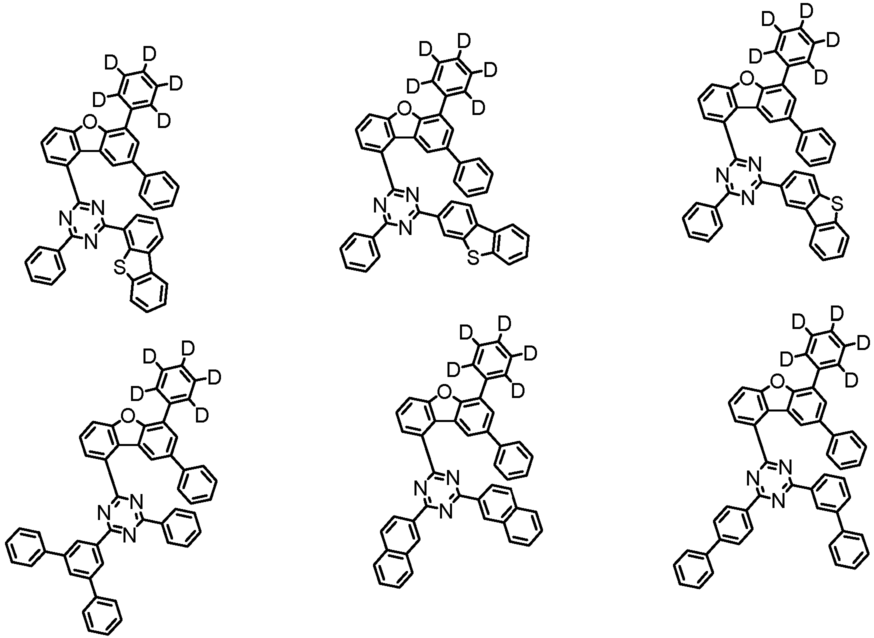

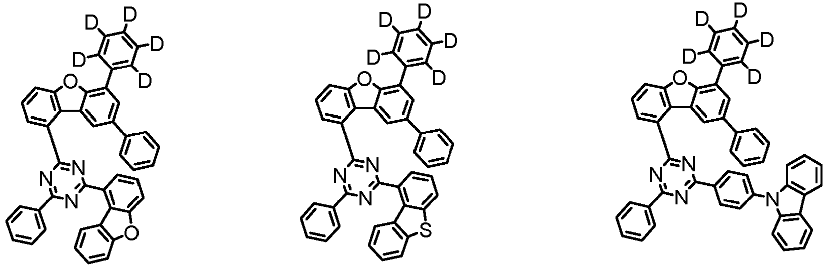

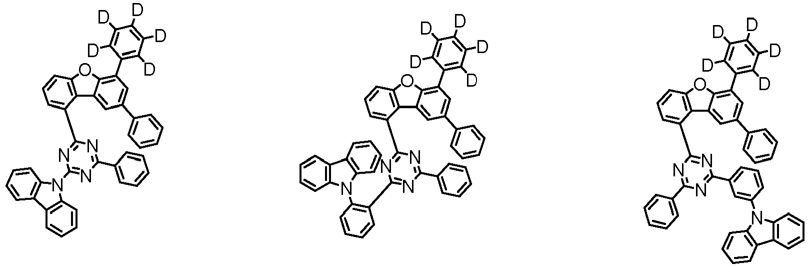

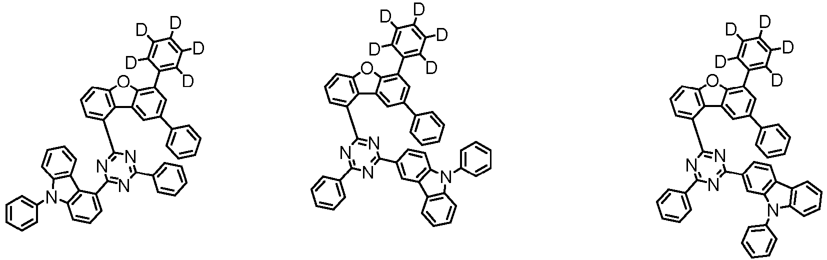

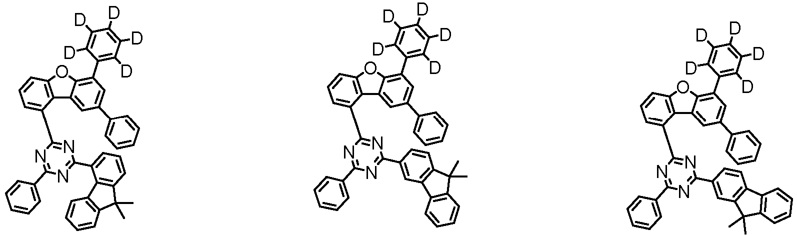

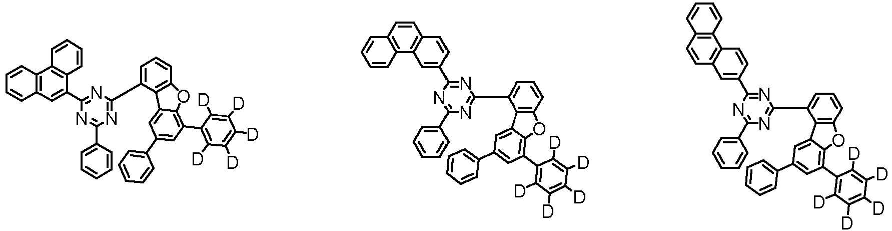

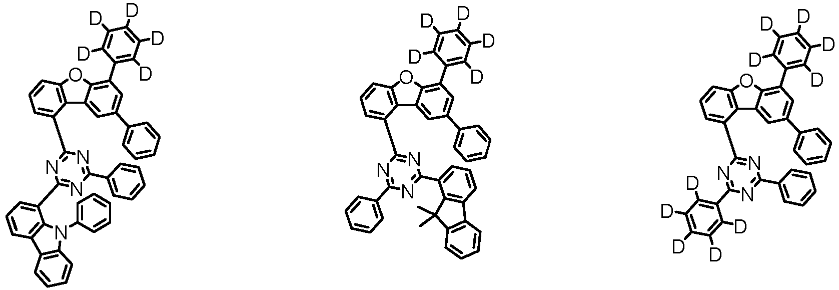

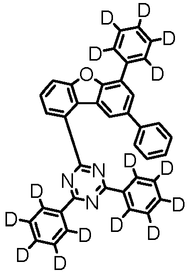

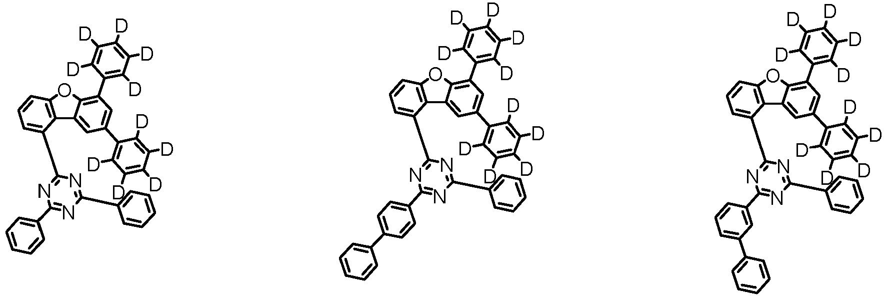

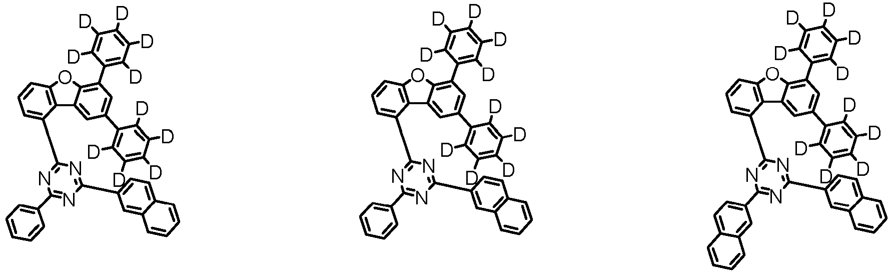

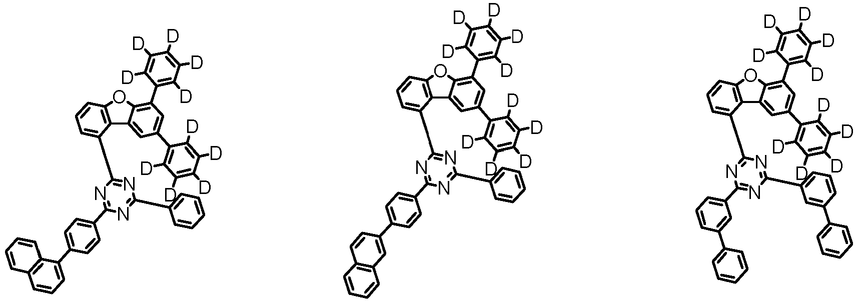

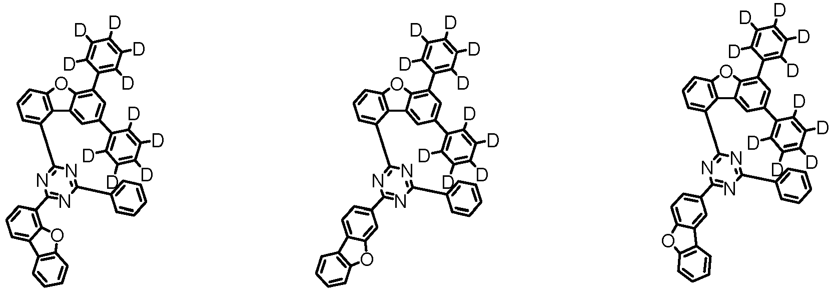

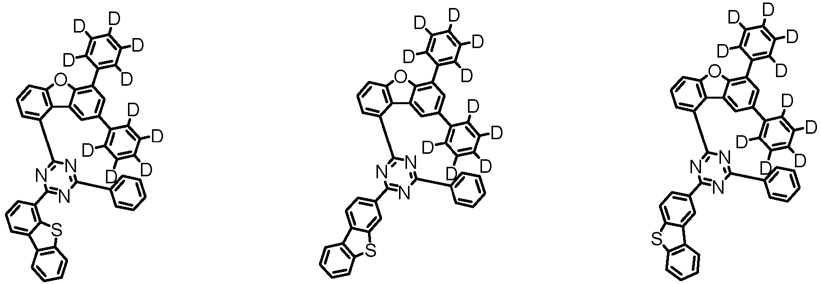

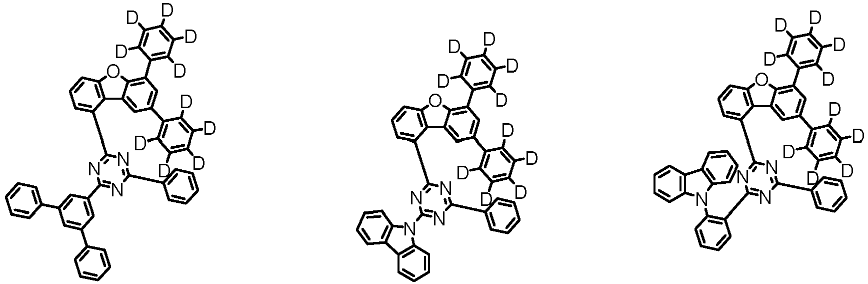

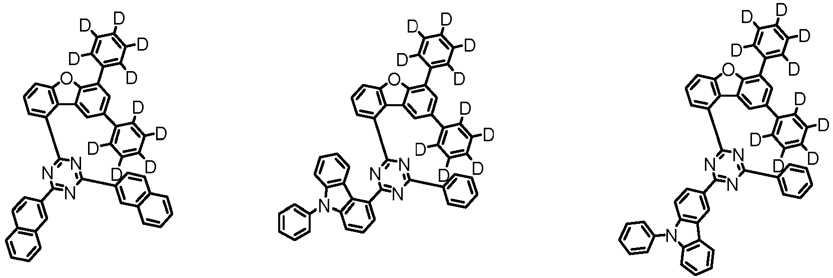

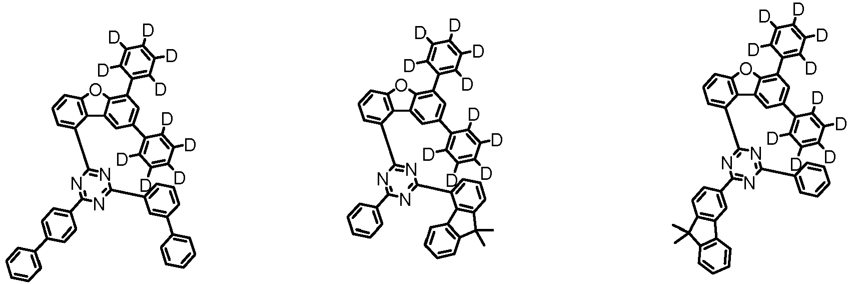

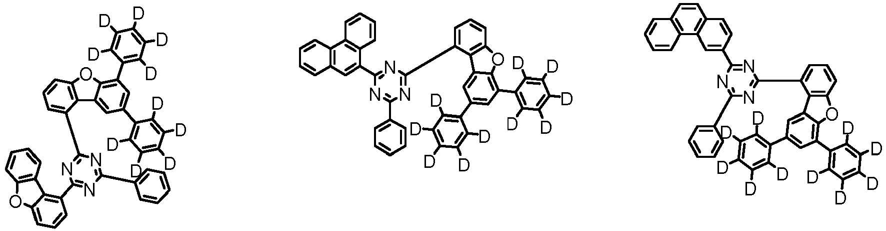

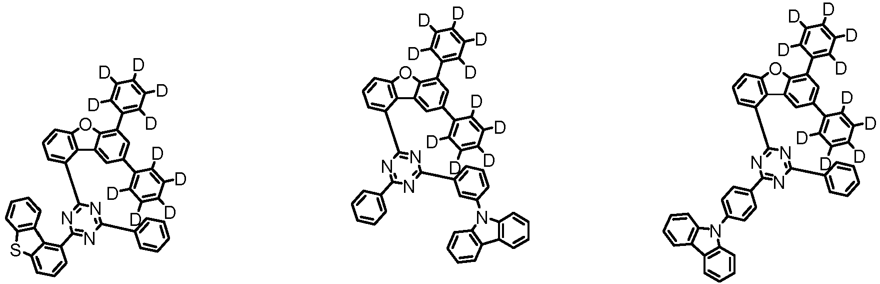

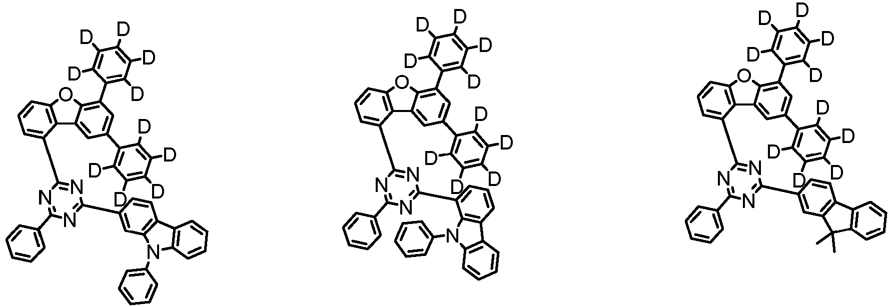

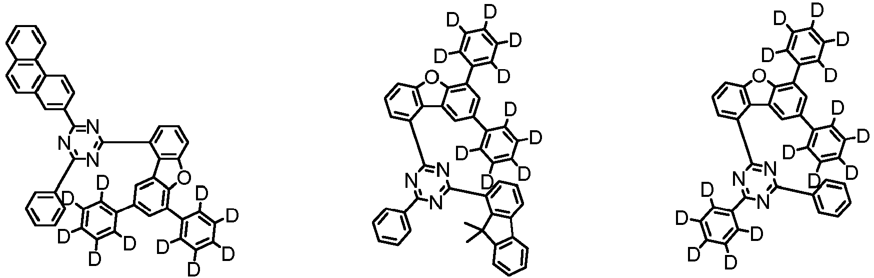

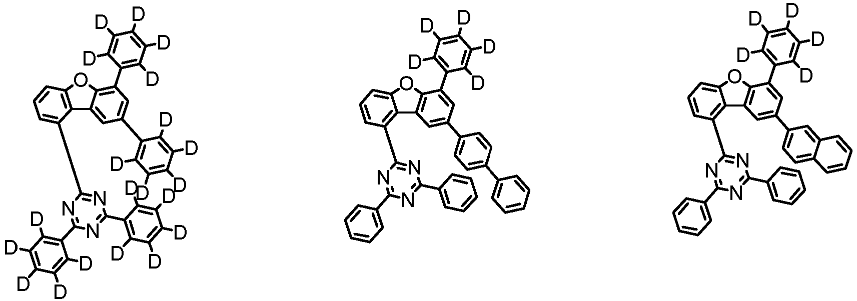

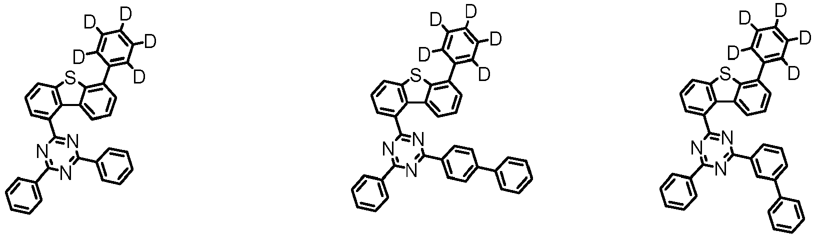

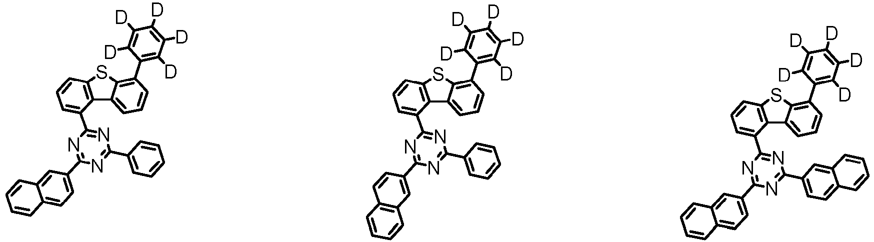

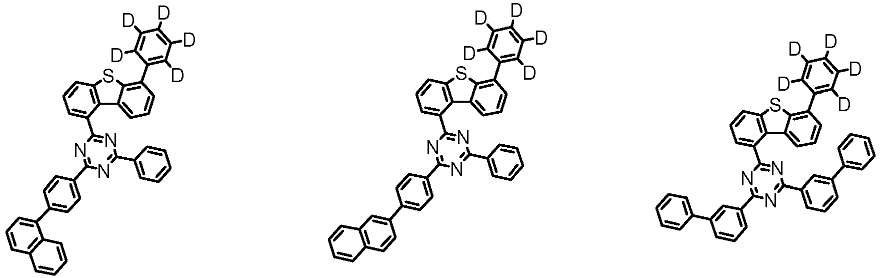

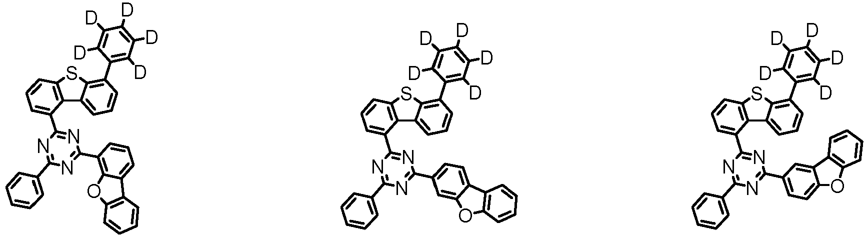

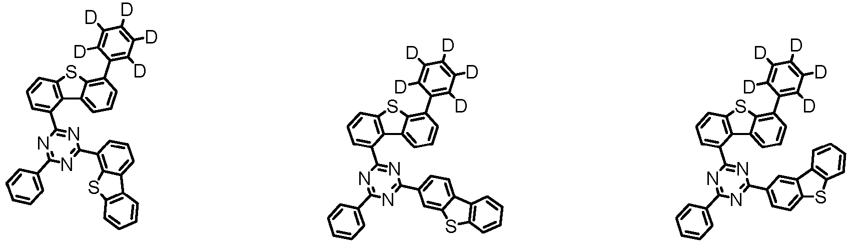

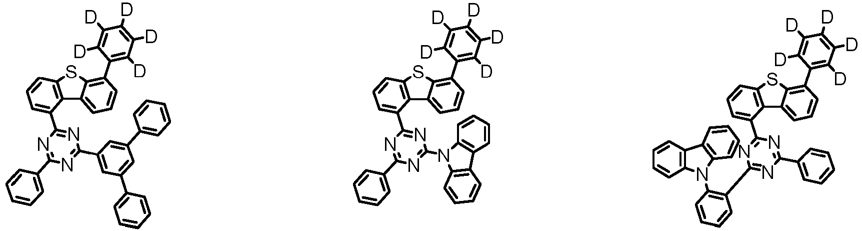

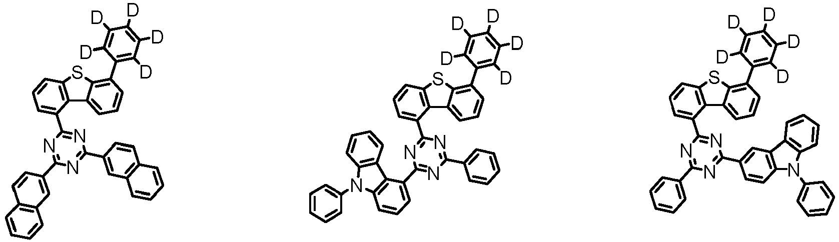

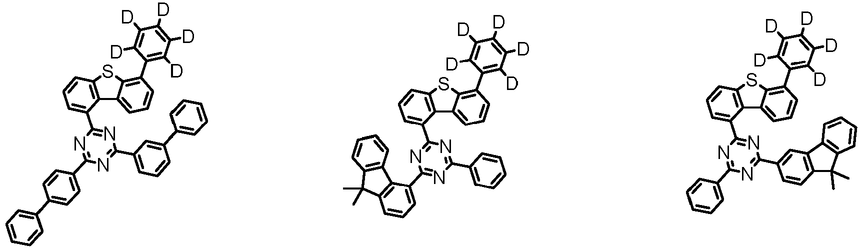

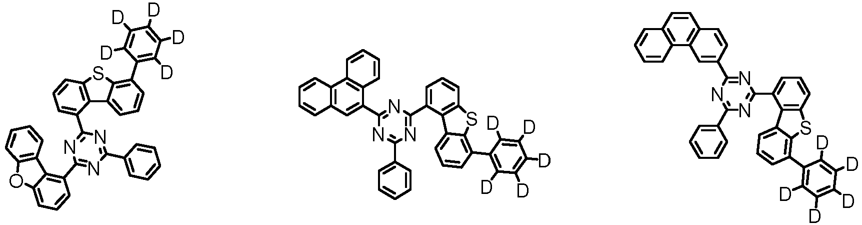

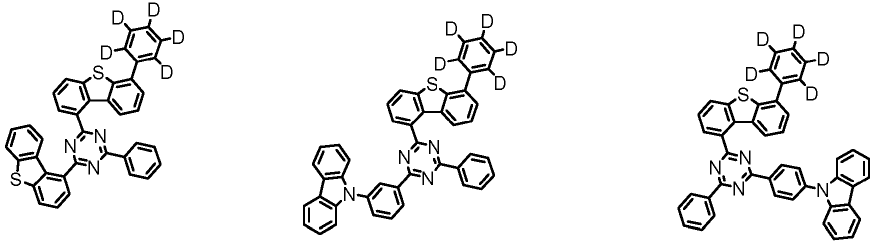

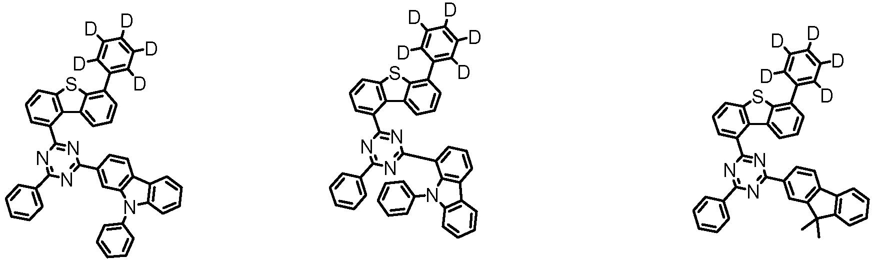

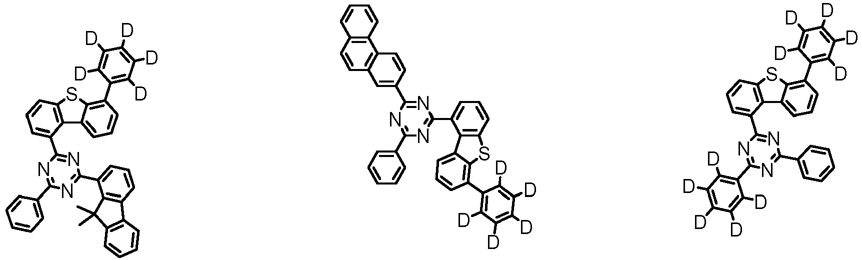

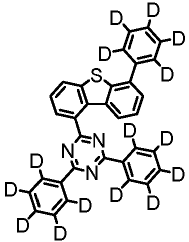

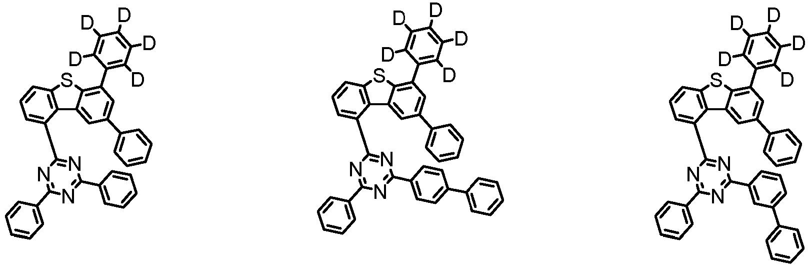

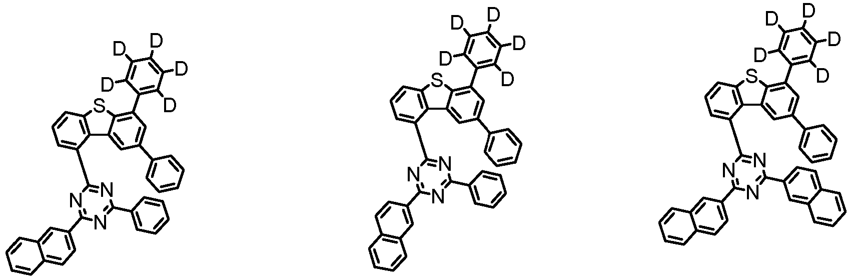

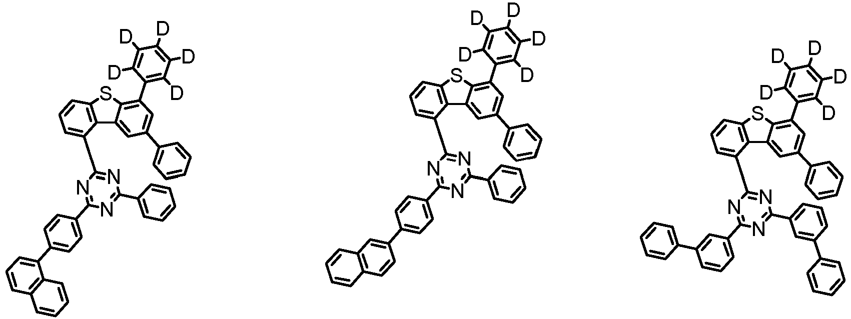

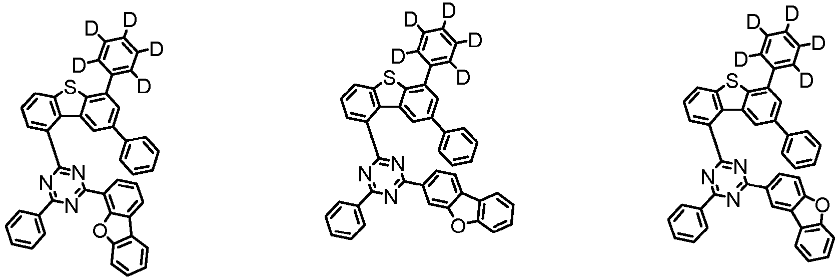

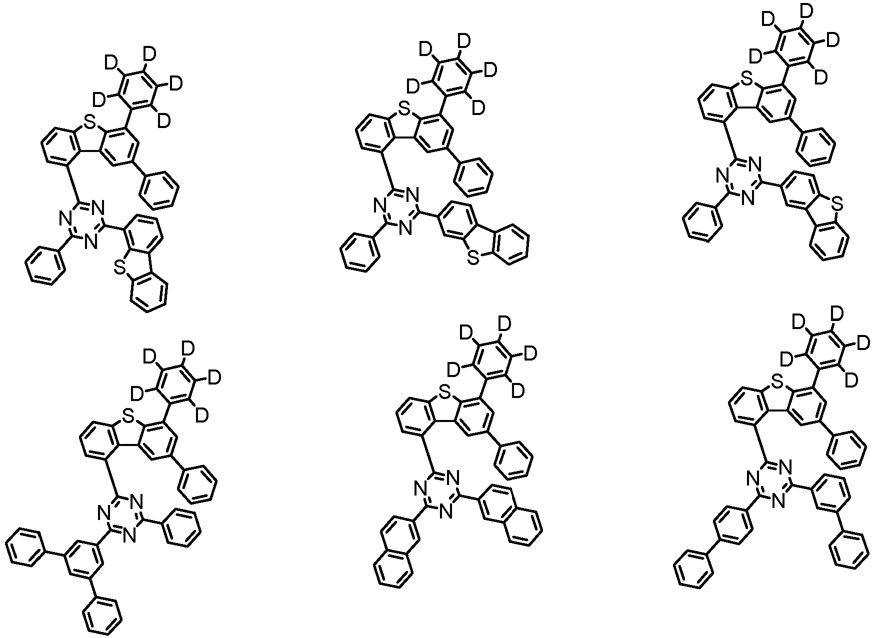

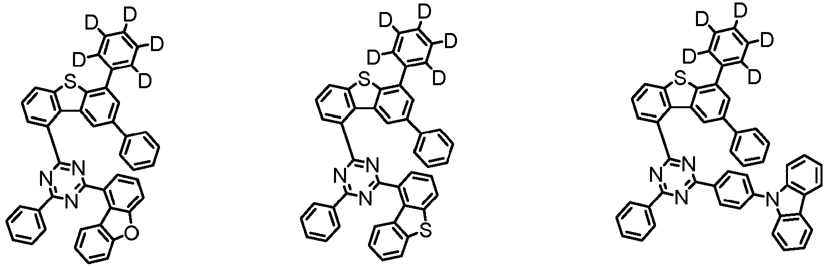

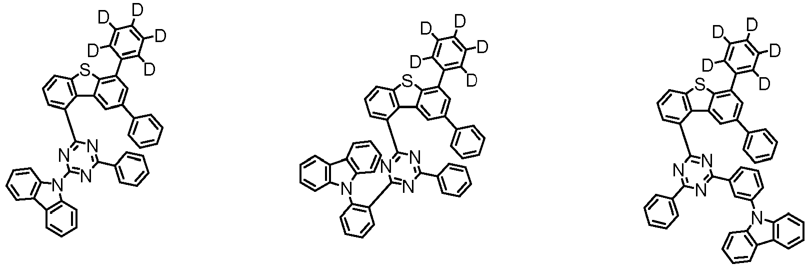

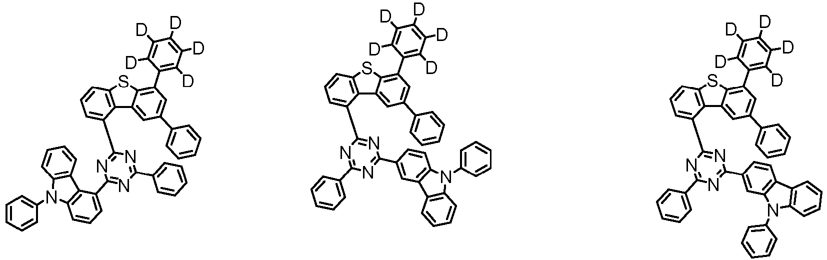

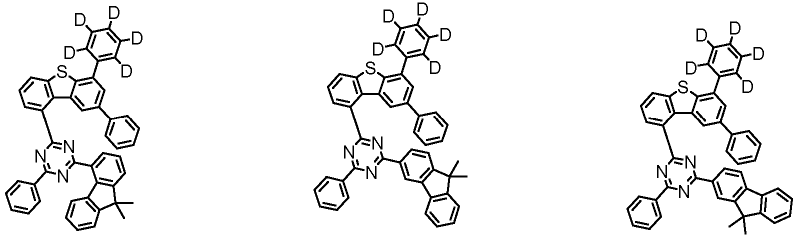

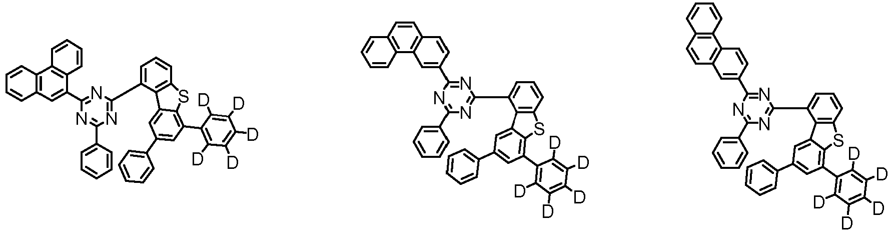

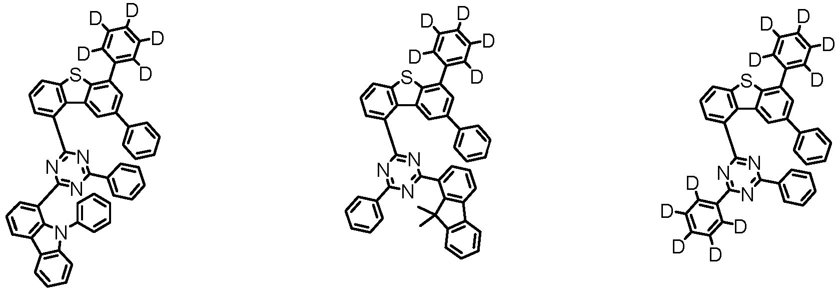

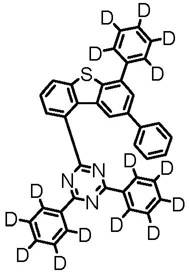

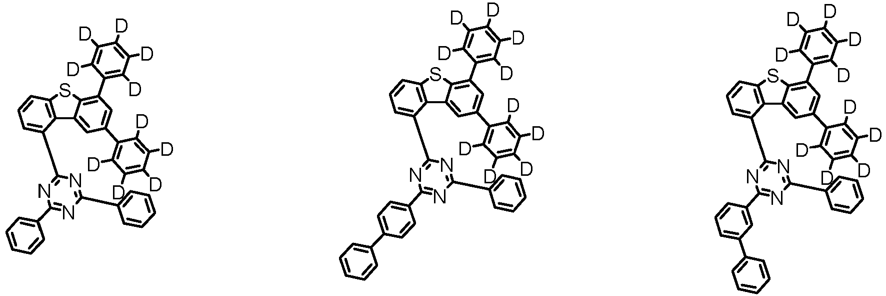

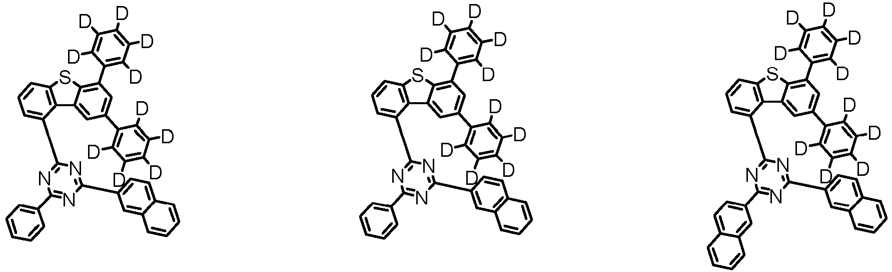

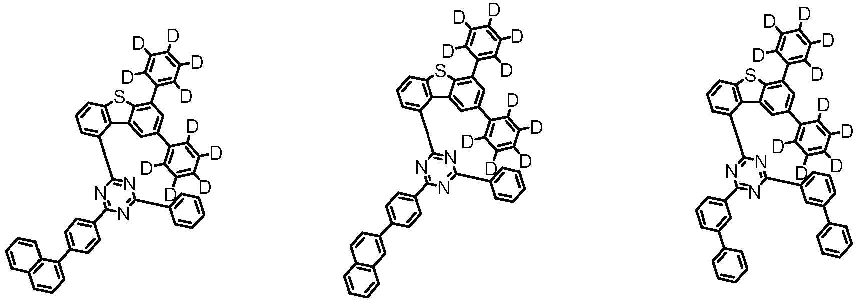

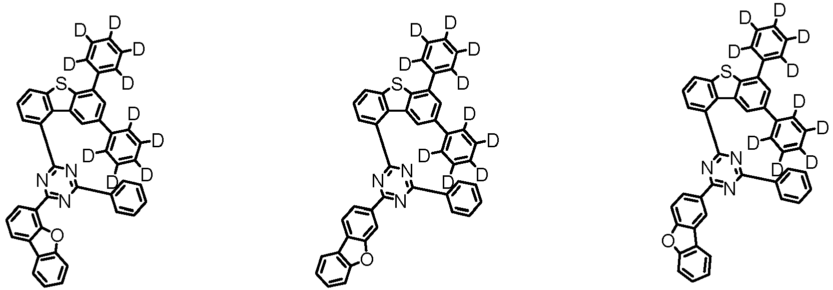

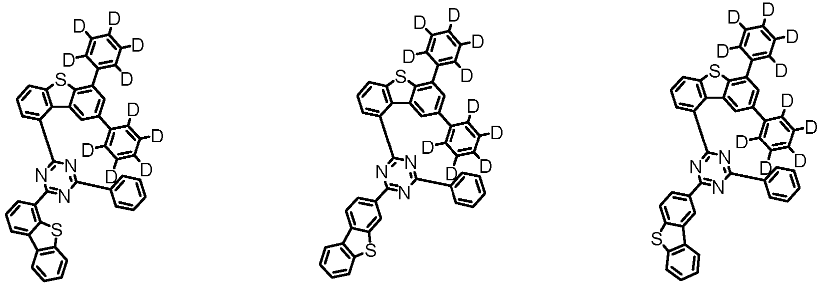

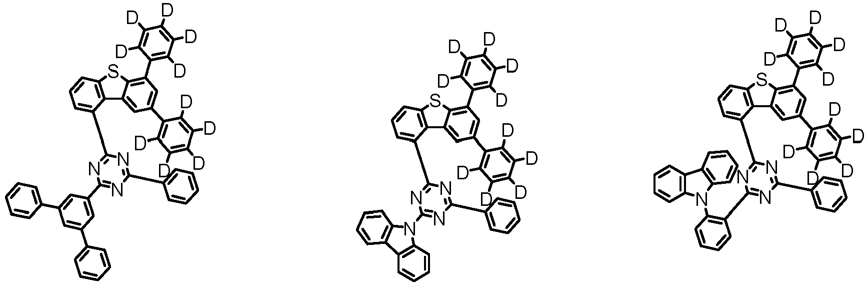

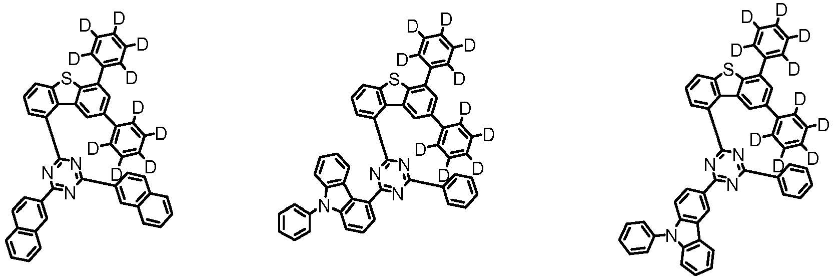

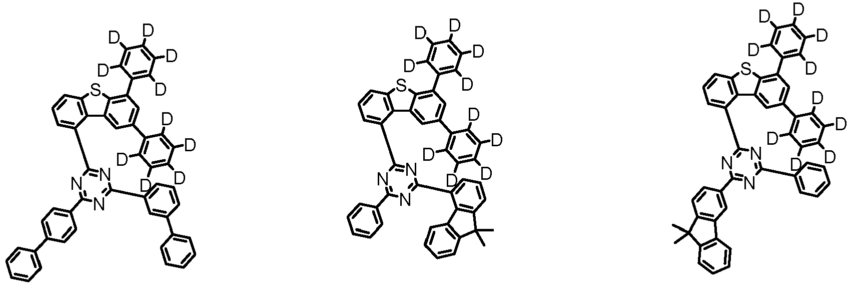

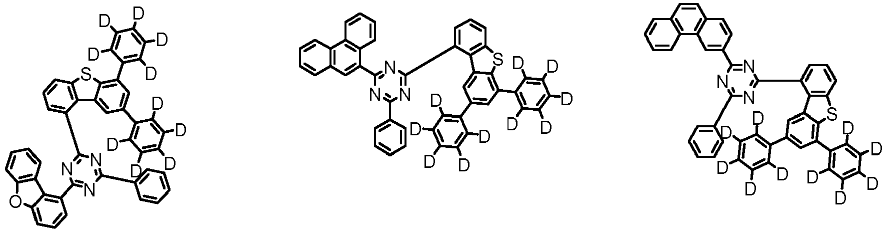

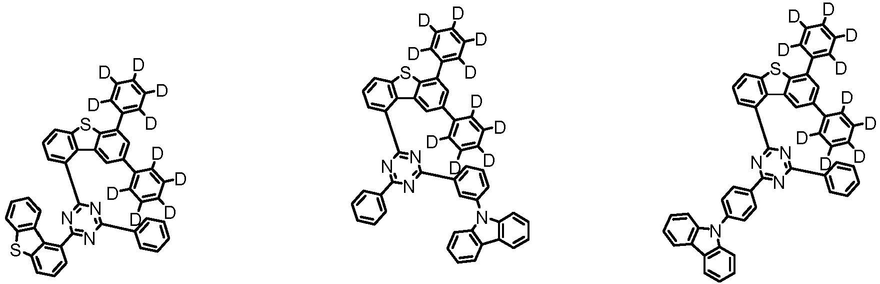

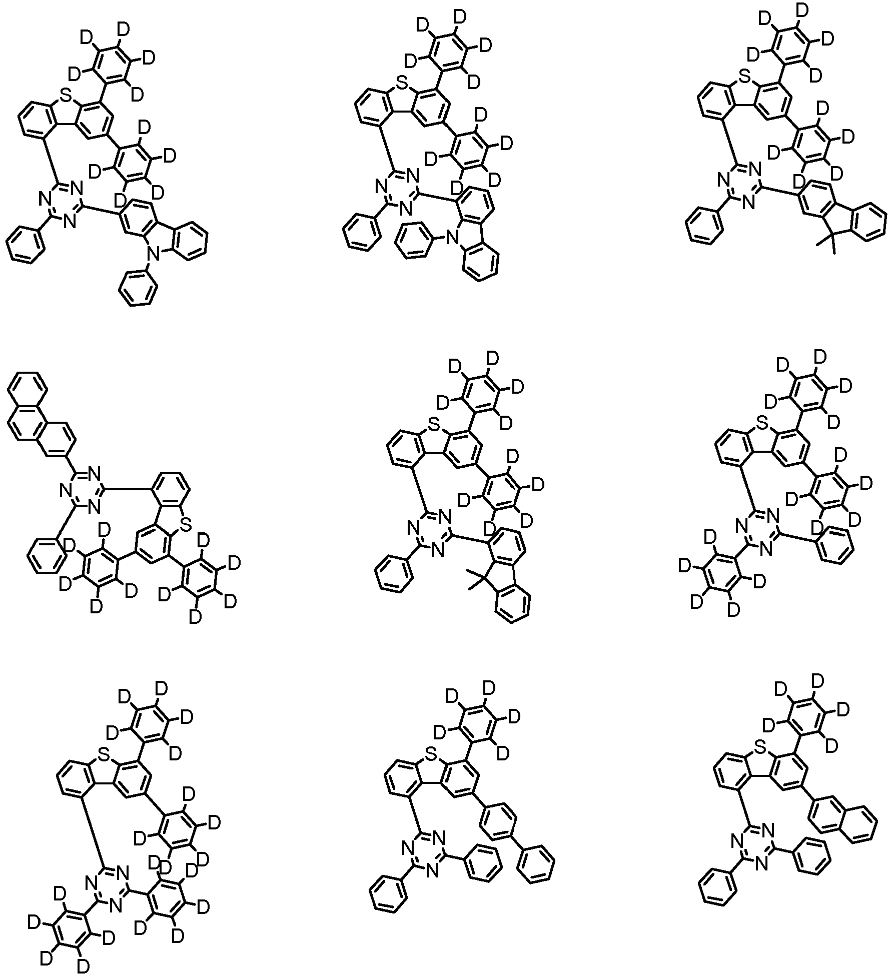

11. Dispositif électroluminescent organique comprenant : une première électrode ; une seconde électrode qui est disposée à l'opposé de la première électrode ; et une ou plusieurs couches de matériau organique qui sont disposées entre la première électrode et la seconde électrode, dans lequel au moins une couche parmi les couches de matériau organique comprend le composé selon l'une quelconque des revendications 1 à 10.

12. Dispositif électroluminescent organique selon la revendication 11,
dans lequel la couche de matériau organique comprenant le composé est une couche d'émission de lumière.

13. Dispositif électroluminescent organique selon la revendication 12,
dans lequel la couche d'émission de lumière comprend en outre un composé représenté par la formule chimique 2 suivante : 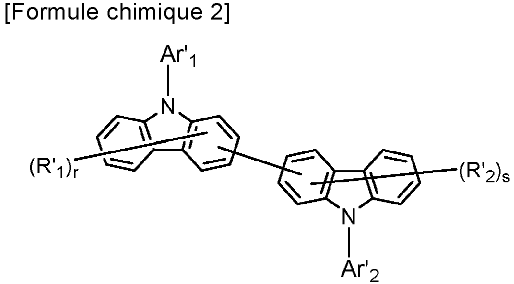
dans la formule chimique 2,
Ar'₁ et Ar'₂ sont chacun indépendamment un aryle en C₆₋₆₀ substitué ou non substitué ; ou un hétéroaryle en C₂₋₆₀ substitué ou non substitué contenant au moins un hétéroatome sélectionné dans le groupe consistant en N, O et S, et
R'₁ et R'₂ sont chacun indépendamment un hydrogène ; un deutérium ; un alkyle en C₁₋₆₀ ; un aryle en C₆₋₆₀ ; ou un hétéroaryle en C₂₋₆₀ contenant au moins un hétéroatome sélectionné dans le groupe consistant en N, O et S ; et
r et s sont chacun indépendamment un nombre entier de 0 à 7.

14. Dispositif électroluminescent organique selon la revendication 13,
dans lequel le composé représenté par la formule chimique 2 est l'un quelconque sélectionné dans le groupe consistant en les composés suivants :
